# EUROPEAN PATENT SPECIFICATION

(11) **EP 4 334 299 B1**
(45) Date of publication and mention of the grant of the patent: **15.07.2026**
(21) Application number: 22725909.0
(22) Date of filing: 03.05.2022
(51) Int. Cl.: C07D 401/04, A61K 31/538, A61P 31/04, C07D 413/14

(54) **PROCESS FOR THE PREPARATION OF ANTIBACTERIAL COMPOUNDS**
VERFAHREN ZUR HERSTELLUNG VON ANTIBAKTERIELLEN VERBINDUNGEN
PROCÉDÉ DE PRÉPARATION DE COMPOSÉS ANTIBACTÉRIENS

(30) Priority: 03.05.2021 IN 202111020227; 24.06.2021 EP 21181594; 05.04.2022 GB 202204981
(43) Date of publication of application: 13.03.2024
(73) Proprietor: Discuva Ltd., Milton Park Abingdon Oxfordshire OX14 4SB (GB)
(72) Inventor: TIMMINS, Peter, Kelsall, Tarporley Cheshire CW6 0GY (GB); CARNIAUX, Jean-Francois, Marcham, Abingdon Oxfordshire OX13 6FJ (GB); BHOWMIK, Dipal, Noida -201301 Uttar Pradesh (IN); SRIVASTAVA, Anil, Noida -201301 Uttar Pradesh (IN); DEB, Prasant, Noida -201301 Uttar Pradesh (IN); MAHAJAN, Akhil, Noida -201301 Uttar Pradesh (IN); REDDY, Vijaya, Noida -201301 Uttar Pradesh (IN); VANDANAPU, Purushotham, Noida -201301 Uttar Pradesh (IN); KHAN, Mohammed Nawaz, Abingdon Oxfordshire OX14 4SB (GB); KUMAR, Gulsham, Noida -201301 Uttar Pradesh (IN); KUMAR, Suresh, Noida -201301 Uttar Pradesh (IN)
(74) Representative: Gill Jennings & Every LLP
(86) International application number: PCT/EP2022/061879
(87) International publication number: WO 2022/233891

(56) References cited:
- WO-A1-2019/086890

## Description

### Field of the Invention

The present invention relates to a process for the production of an antibacterial compound as defined herein.

### Background of the Invention

There is a need for new antibacterial compounds to counter the emergence of bacterial pathogens with resistance to existing antibacterial compounds. The increasing occurrence of bacterial resistance to existing antibiotics threatens to greatly enhance the burden that common infections place on society, with multidrug resistance becoming common amongst a number of bacterial pathogens. For example, antibiotic-resistant strains of the ESKAPE pathogens *(Enterococcus faecium, Staphylococcus aureus, Klebsiella pneumoniae, Acinetobacter baumannii, Pseudomonas aeruginosa* and *Enterobacter* species), such as carbapenem-resistant Enterobacterales (CRE), multi-drug resistant (MDR) *Acinetobacter,* MDR *Pseudomonas aeruginosa,* methicillin-resistant *Staphylococcus aureus* (MRSA) and vancomycin-resistant *Enterococcus* (VRE) have been included in a list of antibiotic-resistant microorganisms identified as posing an urgent or serious threat to human health. Other prominent antibiotic-resistant pathogens include the Gram-positive anaerobe *Clostridium difficile,* drug-resistant *Neisseria gonorrhoeae* and drug-resistant *tuberculosis.*

Specifically, the resistance of Gram-negative bacteria, in particular of the order Enterobacterales, to existing antibiotic agents such as carbapenem (β-lactam) antibiotics is increasing. Antibiotic-resistant Gram-negative Enterobacterales strains, such as carbapenemase-producing Enterobacterales e.g. *Escherichia coli* NDM-1 (New Delhi metallo-β-lactamase) and *Klebsiella pneumoniae* are difficult to treat and are becoming increasingly widespread and virulent. Moreover, new emerging hyper-virulent, multidrug resistant and highly transmissible strains of carbapenem-resistant *Klebsiella pneumoniae* associated with fatal outbreaks have been identified, for example, ST11 carbapenem-resistant hypervirulent *Klebsiella pneumoniae* strains. Such strains are resistant to previously and currently recommended antibiotics and are now a global major public health concern.

Currently, Enterobacterales infections where carbapenem-resistant Enterobacterales (CRE) are suspected, are commonly treated with broad spectrum agents. Such treatment typically consists of a combination of a β-lactam antibiotic and a β-lacatamase inhibitor (BL/BLI), or the use of colistin, an antibiotic of last resort. The BL/BLI combinations, whilst effective in the short term, are predicted to eventually succumb to pre-existing resistance mechanisms of the bacteria. Furthermore, these broad spectrum agents are not active against all Ambler β-lactamase classes, having no activity against metallo-β-lactamases, which confer resistance to a broad range of β-lactam antibiotics, including carbapenem antibiotics.

There is therefore a need for effective scalable process for the production of new antibacterial compounds that can provide effective treatment in a reliable manner for an infection with, or disease caused or exacerbated by, Gram-negative bacteria of the order Enterobacterales which are resistant to known antibiotics such as carbapenems and other β-lactams, or involving multidrug resistant infection agents. There is additionally a need for the provision of antibiotic agents that can avoid or reduce the side-effects associated with known antibacterial compounds.

It is an object of the aspects of the present invention to provide an effective scalable process for the formation of an antibacterial compound that provides a solution to the above mentioned or other problems.

WO 2019/086890 A1 discloses compounds finding application in the treatment of infection with, and diseases caused by, *Enterobacteriaceae.*

### Summary of the Invention

According to a first aspect of the present invention, there is provided a process for the production of compound (I) 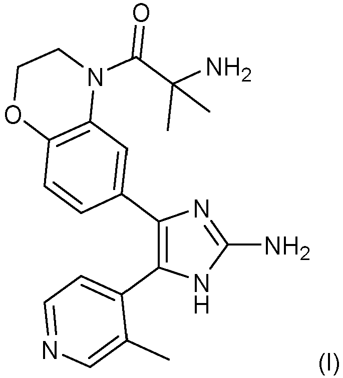 or a pharmaceutically acceptable salt thereof, the process comprising deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or salts thereof

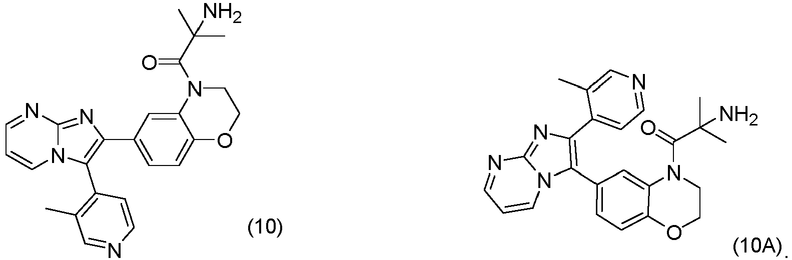

According to a second aspect of the present invention, there is provided a process for the production of compound (I) 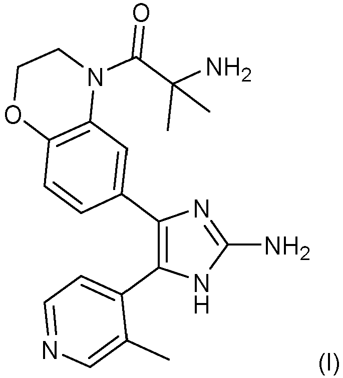 or a pharmaceutically acceptable salt thereof, the process comprising the following step:
coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) 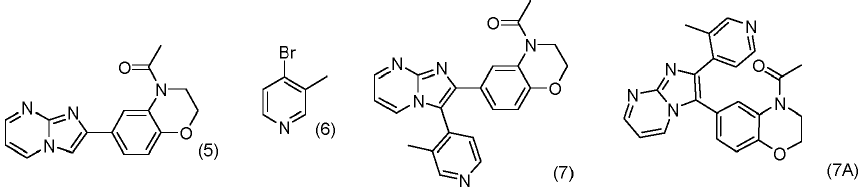
and preferably wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) undergo a metal-scavenging treatment, the metal-scavenging treatment comprising treatment with a metal scavenging agent.

According to a third aspect of the present invention, there is provided a use of a metal-scavenging agent in a process for the production of compound (I) 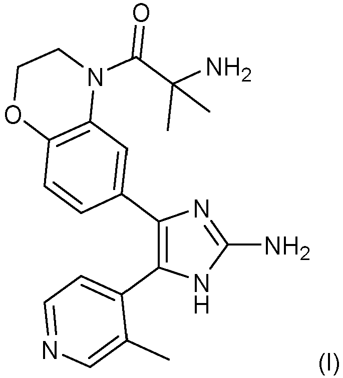 or a pharmaceutically acceptable salt thereof, wherein for the process comprises the step of coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), 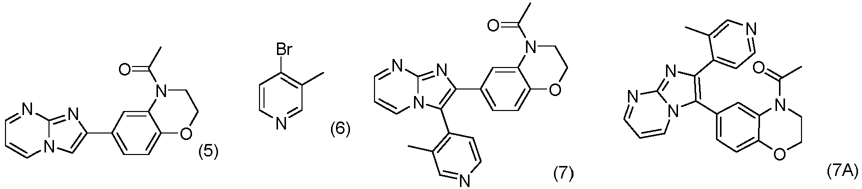 wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is treated with the metal-scavenging agent.

According to a fourth aspect of the present invention, there is provided a process for the production of compound (I) 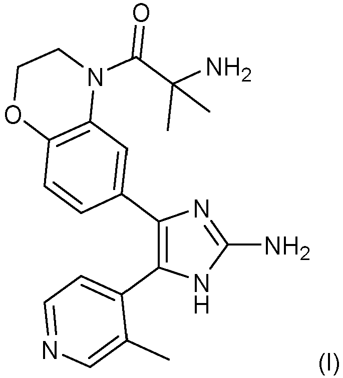 or a pharmaceutically acceptable salt thereof, the process comprising amino acid coupling of 4-(3,4-dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor:

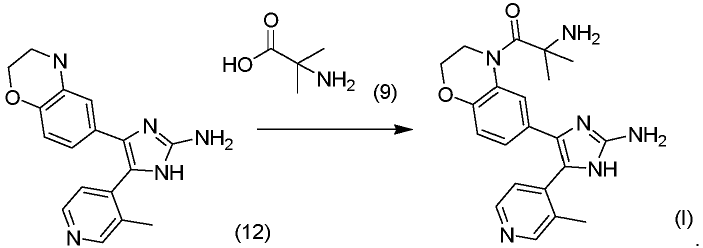

### Definitions

As used herein, the term "disease" or "bacterial disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from trauma, injury, surgery, radiological ablation, poisoning or nutritional deficiencies. The term refers to any disease that involves (e.g. is caused, exacerbated, associated with or characterized by the presence of) a bacterium residing and/or replicating in the body and/or cells of a subject. The term therefore includes diseases caused or exacerbated by bacterial toxins (which may also be referred to herein as "bacterial intoxication").

As used herein, the term "infection" or "bacterial infection" is used to define a condition in which a subject is infected with a bacterium. The infection may be symptomatic or asymptomatic. In the former case, the subject may be identified as infected on the basis of established diagnostic criteria. In the latter case, the subject may be identified as infected on the basis of various tests, including for example biochemical tests, serological tests, microbiological culture and/or microscopy. Thus, the invention typically finds application in the treatment of subjects in which a bacterial infection has been diagnosed or detected.

As used herein, the term "treatment" or "treating" refers to an intervention (e.g. the administration of an agent to a subject) which cures, ameliorates or lessens the symptoms of a disease or removes (or lessens the impact of) its cause(s) (for example, the causative bacterium). In this case, the term is used synonymously with the term "therapy". Thus, the treatment of infection according to the invention may be characterized by the (direct or indirect) bactericidal action of the compounds of the invention. Thus, the compounds of the invention find application in methods of killing, or preventing the growth of, bacterial cells.

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

The term "subject" (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Preferably, the subject is a human.

The terms "Gram-negative bacterium" and "Gram-positive bacterium" are terms of art defining two distinct classes of bacteria on the basis of certain cell wall staining characteristics.

The term "efficacious" includes advantageous effects such as additivity, synergism, reduced side effects, reduced toxicity, improved performance, reduced bacterial burden during infection, or activity.

The term "pharmaceutically acceptable salt" as applied to the compound (I) of the invention defines any organic or inorganic acid addition salt of the free base which are suitable for use in contact with the tissues of humans and animals without undue toxicity, irritation, allergic response and which are commensurate with a reasonable benefit/risk ratio. Suitable pharmaceutically acceptable salts are well known in the art. Examples are the salts formed from inorganic acids (for example hydrochloric, hydrobromic, sulphuric and phosphoric acids), organic carboxylic acids (for example acetic, propionic, glycolic, lactic, pyruvic, malonic, succinic, fumaric, malic, tartaric, citric, ascorbic, maleic, hydroxymaleic, dihydroxymaleic, benzoic, phenylacetic, 4-aminobenzoic, 4-hydroxybenzoic, anthranilic, cinnamic, salicylic, 2-phenoxybenzoic, 2-acetoxybenzoic and mandelic acid) and organic sulfonic acids (for example methanesulfonic acid and p-toluenesulfonic acid).

In its broadest aspect, the present invention contemplates the production of all optical isomers of the compound (I), or pharmaceutically acceptable salts.

In cases where the stereochemical form of the compound (I), or pharmaceutically acceptable salt thereof, is important for pharmaceutical utility, the invention contemplates formulation of an isolated eutomer.

The term "resistant strains" as used herein, refers to strains of bacteria that have shown resistance or non-susceptibility to one or more known antibacterial agent, particularly those widely considered to be standard-of-care in the treatment of infections with such strains. A "non-susceptible strain" is one in which the MIC (minimum inhibitory concentration) of a given compound or class of compounds for that strain has shifted to a higher number than for corresponding susceptible strains. For example, it may refer to strains that are non-susceptible to β-lactam antibiotics, strains that are non-susceptible to one or more fluoroquinolones and/or strains that are non-susceptible to one or more other antibiotics (i.e. antibiotics other than β-lactams and fluoroquinolones). In some instances, the term "resistant" may refer to one strain in which the MIC of a given compound or class of compounds for that strain has shifted to a significantly higher number than for corresponding susceptible strains. A bacterial strain might be said to be resistant to a given antibiotic when it is inhibited *in vitro* by a concentration of this agent that is associated with a high likelihood of therapeutic failure.

The term "multidrug resistant" or "multidrug resistance" as used herein, refers to organisms, such as highly resistant Gram-negative bacteria (e.g. carbapenemase-producing *Klebsiella pneumoniae),* showing *in vitro* and/or *in vivo* resistance to more than one antimicrobial agent. Such organisms may be resistant to all of the currently available antimicrobial agents or remain susceptible only to older, potentially more toxic, antimicrobial agents.

The term "hypervirulent" as used herein, refers to organisms that are exceptionally virulent, generally as a result of the acquisition of a virulence plasmid. Such organisms are capable of producing severe illness. For completeness, "virulent" refers to organisms capable of producing extremely severe or harmful effects and illness.

The term "antibacterial treatment" as used herein is meant treatment of the infection or disease of a subject with an antibacterial agent other than compound (I), or a pharmaceutically acceptable salt thereof. Typically, in the context of the present invention, this is with known antibiotics or treatment methods, including carbapenem or other β-lactam antibiotics, other than compound (I), or a pharmaceutically acceptable salt thereof.

### Detailed Description of the Invention

The present invention provides the solution to a previously unmet clinical need with regard to the treatment of infection with, or disease caused or exacerbated by Gram-negative bacteria of the order Enterobacterales, particularly carbapenem-resistant Enterobacterales (CRE) and extended spectrum β-lactamase (ESBL) Enterobacterales, as well as treatment of infection with, or disease caused or exacerbated by Gram-negative bacteria of the genus *Haemophilus.*

It has been surprisingly and advantageously found that compound (I) or a pharmaceutically acceptable salt thereof, enables the treatment of infection with, or disease caused or exacerbated by Gram-negative bacteria of the order Enterobacterales and/or Gram-negative bacteria of the genus *Haemophilus,* including Gram-negative bacteria of the order Enterobacterales and/or Gram-negative bacteria of the genus *Haemophilus* which are resistant to known β-lactam antibiotics and a major cause of mortality.

Compound (I), or a pharmaceutically acceptable salt thereof, may be particularly advantageous in the treatment of multi-site infection, i.e. infections with Gram-negative bacteria of the order Enterobacterales and/or Gram-negative bacteria of the genus *Haemophilus,* and occurring at different infection sites (intracellular sites in tissues and/or organs) of a subject (shown by the data provided in the Examples section of the present invention). Compound (I), or a pharmaceutically acceptable salt thereof, may further be advantageously utilised in the treatment of infection with, or diseases caused or exacerbated by Gram-negative bacteria of the order Enterobacterales and/or Gram-negative bacteria of the genus *Haemophilus,* where antibacterial treatment of the disease or infection in the subject requiring treatment has failed, or where the subject is allergic or otherwise contra-indicated to any agents used, or considered for use, in antibacterial treatment.

Compound (I), or a pharmaceutically acceptable salt thereof, appears to be rapidly bactericidal and is considered highly active against all currently tested clinical mechanisms of resistance, including all Ambler β-lactamase classes (including metallo-β-lactams). Compound (I), or a pharmaceutically acceptable salt thereof, does not appear to be subject to deactivation by the full range of carbapenem metabolising enzymes, demonstrating activity against all currently tested CREs independent of the carbapenemase or other resistance characteristics. In contrast to known BL/BLI combination treatments, compound (I), or a pharmaceutically acceptable salt thereof, has shown a low propensity for tested resistance development *in vitro.*

Furthermore, compound (I), or a pharmaceutically acceptable salt thereof, displays no cross resistance with currently tested antibiotic classes, and no observed contraindication. Compound (I), or a pharmaceutically acceptable salt thereof, also demonstrates both *in vitro* and *in vivo* safety in pharmacology studies.

Accordingly, compound (I), or a pharmaceutically acceptable salt thereof, may be used in the treatment of bacterial infections and diseases caused or exacerbated by Gram-negative bacteria of the order Enterobacterales and/or Gram-negative bacteria of the genus *Haemophilus,* in particular, those bacteria resistant to known antibiotics, such as carbapenem-resistant Enterobacterales (CRE) and extended spectrum β-lactamase (ESBL) Enterobacterales. The compound (I), or a pharmaceutically acceptable salt thereof, has bactericidal activity against Gram-negative bacteria of the order Enterobacterales and/or Gram-negative bacteria of the genus *Haemophilus,* in particular, those bacteria resistant to known antibiotics, such as carbapenem-resistant Enterobacterales (CRE) and extended spectrum β-lactamase (ESBL) Enterobacterales.

The present invention relates to a process for production of compound (I), or a pharmaceutically acceptable salt thereof. This process is effective and scalable. It is a route of synthesis that may advantageously use a commercially available starting material, can be adjusted so as to largely avoid purification by column chromatography, and produces a compound (I), or a pharmaceutically acceptable salt thereof, at high yield and purity. Any harmful reagents, such as metal ions of metal catalysts, utilised during the process may be advantageously removed in subsequent steps of the process so as to be present at only a trace level in the final product.

The process for the production of compound (I), or a pharmaceutically acceptable salt thereof, comprises the deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or salts thereof

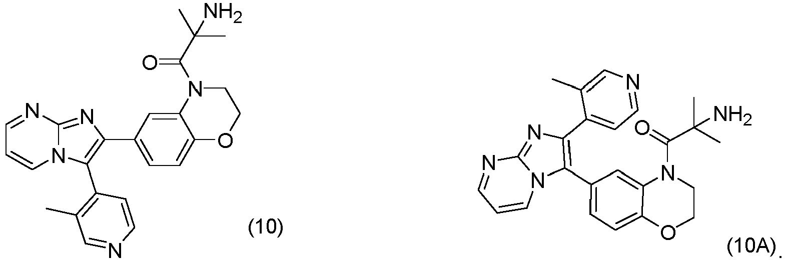

This process isolates a compound (I), or a pharmaceutically acceptable salt, thereof, at high yield and purity.

The process may comprise the deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A).

Generally, when a mixture of regioisomers 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is used in the process, regiosiomer 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is predominant.

When one of the regioisomers 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), is used in the process, regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) is generally selected.

Alternatively, the process may comprise the deprotection of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A).

Generally, when a mixture of a salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is used in the process, the salt of regiosiomer 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is predominant.

When one of the salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) or the salt of regioisomer 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is used in the process, the salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) is generally selected.

Preferably, the salts of the two regioisomers (10) and (10A) will be the same salt.

When the process comprises the deprotection of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), each of the salts of (10) and (10A) may be a hydrochloride salt, such as a dihydrochloride (bishydrochloride) salt. It will be appreciated that the salts of the two regioisomers (10) and (10A) will be the same hydrochloride salt, for example, both may be the dihydrochloride salt. Accordingly, the process may comprise a deprotection of a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A).

Generally, when a mixture of a hydrochloride salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is used in the process, the hydrochloride salt of regiosiomer 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is predominant.

When one of the hydrochloride salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) or the hydrochloride salt of regioisomer 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), is used in the process, the hydrochloride salt of regioisomer 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) is generally selected.

Preferably, when the process comprises the deprotection of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), the deprotection is of the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride (bishydrochloride) salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one:

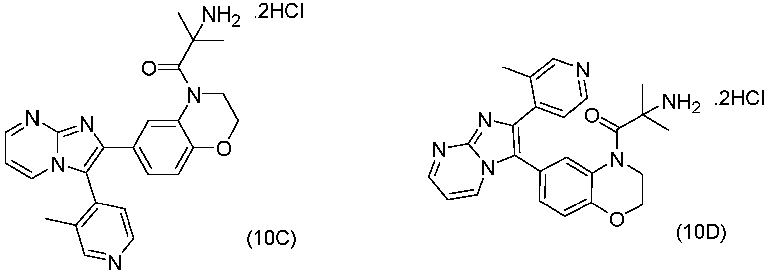

Generally, when a mixture of the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and the dihydrochloride (bishydrochloride) salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one is used in the process, the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one is predominant. When one of the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one or dihydrochloride (bishydrochloride) salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, is used in the process, the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one is generally selected.

The process preferably comprises deprotection of the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one.

Deprotection of the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one advantageous facilitates compound (I), or a pharmaceutically acceptable salt thereof, at high yield.

The deprotection may be base or acid catalysed, preferably base catalysed.

Suitable deprotection reagents include, but are not limited to: NH₂OH.HCl (hydroxylamine hydrochloride), and NH₂NH₂ (hydrazine) or the hydrate thereof. Preferably, the deprotection reagent is NH₂OH.HCl (hydroxylamine hydrochloride). It has been surprisingly and advantageously found that when the deprotection uses NH₂OH.HCl (hydroxylamine hydrochloride), this advantageously improves the yield of compound (I), or a pharmaceutically acceptable salt thereof.

Preferably, when the deprotection is of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10), such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, the deprotection reagent may be accompanied by a base. Suitable bases include 1,8-diazabicyclo(5.4.0)undec-7-ene (DBU), N,N-diisopropylethylamine (DIPEA), piperidine, morpholine, pyrollidine, and triethylamine (TEA). Preferably, the base is selected from 1,8-diazabicyclo(5.4.0)undec-7-ene (DBU), N,N-diisopropylethylamine (DIPEA) and triethylamine (TEA). More preferably, the base is selected to be triethylamine (TEA).

The solvent for the deprotection may be selected from any suitable solvent or combinations thereof, for example, isopropyl alcohol (IPA), ethanol (EtOH), dichloromethane (DCM), methanol (MeOH), toluene, tetrahydrofuran (THF), methyl tert-butyl ether (TBME), and acetonitrile (CH₃CN). Preferably, the solvent is selected from methanol, dichloromethane and isopropyl alcohol (IPA). More preferably, the solvent is selected to be isopropyl alcohol (IPA).

Preferably, when the deprotection is of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10), such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, the deprotection may be carried out at a temperature of from 30 to 80 °C, such as from 40 to 70 °C, preferably 40 to 60 °C.

Preferably, when the deprotection is of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), the deprotection reagent is selected to be NH₂OH.HCl (hydroxylamine hydrochloride), and the solvent is selected to be isopropyl alcohol (IPA).

Preferably, when the deprotection is of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10), such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, the deprotection reagent is selected to be NH₂OH.HCl (hydroxylamine hydrochloride) with triethylamine (TEA) as base, in isopropyl alcohol (IPA) as solvent.

Preferably, when the deprotection is of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), and NH₂OH.HCl (hydroxylamine hydrochloride) is utilised, the deprotection may result in a hydrochloride salt of compound (I). Compound (I), or a pharmaceutically acceptable salt thereof, may be obtained from this hydrochloride salt through suitable extraction methods known to those skilled in the part. Compound (I), or a pharmaceutically acceptable salt thereof, may be produced through treatment of the hydrochloride salt of compound (I) under basic conditions. Such treatment may include the use of a base, for example sodium bicarbonate. The compound (I), or a pharmaceutically acceptable salt thereof, may be obtained using 10% methanol/DCM.

Preferably, when the deprotection is of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10), such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, and NH₂OH.HCl (hydroxylamine hydrochloride) is utilised, the deprotection may result in a hydrochloride salt, such as a dihydrochloride salt, of compound (I). Compound (I), or a pharmaceutically acceptable salt thereof, may be produced through treatment of the hydrochloride salt of compound (I) under basic conditions. Compound (I), or a pharmaceutically acceptable salt thereof, may be produced through treatment of the hydrochloride salt of compound (I) with: an ion-exchange resin such as Amberlite IRN78 and MP-carbonate; addition of base such as potassium carbonate or sodium bicarbonate; addition of ammonia in a solvent such as methanol or isopropyl alcohol (IPA); or addition of ammonium hydroxide (NH₄OH). Preferably, compound (I), or a pharmaceutically acceptable salt thereof, is produced through treatment of the dihydrochloride salt of compound (I) with ammonium hydroxide (NH₄OH). Optionally, prior to addition of ammonium hydroxide, the dihydrochloride salt of compound (I) may be neutralised in water. Optionally, dichloromethane may be introduced to aid formation of compound (I). Such treatment of the dihydrochloride salt of compound (I) facilitates the provision of compound (I), or a pharmaceutically acceptable salt thereof, at a high yield and purity.

The process of production of compound (I), or a pharmaceutically acceptable salt thereof, may further comprise a preceding step to facilitate the formation of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof.

Accordingly, the process of production of compound (I), or a pharmaceutically acceptable salt thereof, may further comprise an amino acid coupling in which 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A): 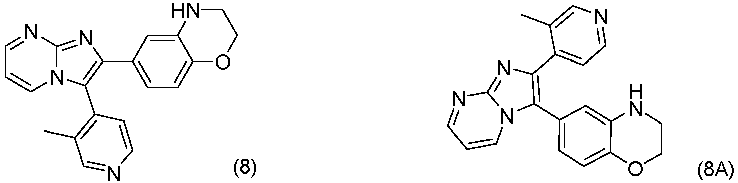 may be utilised to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof.

Generally, when a mixture of the regioisomers 6-(3-(3-methylpyridin-4-yl)imidazo[1,2-a]pyrimidin-2-yl)-3,4-dihydro-2H-benzo[b][1,4]oxazine (8) and 6-(2-(3-methylpyridin-4-yl)imidazo[1,2-a]pyrimidin-3-yl)-3,4-dihydro-2H-benzo[b][1,4]oxazine (8A) is used in the process, the regiosiomer 6-(3-(3-methylpyridin-4-yl)imidazo[1,2-a]pyrimidin-2-yl)-3,4-dihydro-2H-benzo[b][1,4]oxazine (8) is predominant.

When the regioisomer 6-(3-(3-methylpyridin-4-yl)imidazo[1,2-a]pyrimidin-2-yl)-3,4-dihydro-2H-benzo[b][1,4]oxazine (8) or the regioisomer 6-(2-(3-methylpyridin-4-yl)imidazo[1,2-a]pyrimidin-3-yl)-3,4-dihydro-2H-benzo[b][1,4]oxazine (8A) is used in the process, the regioisomers 6-(3-(3-methylpyridin-4-yl)imidazo[1,2-a]pyrimidin-2-yl)-3,4-dihydro-2H-benzo[b][1,4]oxazine (8) is generally selected.

The salts of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) are as described above, and may be the dihydrochloride (bishydrochloride) salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride (bishydrochloride) salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one.

6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A) may be reacted with 2-amino-2-methylpropanoic acid (9) in the presence of a coupling agent or coupling agent precursor to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof.

Preferably, the amino acid coupling step forms a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one.

Preferably, -(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A), is reacted with 2-amino-2-methylpropanoic acid (9) in the presence of a coupling agent or coupling agent precursor to form a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one.

Preferably, when formation of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), is desired, suitable coupling agents or coupling agent precursors include, but are not limited to thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂). Suitable solvents for this amino acid coupling reaction include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) and hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

When formation of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is desired, N,N'dimethylpropyleneurea (DMPU) or hexamethylphosphoramide (HMPA) may be used in combination with thionyl chloride.

Preferably, when formation of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is desired, thionyl chloride (SOCl₂) is utilised as the coupling agent or coupling agent precursor, in either of DMPU and acetonitrile (CH₃CN), or HMPU and acetonitrile.

Preferably, when formation of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) such as the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) such as the dihydrochloride salt of (10D) 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one is desired, suitable coupling agents or coupling agent precursors include, but are not limited to, thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), (1-cyano-2-ethoxy-2-oxoethylidenaminooxy)dimethylamino-morpholino-carbenium hexafluorophosphate (COMU), hydroxybenotriazole, fluoro-dipyrrolidinocarbenium hexafluorophosphate (BTFFH), Ti(iOPr)₄ and B(OCH₂CF₃)₃. Preferably, the coupling agent or coupling agent precursor is selected from hydroxybenotriazole, 1-ethyl-2-)3'-dimethylaminopropyl)carbodiimide.HCl, 1,1'-carbonyldiimidazole (CDI), (1-cyano-2-ethoxy-2-oxoethylidenaminooxy)dimethylamino-morpholino-carbenium hexafluorophosphate (COMU), propanephosphonic acid anhydride (T3P), and thionyl chloride (SOCl₂). More preferably, the coupling agent or coupling agent precursor is thionyl chloride. Sutiable solvents for this amino acid coupling reaction include, but are not limited to: N,N'dimethylpropyleneurea (DMPU), dimethyl sulfoxide (DMSO), dimethylformamide (DMF), hexamethylphosphoramide (HMPA), acetonitrile (ACN), N-methyl-2-pyrolidone (NMP), 1,3-dimethyl-2-imidazolidinone (DMI), gamma-valerolactone (GVL), N-methylmorpholine (NMM), sulfolane, dichloromethane (DCM), or combinations thereof. Preferably, the solvent is selected from N-methyl-2-pyrolidone (NMP), 1,3-dimethyl-2-imidazolidinone (DMI), gamma-valerolactone (GVL), N-methylmorpholine (NMM), sulfolane, dichloromethane (DCM), and acetonitrile, or combinations thereof. More preferably, the solvent is selected from acetonitrile or sulfolane, most preferably acetonitrile. Preferably, this amino acid coupling reaction is carried out in the presence of a base, such as pyridine or other tertiary amines, preferably pyridine.

Preferably, when formation of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) such as the dihydrochloride salt (10C) 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one, and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) such as the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one is desired, the coupling agent or coupling agent precursor is thionyl chloride and the solvent is acetonitrile or sulfolane, preferably acetonitrile, and the reaction is carried out in pyridine.

The process may further comprise an additional preceding step for the production of 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A).

This additional step may be utilised regardless of the subsequent reaction steps employed to form compound (I), i.e. whether 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A) are reacted to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof.

The process may therefore comprise an additional preceding step of deacylation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), to form 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A).

Generally, a mixture of the regioisomers of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is used in the process.

Generally, when a mixture of regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is used in the process, regiosiomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is predominant.

When one of the regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), is used in the process, regioisomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is generally selected.

The deacylation may be acid or base catalysed, preferably base catalysed. Suitable acid catalysts for the deacylation include, but are not limited to: HCI such as 6.0 N HCI, whilst suitable base catalysts include, but are not limited to: NaOH. The NaOH may be used in either organic or aqueous form, such as 2.0 M NaOH or 30% NaOH solution. Preferably, the deacylation is base catalysed using NaOH, more preferably 30% NaOH solution. Deacylation using a base catalyst, specifically NaOH, such as 30% NaOH solution advantageously enables the desired 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) products to be more easily isolated.

Preferably, the deacylation is carried out in a solvent such as methanol.

Preferably, the deacylation is carried out using a base catalyst, preferably, NaOH, and more preferably 30% aq. NaOH, in methanol.

Preferably, the deacylation is carried out at a temperature of from 50 to 80 °C, such as from 60 to 80 °C, or from 60 to 75 °C. The deacylation is preferably carried out for a duration of 4 to 8 hours, such as 5 to 6 hours.

In the context of the process according to the first aspect, 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), preferably 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of the regioisomers (7) and (7A)), may be produced by any suitable synthetic route. 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be produced according to the coupling reaction defined in the second aspect of the present invention. 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be produced according to Steps 1 to 4 of Methods 1, 2, 4 or 5, or Steps 1 to 3 of Methods 3 or 6, or combinations thereof, preferably Steps 1 to 3 of Methods 3 or 6.

According to a second aspect of the present invention, there is provided a process for the production of compound (I) 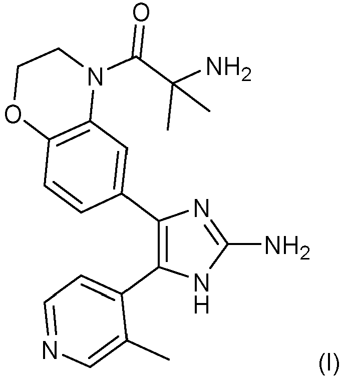 or a pharmaceutically acceptable salt thereof, the process comprising the following step:
coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) 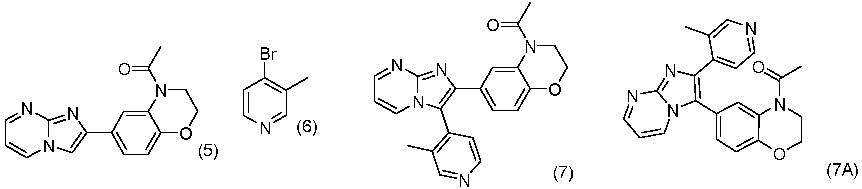
and preferably wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) undergo a metal-scavenging treatment, the metal-scavenging treatment comprising treatment with a metal-scavenging agent.

In the process according to the second aspect of the present invention, 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may subsequently be utilised for a deacylation, and subsequent reaction steps, as described above in relation to the first aspect of the present invention to achieve the formation of compound (I), or a pharmaceutically acceptable salt thereof, or for the deprotection, and subsequent reaction steps, as described below in relation to the fourth aspect of the present invention to achieve the formation of compound (I), or a pharmaceutically acceptable salt thereof.

It will be appreciated that the coupling step outlined in the process of the second aspect of the present invention may be utilised in the process according to the first or fourth aspects of the present invention to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) as detailed therein. The coupling step defined in the second aspect of the present invention may thus be combined with those steps described for the production of compound (I), or a pharmaceutically acceptable salt thereof, according to the first or fourth aspects of the present invention, to arrive at compound (I), or a pharmaceutically acceptable salt thereof.

Accordingly, all features of the second aspect of the present invention as described here, whether preferred or optional, are applicable to the first or fourth aspect of the present invention, and vice versa.

Generally, a mixture of the regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is formed.

Generally, when a mixture of regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is used in the process, regiosiomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is predominant.

When one of the regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), is used in the process, regioisomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is generally selected.

It has been surprisingly and advantageously found that the process of the second aspect of the present invention facilitates advantageous regioisomer formation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) over 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) at 98% to 2% respectively.

In the coupling reaction, 1-(6-(imidazo[1,2-a]pyrimidin-2-yl)-2,3-dihydro-4H-benzo[b][1,4]oxazin-4-yl)ethan-1-one (5) may be reacted with 4-bromo-3-methylpyridine (6), or a salt thereof. Salts thereof include 4-bromo-3-methylpyridine hydrochloride (6A). Preferably, 1-(6-(imidazo[1,2-a]pyrimidin-2-yl)-2,3-dihydro-4H-benzo[b][1,4]oxazin-4-yl)ethan-1-one (5) is reacted with a salt of 4-bromo-3-methylpyridine (6). Preferably, 4-bromo-3-methylpyridine hydrochloride (6A) is utilised. Preferably, 1-(6-(imidazo[1,2-a]pyrimidin-2-yl)-2,3-dihydro-4H-benzo[b][1,4]oxazin-4-yl)ethan-1-one (5) is reacted with 4-bromo-3-methylpyridine hydrochloride (6A):

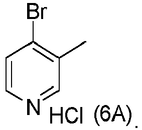

Suitable metal catalysts for the coupling reaction include, but are not limited to: transition metal-based catalysts such as platinum-based catalysts, palladium-based catalysts, and iridium-based catalysts. Typically, the transition metal-based catalysts are utilised in conjunction with a base. Suitable bases include, but are not limited to: potassium salts such as potassium acetate or potassium carbonate or caesium salts such as caesium carbonate. Preferably, the metal catalyst is a palladium-based catalyst, more preferably palladium (II) acetate. Palladium is particularly effective as catalyst in this coupling reaction. Preferably, the metal catalyst is a palladium-based catalyst utilised in conjunction with a base, more preferably a palladium-based catalyst utilised in conjunction with potassium carbonate, and more preferably palladium (II) acetate utilised in conjunction with potassium carbonate.

The coupling reaction may take place in the presence of a homogeneous catalytic system comprising pivalic acid, and tricyclohexylphosphine tetrafluoroborate ((C₆H₁₁)₃P.HBF₄) (ligand).

Suitable solvents for the coupling reaction include dimethylacetamide (DMA) sulfolane, N,N-dimethyl formamide (DMF), dimethyl carbonate, gamma-valerolactone and acetonitrile (ACN). Preferably, the solvent is selected from sulfolane and N-N dimethyl formamide (DMF). More preferably, the solvent is selected to be dimethyl formamide (DMF).

Such a coupling reaction may require control of the residual metal from the metal catalyst in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A). This level of residual metal in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is preferably reduced as far as possible, preferably to less than 150 ppm, or less than 120 ppm, such as less than 100 ppm, or less than 80 ppm, or 50 ppm. Accordingly, only a trace level of residual metal may be present in compound (I), or a pharmaceutically acceptable salt thereof, when formed from 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A).

To control the residual levels of metal present in the products of the coupling reaction 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) after use of a metal catalyst, a metal-scavenging treatment may be employed following production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to reduce metal content. Suitable metal-scavenging treatments include, but are not limited to: charcoal absorption; absorption on different grades of silica columns or silica plug filtration; acidic treatments; and treatment with metal-scavenging agents such as trithiocyanuric acid, silamate thiol resin, neutral alumina, florocil, quadrasil, and 10% aqueous cysteine, or combinations thereof, and combinations thereof. Preferably, the metal-scavenging treatment comprises treatment with a metal-scavenging agent. Preferably, the metal-scavenging agent is trithiocyanuric acid.

Trithiocyanuric acid has CAS number 638-16-4, and may also be known in the art as trimercapto triazine (TMT) or 1,3,5-triazine-2,4,6-trithiol.

When a metal-scavenging agent is utilised, it will be appreciated that the metal-scavenging agent may be used in excess with respect to the amount of metal of the metal catalyst used in the process. The level of residual metal is thus preferably reduced as far as possible as discussed above.

Preferably, the metal catalyst is a palladium-based catalyst, more preferably palladium (II) acetate. Accordingly, the metal-scavenging treatment is preferably capable of scavenging palladium. Preferably, the metal-scavenging treatment comprises treatment with palladium-scavenging agent. Preferably, the palladium-scavenging agent is trithiocyanuric acid .

The treatment with trithiocyanuric acid as metal-scavenging agent may be carried out once or multiple times, preferably twice. Preferably, the metal-scavenging treatment comprises treatment with trithiocyanuric acid in addition to absorption on silica columns or silica plug filtration and charcoal absorption. More preferably, the metal-scavenging treatment comprises treatment with trithiocyanuric acid in addition to silica plug filtration, and charcoal absorption. Typically, the silica plug filtration is carried out first and may use 10% MeOH/DCM. Typically, the treatment with trithiocyanuric acid is carried out alongside the charcoal absorption. The treatment with trithiocyanuric acid may be carried out in solvent selected from dichloromethane, methanol, ethanol, IPA and water, or combinations thereof. For this process, the metal being scavenged is preferably palladium, as a palladium catalyst is preferably utilised.

Solvents such as dichloromethane, methanol, ethanol, IPA and water, or combinations thereof, may be used during the metal-scavenging treatment.

Following metal-scavenging, the level of metal, preferably palladium, in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be less than 150 ppm, or less than 120 ppm, such as less than 100 ppm, or less than 80 ppm, or 50 ppm. When the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is subsequently utilised in further reactions such as those set out in relation to the first or fourth aspects of the present invention to produce compound (I), or a pharmaceutically acceptable salt thereof, the level of metal, preferably palladium, in compound (I), or a pharmaceutically acceptable salt thereof, may be less than 10 ppm, such as less than 5 ppm. Such an amount is considered an acceptable trace amount.

The level of residual metal in compound (I), or other intermediates of the production process such as 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be measured by inductively coupled plasma mass spectrometry (ICP-MS).

Preferably, the coupling reaction takes place under an argon atmosphere.

Preferably, the reagents used for the coupling reaction are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and N,N-dimethyl formamide (DMF). Preferably, the metal-scavenging agent is trithiocyanuric acid.

In the context of the process according to the second aspect, it will be appreciated that 1-(6-(imidazo[1,2-a]pyrimidin-2-yl)-2,3-dihydro-4H-benzo[b][1,4]oxazin-4-yl)ethan-1-one (5) (the starting point for the coupling reaction) may be produced by any suitable synthetic route. Preferably, 1-(6-(imidazo[1,2-a]pyrimidin-2-yl)-2,3-dihydro-4H-benzo[b][1,4]oxazin-4-yl)ethan-1-one (5) is produced according to Steps 1 to 3 of Methods 1, 2, 4 or 5, or Steps 1 and 2 of Methods 3 or 6, or combinations thereof, preferably Steps 1 and 2 of Methods 3 or 6.

Step 4 of Methods 1, 2, 4 and 5, and Step 3 of Methods 3 and 6, detailed below disclose preferred reaction conditions for the coupling reaction detailed in the process of the second aspect of the present invention.

Accordingly, there is also provided a use of a metal-scavenging agent in a process for the production of compound (I) 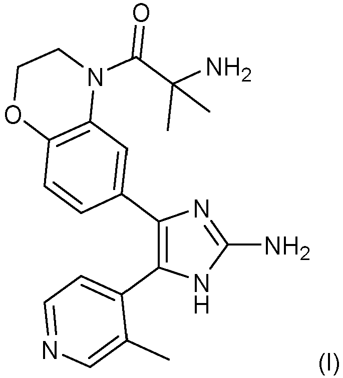
or a pharmaceutically acceptable salt thereof, wherein for the process comprises the step of coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), 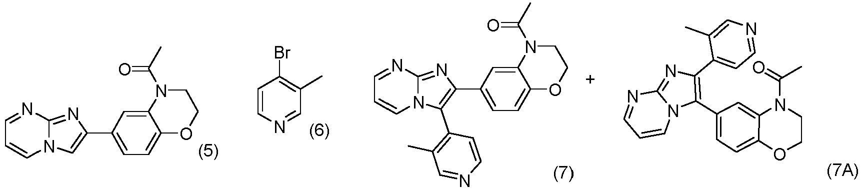
wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) are treated with a metal-scavenging agent.

All features of the second aspect of the present invention as described here, whether preferred or optional, are applicable to the use of the third aspect of the present invention, and vice versa.

According to a fourth aspect of the present invention, there is provided a process for the production of compound (I) 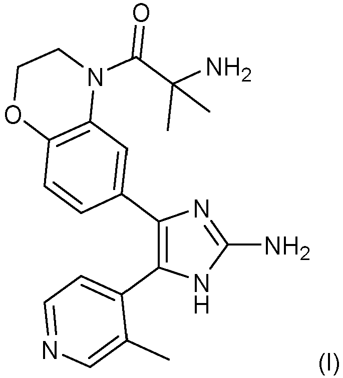 or a pharmaceutically acceptable salt thereof, the process comprising amino acid coupling of 4-(3,4-Dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor:

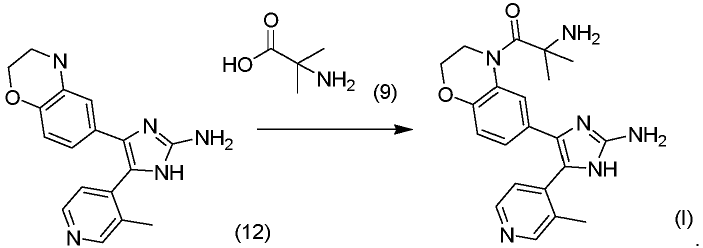

The coupling agent or coupling agent precursor may be selected from thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂). Suitable solvents for the amino acid coupling reaction include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) and hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

For the amino acid coupling, N,N'dimethylpropyleneurea (DMPU) or hexamethylphosphoramide (HMPA) may be used in combination with thionyl chloride.

Preferably, thionyl chloride (SOCl₂) is utilised as the coupling agent or coupling agent precursor, in either of DMPU and acetonitrile (CH₃CN), or HMPU and acetonitrile (CH₃CN).

The process according to the fourth aspect of the present invention may further comprise a preceding step for the formation of compound (12).

The process may further comprise the deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12)

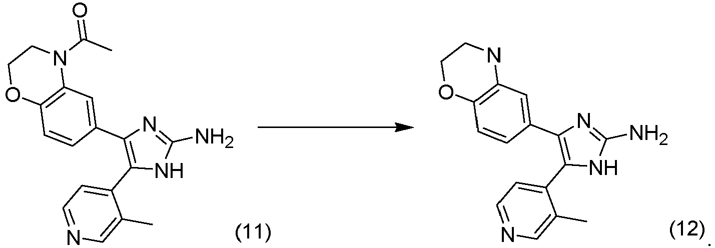

The deacylation may be base or acid catalysed, preferably acid catalysed. Suitable acid catalysts for the deacylation include, but are not limited to: HCl such as 6.0 N HCI, whilst suitable base catalysts include, but are not limited to: NaOH such as 2.0 M NaOH. Preferably, the deacylation is acid catalysed using HCI, preferably 6.0 N HCI, and preferably in the presence of methanol (MeOH).

The process according to the fourth aspect of the present invention may further comprise an additional preceding step for the formation of 1-(6-(2-Amino-5-(3-methylpyridin-4-yl)-1H-imidazol-4-yl)-2,3-dihydro-4H-benzo[b][1,4]oxazin-4-yl)ethan-1-one (11).

The process may further comprise the deprotection of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), to form 1-(6-(2-Amino-5-(3-methylpyridin-4-yl)-1H-imidazol-4-yl)-2,3-dihydro-4H-benzo[b][1,4]oxazin-4-yl)ethan-1-one (11).

Generally, the mixture of regiosiomers of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) is used.

Generally, when a mixture of regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is used in the process, regiosiomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is predominant.

When one of the regioisomers 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), is used in the process, regioisomer 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) is generally selected.

The deprotection may be base or acid catalysed, preferably base catalysed.

Suitable deprotection reagents include, but are not limited to: NH₂OH.HCl (hydroxylamine hydrochloride), and NH₂NH₂ (hydrazine) or the hydrate thereof. Preferably, the deprotection reagent is NH₂OH.HCl (hydroxylamine hydrochloride).

The solvent for the deprotection may be selected from any suitable solvent or combinations thereof, for example, isopropyl alcohol (IPA), ethanol (EtOH), dichloromethane (DCM), methanol (MeOH), and acetonitrile (CH₃CN). Preferably, the solvent is selected from methanol, dichloromethane and isopropyl alcohol (IPA). More preferably the solvent is selected to be isopropyl alcohol (IPA). Preferably, the deprotection reagent is NH₂OH.HCl (hydroxylamine hydrochloride), in isopropyl alcohol (IPA) as solvent.

In the context of the process according to the fourth aspect, 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be produced by any suitable synthetic route. 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be produced according to the coupling reaction defined in the second aspect of the present invention. 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be produced according to Steps 1 to 4 of Methods 1, 2, 4 or 5, or Steps 1 to 3 of Methods 3 or 6, or combinations thereof, preferably Steps 1 to 3 of Methods 3 or 6.

It will therefore be appreciated that the fourth aspect presents an alternative route of formation for the production of compound (I), or a pharmaceutically acceptable salt thereof from 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), to that provided in the first aspect of the present invention. As noted above, the production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be produced in the same way for both the first and fourth aspects such that the alternative synthesis differs solely in the steps following the production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) that lead to the formation of compound (I), or a pharmaceutically acceptable salt thereof.

According to a further aspect of the present invention, there is provided a process for the production of compound (I) 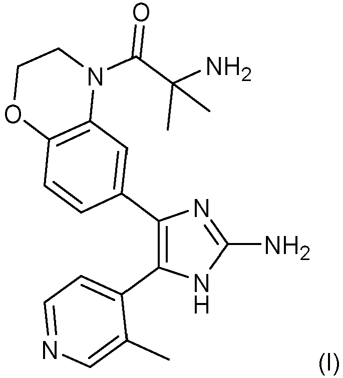 or a pharmaceutically acceptable salt thereof, the process comprising the step of the deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12).

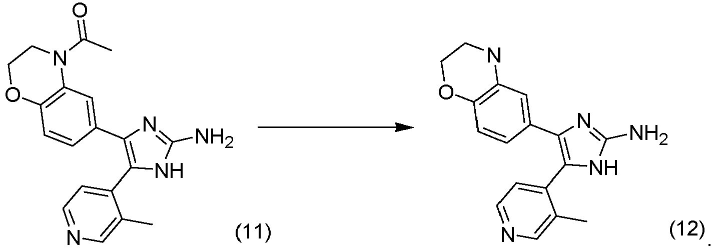

The deacylation may be base or acid catalysed, preferably acid catalysed. Suitable acid catalysts for the deacylation include, but are not limited to: HCl such as 6.0 N HCI, whilst suitable base catalysts include, but are not limited to: NaOH such as 2.0 M NaOH. Preferably, the deacylation is acid catalysed using HCI, preferably 6.0 N HCI, and preferably in the presence of methanol (MeOH).

Suitable methods for synthesising compound (I) include, but are not limited to, the following Methods 1, 2 and 3. Methods 1, 2 and 3 represent preferred methods for the synthesis of compound (I), said methods comprising the preferred connected steps detailed below. It will however be appreciated that, in the context of the present invention, these preferred steps are separable, and one or more of the steps may be substituted with any other suitable step identified by a skilled person. Of Methods 1, 2 and 3, compound (I) is preferably formed by Method 3.

### Method 1

Compound (I) may be synthesised according to the following method:

### Step 1

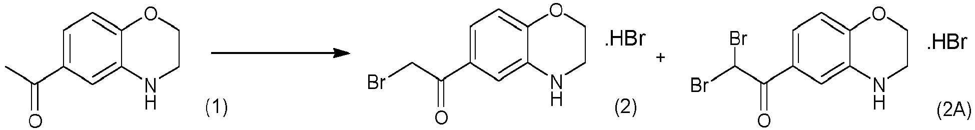

Bromination of 1-(3,4-dihydro-2H-1,4-benzoxazin-6-yl)ethan-1-one (1) to form 2-bromo-1-(3,4-dihydro-2H-benzo[b][1,4]oxazin-6-yl)ethan-1-one hydrobromide (2) and 2,2-dibromo-1-(3,4-dihydro-2H-benzo[b][1,4]oxazin-6-yl)ethan-1-one (2A) (by product).

Preferably, the reagents used for the bromination of Step 1 are selected to be hydrogen bromide (HBr) in acetic acid (AcOH), preferably a 33% solution, and Br₂ in glacial acetic acid.

### Step 2

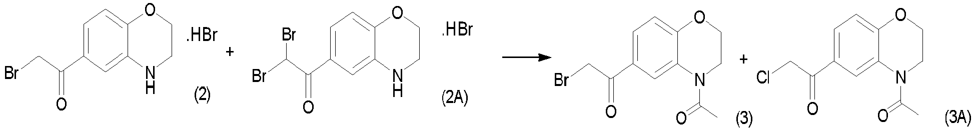

Acylation of 2-bromo-1-(3,4-dihydro-2H-benzo[b][1,4]oxazin-6-yl)ethan-1-one hydrobromide (2) and 2,2-dibromo-1-(3,4-dihydro-2H-benzo[b][1,4]oxazin-6-yl)ethan-1-one (2A with an acylating agent to form 1-(4-Acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A).

Suitable acylating agents for Step 2 include, but are not limited to: acyl halides such as acetyl chloride; and anhydrides of carboxylic acids. Preferably, the acylating agent is acetyl chloride (CH₃COCl).

Preferably, the reagent used for the acylation of Step 2 is acetyl chloride (CH₃COCl) as acylating agent in THF (tetrahydrofuran).

### Step 3

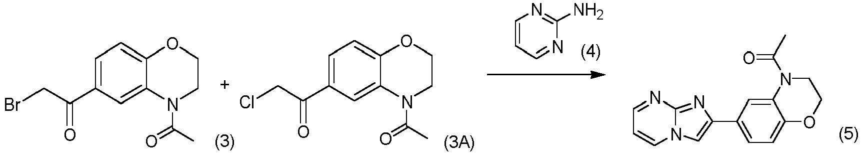

Cyclisation of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A) with pyrimidin-2-amine (4) to form 1-(6-(imidazo[1,2-a]pyrimidin-2-yl)-2,3-dihydro-4H-benzo[b][1,4]oxazin-4-yl)ethan-1-one (5).

Preferably, the solvent used for the cyclisation of Step 3 is ethanol (EtOH).

Preferably, the cyclisation took place at a temperature of from 60 to 85 °C, such as from 75 to 85 °C, for between 12 to 36 hours, such as from 18 to 30 hours.

### Step 4

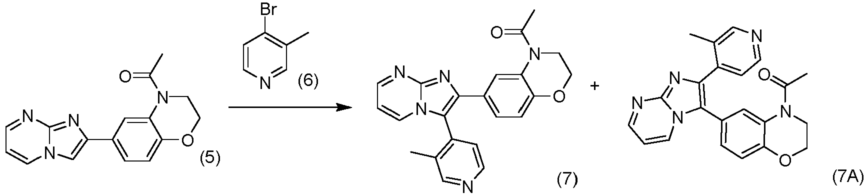

Coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6) in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A).

Suitable metal catalysts for Step 4 include, but are not limited to: transition metal-based catalysts such as platinum-based catalysts, palladium-based catalysts, and iridium-based catalysts. Typically, the transition metal-based catalysts are utilised in conjunction with a base. Suitable bases include, but are not limited to: potassium salts such as potassium acetate or potassium carbonate or caesium salts such as caesium carbonate. Preferably, the metal catalyst is a palladium-based catalyst, more preferably palladium (II) acetate. Preferably, the metal catalyst is a palladium-based catalyst utilised in conjunction with a base, more preferably a palladium-based catalyst utilised in conjunction with potassium carbonate, and more preferably palladium (II) acetate utilised in conjunction with potassium carbonate.

The coupling reaction of Step 4 may take place in the presence of a homogeneous catalytic system comprising pivalic acid, and tricyclohexylphosphine tetrafluoroborate ((C₆H₁₁)₃P.HBF₄) (ligand).

Suitable solvents for the coupling reaction include dimethylacetamide (DMA), sulfolane, N,N-dimethyl formamide (DMF), dimethyl carbonate, gamma-valerolactone and acetonitrile (ACN). Preferably, the solvent is selected to be dimethyl acetamide (DMA).

Preferably, the reagents used for the coupling of Step 4 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and dimethylacetamide (DMA).

Such a coupling reaction may require control of the residual metal from the metal catalyst in the product-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A). This level of residual metal in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is preferably reduced as far as possible, preferably to less than 150 ppm, or less than 120 ppm, such as less than 100 ppm, or less than 80 ppm, or 50 ppm. Accordingly, only a trace level of residual metal may be present in compound (I), or a pharmaceutically acceptable salt thereof, when formed from -(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A).

In order to control the residual levels of metal present in the products of the coupling reaction 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) after use of a metal catalyst, a metal-scavenging treatment may be employed following production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to reduce metal content. Suitable metal-scavenging treatments include, but are not limited to: charcoal absorption; absorption on different grades of silica columns or silica plug filtration; acidic treatments; and treatment with metal-scavenging agents such as trithiocyanuric acid, silamate thiol resin, neutral alumina, florocil, quadrasil, and 10% aqueous cysteine, or combinations thereof; and combinations thereof. Preferably, the metal-scavenging treatment comprises treatment with a metal-scavenging agent. Preferably, the metal-scavenging agent is trithiocyanuric acid.

When a metal-scavenging agent is utilised, it will be appreciated that the metal-scavenging agent may be used in excess with respect to the amount of metal of the metal catalyst used in the process. The level of residual metal is then preferably reduced as far as possible as discussed above.

Preferably, the metal catalyst is a palladium-based catalyst, more preferably palladium (II) acetate. Accordingly, the metal-scavenging treatment is preferably capable of scavenging palladium. Preferably, the metal-scavenging treatment comprises treatment with palladium-scavenging agent. Preferably, the palladium-scavenging agent is trithiocyanuric acid.

The treatment with trithiocyanuric acid as metal-scavenging agent may be carried out once or multiple times, preferably twice. Preferably, the metal-scavenging treatment comprises treatment with trithiocyanuric acid in addition to absorption on silica columns or silica plug filtration and charcoal absorption. More preferably, the metal-scavenging treatment comprises treatment with trithiocyanuric acid in addition to silica plug filtration, and charcoal absorption. Typically, the silica plug filtration is carried out first and may use 10% MeOH/DCM. Typically, the treatment with trithiocyanuric acid is carried out alongside the charcoal absorption. The treatment with trithiocyanuric acid may be carried out in solvent selected from dichloromethane, methanol, ethanol, IPA and water, or combinations thereof. For this process, the metal being scavenged is preferably palladium, as a palladium catalyst is preferably utilised.

Solvents such as dichloromethane, methanol, ethanol, IPA and water, or combinations thereof, may be used during the metal-scavenging treatment.

Following metal-scavenging, the level of metal, preferably palladium, in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be less than 150 ppm, or less than 120 ppm, such as less than 100 ppm, or less than 80 ppm, or 50 ppm. When the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is subsequently utilised in further reactions such as those set out in relation to the first or fourth aspects of the present invention, the level of metal, preferably palladium, in compound (I), or a pharmaceutically acceptable salt thereof, may be less than 10 ppm, such as less than 5 ppm. Such an amount is considered an acceptable trace amount.

The level of residual metal in compound (I), or other intermediates of the production process such as such as 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be measured by inductively coupled plasma mass spectrometry (ICP-MS).

Preferably, the coupling reaction takes place under an argon atmosphere.

Preferably, the reagents used for the coupling reaction of Step 4 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and N,N-dimethyl formamide (DMF). Preferably, the metal-scavenging agent is trithiocyanuric acid.

### Step 5

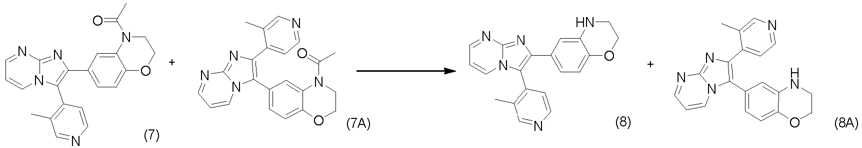

Deacylation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) to form 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (mixture of regioisomers 8 and 8A).

The deacylation of Step 5 may be acid or base catalysed, preferably base catalysed. Suitable acid catalysts for the deacylation of Step 5 include, but are not limited to: HCl such as 6.0 N HCI, whilst suitable base catalysts include, but are not limited to: NaOH, such as 2.0 M NaOH. Preferably, the deacylation is base catalysed using NaOH, more preferably 2.0 M NaOH.

### Step 6

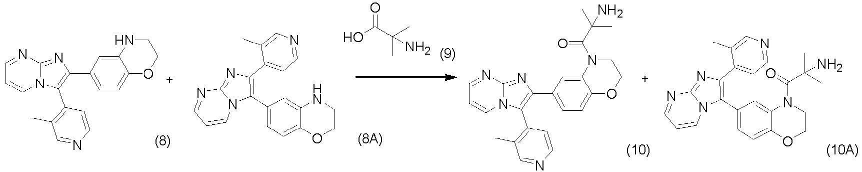

Amino acid coupling of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (mixture of regioisomers 8 and 8A) with 2-amino-2-methylpropanoic acid (9) in the presence of a coupling agent or coupling agent precursor to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (mixture of regioisomers 10 & 10A).

Suitable coupling agents or coupling agent precursors for Step 6 include, but are not limited to thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂).

Sutiable solvents for Step 6 include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) or hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

The amino acid coupling of Step 6 may use N,N'dimethylpropyleneurea (DMPU) or hexamethylphosphoramide (HMPA) in combination with thionyl chloride.

Preferably, for Step 6, thionyl chloride (SOCl₂) is utilised as the coupling agent or coupling agent precursor, in either of DMPU and acetonitrile (CH₃CN), or HMPU and acetonitrile.

### Step 7

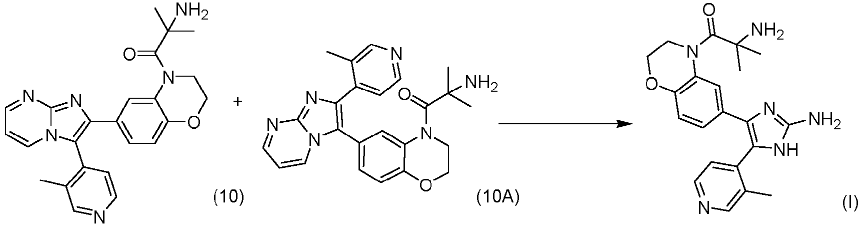

Deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (mixture of regioisomers 10 & 10A) to form compound (I).

The deprotection of Step 7 may be base or acid catalysed, preferably base catalysed.

Suitable deprotection reagents include, but are not limited to: NH₂OH.HCl (hydroxylamine hydrochloride); and NH₂NH₂ (hydrazine) or the hydrate thereof. Preferably, the deprotection reagent is NH₂OH.HCl (hydroxylamine hydrochloride).

The solvent for the deprotection of Step 7 may be selected from any suitable solvent, for example, isopropyl alcohol (IPA), ethanol (EtOH), dichloromethane (DCM), methanol (MeOH), and acetonitrile (CH₃CN). Preferably, the solvent is selected to be isopropyl alcohol (IPA).

Preferably, the deprotection reagent of Step 7 is selected to be NH₂OH.HCl (hydroxylamine hydrochloride), in isopropyl alcohol (IPA).

It will be appreciated that when NH₂OH.HCl (hydroxylamine hydrochloride) is utilised, the deprotection may result in a hydrochloride salt of compound (I). Compound (I) may be produced through the treatment of the hydrochloride salt of compound (I) under basic conditions. Such treatment may include the use of a base, for example sodium bicarbonate. The compound (I), or a pharmaceutically acceptable salt thereof, may be obtained using 10% methanol/DCM.

### Method 2

Compound (I) may be synthesised according to the following method:

### Step 1

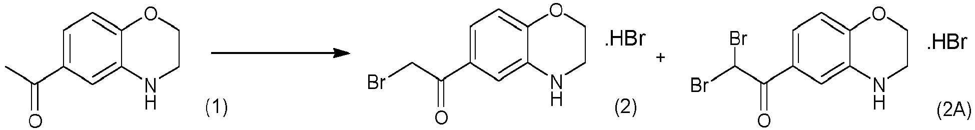

Bromination of 1-(3,4-dihydro-2H-1,4-benzoxazin-6-yl)ethan-1-one (1) to form 2-bromo-1-(3,4-dihydro-2H-benzo[b][1,4]oxazin-6-yl)ethan-1-one hydrobromide (2), and 2,2-dibromo-1-(3,4-dihydro-2H-benzo[b][1,4]oxazin-6-yl)ethan-1-one (2A) by-product.

Preferably, the reagents used for the bromination of Step 1 are selected to be hydrogen bromide (HBr) in acetic acid (AcOH), preferably a 33% solution, and Br₂ in glacial acetic acid.

### Step 2

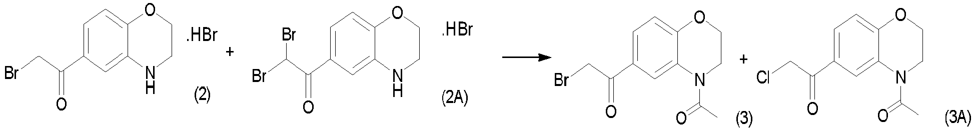

Acylation of 2-bromo-1-(3,4-dihydro-2H-benzo[b][1,4]oxazin-6-yl)ethan-1-one hydrobromide (2) and 2,2-dibromo-1-(3,4-dihydro-2H-benzo[b][1,4]oxazin-6-yl)ethan-1-one (2A) by-product with an acylating agent to form 1-(4-Acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A).

Suitable acylating agents for Step 2 include, but are not limited to: acyl halides such as acetyl chloride; and anhydrides of carboxylic acids. Preferably, the acylating agent is acetyl chloride (CH₃COCl).

Preferably, the reagent used for the acylation of Step 2 is acetyl chloride (CH₃COCl) as acylating agent in THF (tetrahydrofuran).

### Step 3

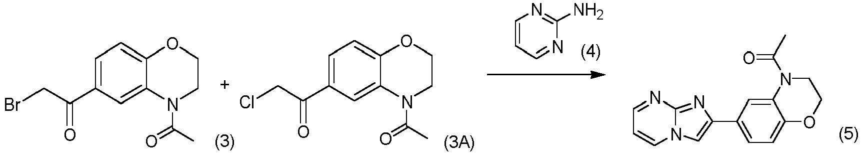

Cyclisation of 1-(4-Acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A) with pyrimidin-2-amine (4) to form 1-(6-(imidazo[1,2-a]pyrimidin-2-yl)-2,3-dihydro-4H-benzo[b][1,4]oxazin-4-yl)ethan-1-one (5).

Preferably, the solvent used for the cyclisation of Step 3 is ethanol (EtOH).

Preferably, the cyclisation took place at a temperature of from 60 to 85 °C, such as from 75 to 85 °C, for between 12 to 36 hours, such as from 18 to 30 hours.

### Step 4

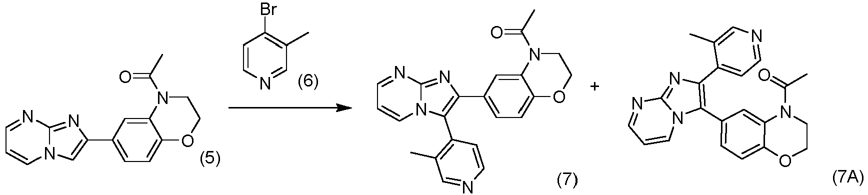

Coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6) in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A).

Suitable metal catalysts for Step 4 include, but are not limited to: transition metal-based catalysts such as platinum-based catalysts, palladium-based catalysts, and iridium-based catalysts. Typically, the transition metal-based catalysts are utilised in conjunction with a base. Suitable bases include, but are not limited to: potassium salts such as potassium acetate or potassium carbonate, or caesium salts such as caesium carbonate. Preferably, the metal catalyst is a palladium-based catalyst, more preferably palladium (II) acetate. Preferably, the metal catalyst is a palladium-based catalyst utilised in conjunction with a base, more preferably a palladium-based catalyst utilised in conjunction with potassium carbonate, and more preferably palladium (II) acetate utilised in conjunction with potassium carbonate.

The coupling reaction of Step 4 may take place in the presence of a homogeneous catalytic system comprising pivalic acid, and tricyclohexylphosphine tetrafluoroborate ((C₆H₁₁)₃P.HBF₄) (ligand).

Suitable solvents for the coupling reaction include dimethylacetamide (DMA), sulfolane, N,N-dimethyl formamide (DMF), dimethyl carbonate, gamma-valerolactone and acetonitrile (ACN). Preferably, the solvent is selected to be dimethyl acetamide (DMA).

Preferably, the reagents used for the coupling of Step 4 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and dimethylacetamide (DMA).

Such a coupling reaction may require control of the residual metal from the metal catalyst in the product-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A). This level of residual metal in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is preferably reduced as far as possible, preferably to less than 150 ppm, or less than 120 ppm, such as less than 100 ppm, or less than 80 ppm, or 50 ppm. Accordingly, only a trace level of residual metal may be present in compound (I), or a pharmaceutically acceptable salt thereof, when formed from -(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A).

In order to control the residual levels of metal present in the products of the coupling reaction 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) after use of a metal catalyst, a metal-scavenging treatment may be employed following production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to reduce metal content. Suitable metal-scavenging treatments include, but are not limited to: charcoal absorption; absorption on different grades of silica columns or silica plug filtration; acidic treatments; and treatment with metal-scavenging agents such as trithiocyanuric acid, silamate thiol resin, neutral alumina, florocil, quadrasil, and 10% aqueous cysteine, or combinations thereof; and combinations thereof. Preferably, the metal-scavenging treatment comprises treatment with a metal-scavenging agent. Preferably, the metal-scavenging agent is trithiocyanuric acid.

When a metal-scavenging agent is utilised, it will be appreciated that the metal-scavenging agent may be used in excess with respect to the amount of metal of the metal catalyst used in the process. The level of residual metal is then preferably reduced as far as possible as discussed above.

Preferably, the metal catalyst is a palladium-based catalyst, more preferably palladium (II) acetate. Accordingly, the metal-scavenging treatment is preferably capable of scavenging palladium. Preferably, the metal-scavenging treatment comprises treatment with palladium-scavenging agent. Preferably, the palladium-scavenging agent is trithiocyanuric acid.

The treatment with trithiocyanuric acid as metal-scavenging agent may be carried out once or multiple times, preferably twice. Preferably, the metal-scavenging treatment comprises treatment with trithiocyanuric acid in addition to absorption on silica columns or silica plug filtration and charcoal absorption. More preferably, the metal-scavenging treatment comprises treatment with trithiocyanuric acid in addition to silica plug filtration, and charcoal absorption. Typically, the silica plug filtration is carried out first and may use 10% MeOH/DCM. Typically, the treatment with trithiocyanuric acid is carried out alongside the charcoal absorption. The treatment with trithiocyanuric acid may be carried out in solvent selected from dichloromethane, methanol, ethanol, IPA and water, or combinations thereof. For this process, the metal being scavenged is preferably palladium, as a palladium catalyst is preferably utilised.

Solvents such as dichloromethane, methanol, ethanol, IPA and water, or combinations thereof, may be used during the metal-scavenging treatment.

Following metal-scavenging, the level of metal, preferably palladium, in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be less than 150 ppm, or less than 120 ppm, such as less than 100 ppm, or less than 80 ppm, or 50 ppm. When the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is subsequently utilised in further reactions such as those set out in relation to the first or fourth aspects of the present invention, the level of metal, preferably palladium, in compound (I), or a pharmaceutically acceptable salt thereof, may be less than 10 ppm, such as less than 5 ppm. Such an amount is considered an acceptable trace amount.

The level of residual metal in compound (I), or other intermediates of the production process such as such as 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be measured by inductively coupled plasma mass spectrometry (ICP-MS).

Preferably, the coupling reaction takes place under an argon atmosphere.

Preferably, the reagents used for the coupling reaction of Step 4 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and N,N-dimethyl formamide (DMF). Preferably, the metal-scavenging agent is trithiocyanuric acid.

### Step 5

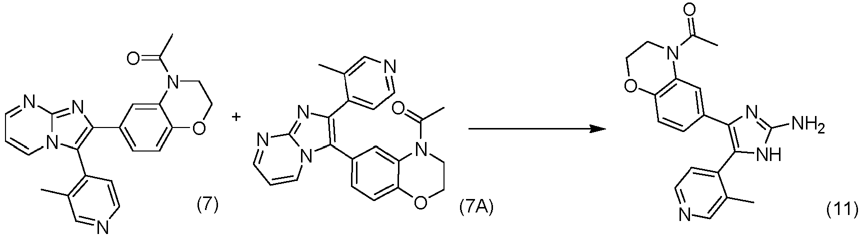

Deprotection of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) to form 1-(6-(2-Amino-5-(3-methylpyridin-4-yl)-1H-imidazol-4-yl)-2,3-dihydro-4H-benzo[b][1,4]oxazin-4-yl)ethan-1-one (11).

The deprotection of Step 5 may be base or acid catalysed, preferably base catalysed.

Suitable deprotection reagents include, but are not limited to: NH₂OH.HCl (hydroxylamine hydrochloride); and NH₂NH₂ (hydrazine) or the hydrate thereof. Preferably, the deprotection reagent is NH₂OH.HCl (hydroxylamine hydrochloride).

The solvent for the deprotection of Step 5 may be selected from any suitable solvent, for example, isopropyl alcohol (IPA), ethanol (EtOH), dichloromethane (DCM), methanol (MeOH), and acetonitrile (CH₃CN). Preferably, the solvent is isopropyl alcohol (IPA).

Preferably, the deprotection reagent for the deprotection of Step 5 is selected to be NH₂OH.HCl (hydroxylamine hydrochloride), in isopropyl alcohol (IPA).

### Step 6

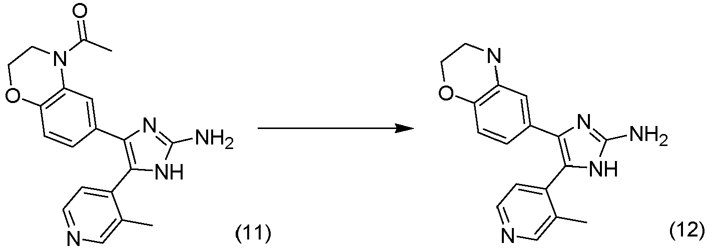

Deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12).

The deacylation of Step 6 may be base or acid catalysed, preferably acid catalysed. Suitable acid catalysts for the deacylation of Step 6 of Method 2 include, but are not limited to: HCl such as 6.0 N HCl, whilst suitable base catalysts include, but are not limited to: NaOH such as 2.0 M NaOH. Preferably, the deacylation is acid catalysed using HCl, preferably 6.0 N HCl, and preferably in the presence of methanol (MeOH).

### Step 7

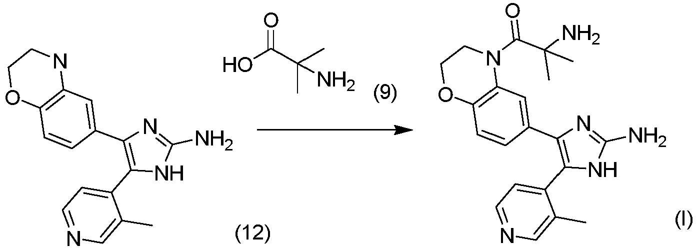

Amino acid coupling of 4-(3,4-Dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor to form compound (I).

Suitable coupling agents or coupling agent precursors for Step 7 include, but are not limited to thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂).

Sutiable solvents for Step 7 include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) and hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

The amino acid coupling of Step 7 may use N,N'dimethylpropyleneurea (DMPU) or hexamethylphosphoramide (HMPA) in combination with thionyl chloride.

Preferably, the reagents for the amino acid coupling of Step 7 are selected to be thionyl chloride (SOCl₂) as coupling agent or coupling agent precursor, in either of DMPU and acetonitrile (CH₃CN), or HMPU and acetonitrile.

For Methods 1 and 2, it has been surprisingly and advantageously found that Step 4 facilitates advantageous regioisomer formation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) over 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) at 98% to 2% respectively.

Accordingly, also encompassed in the present invention is a method of synthesising 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) from 1-(6-(imidazo[1,2-a]pyrimidin-2-yl)-2,3-dihydro-4H-benzo[b][1,4]oxazin-4-yl)ethan-1-one (5), i.e. step 4 of Methods 1 and 2: 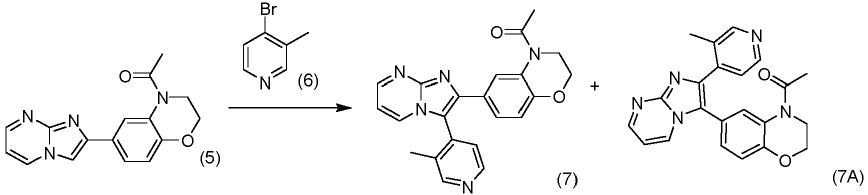 the method comprising coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6) in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A).

Preferably, the metal catalyst is a palladium-based catalyst. More preferably, the metal catalyst is utilised in conjunction with a base, said base being selected from a potassium or caesium salt, preferably a potassium salt. More preferably, the metal catalyst is a palladium-based catalyst and utilised in conjunction with potassium carbonate or potassium actetate. More preferably, the metal catalyst is a palladium-based catalyst and utilised in conjunction with potassium carbonate. More preferably, the metal catalyst is palladium (II) acetate and utilised in conjunction with potassium carbonate.

Preferably, the reagents used for the substitution of Step 4 of Methods 1 and 2 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and dimethylacetamide (DMA).

Such a coupling reaction may require control of the residual metal from the metal catalyst in the product-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A). This may be through methods discussed above for Step 4 of Methods 1 and 2, such as using a metal-scavenging agent like trithiocyanuric acid.

In addition, for Methods 1 and 2 it has been surprisingly and advantageously found that when the deprotection of Step 7 of Method 1 and Step 5 of Method 2 uses NH₂OH.HCl (hydroxylamine hydrochloride), this advantageously improves yield.

Accordingly, also encompassed in the present invention is a method of synthesising compound (I) from 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), i.e. Step 7 of Method 1: 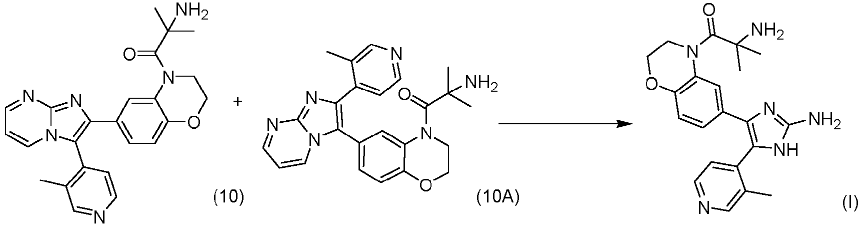 the method comprising deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (mixture of regioisomers 10 & 10A) to form compound (I).

Preferably, the deprotection reagent is NH₂OH.HCl (hydroxylamine hydrochloride).

Any suitable solvent may be utilised in Step 7 of Method 1, for example, the solvent may be selected from isopropyl alcohol (IPA), ethanol (EtOH), methanol (MeOH), dichloromethane (DCM), and acetonitrile (CH₃CN). Preferably, the solvent is isopropyl alcohol (IPA).

Preferably, the deprotection reagent for the deprotection of Step 7 of Method 1 is NH₂OH.HCl (hydroxylamine hydrochloride), in isopropyl alcohol (IPA).

Accordingly, also encompassed in the present invention is a method of synthesising 1-(6-(2-Amino-5-(3-methylpyridin-4-yl)-1H-imidazol-4-yl)-2,3-dihydro-4H-benzo[b][1,4]oxazin-4-yl)ethan-1-one (11) from 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) , i.e. Step 5 of Method 2: 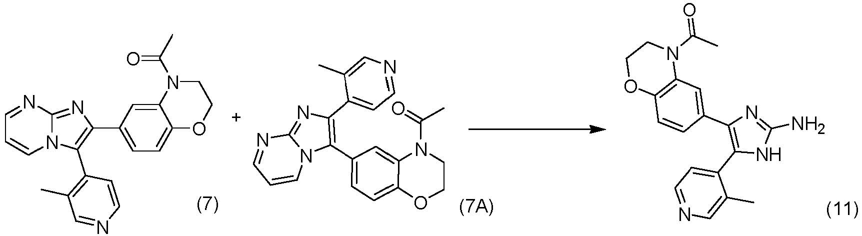 the method comprising deprotection of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) to form 1-(6-(2-Amino-5-(3-methylpyridin-4-yl)-1H-imidazol-4-yl)-2,3-dihydro-4H-benzo[b][1,4]oxazin-4-yl)ethan-1-one (11).

Preferably, the deprotection reagent is NH₂OH.HCl (hydroxylamine hydrochloride).

Any suitable solvent may be utilised in Step 5 of Method 2, for example, the solvent may be selected from isopropyl alcohol (IPA), ethanol (EtOH), methanol (MeOH) and acetonitrile (CH₃CN). Preferably, the solvent is isopropyl alcohol (IPA).

Preferably, the deprotection reagent for the deprotection of Step 5 of Method 2 is NH₂OH.HCl (hydroxylamine hydrochloride), in isopropyl alcohol (IPA).

Also encompassed in the present invention is a method of synthesising compound (I) from 4-(3,4-Dihydro-2H-benzo[b][1,4]oxazin-6-yl)-5-(3-methylpyridin-4-yl)-1H-imidazol-2-amine (12), i.e. step 7 of Method 2: 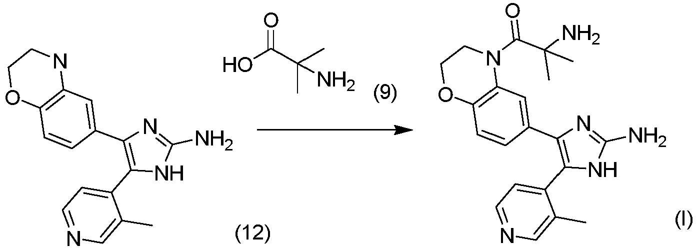 wherein the method comprises amino acid coupling of 4-(3,4-Dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor to form compound (I).

Suitable coupling agents or coupling agent precursors for Step 7 of Method 2 include, but are not limited to thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂).

Sutiable solvents for Step 7 of Method 2 include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) and hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

The amino acid coupling of Step 7 of Method 2 may use N,N'dimethylpropyleneurea (DMPU) or hexamethylphosphoramide (HMPA) in combination with thionyl chloride.

Preferably, the reagents for the amino acid coupling of Step 7 of Method 2 are selected to be thionyl chloride (SOCl₂) as coupling agent or coupling agent precursor, in either of DMPU and acetonitrile (CH₃CN), or HMPU and acetonitrile.

Also encompassed in the present invention is a method of synthesising compound (I) from 1-(6-(2-Amino-5-(3-methylpyridin-4-yl)-1H-imidazol-4-yl)-2,3-dihydro-4H-benzo[b][1,4]oxazin-4-yl)ethan-1-one (11), i.e. Steps 6 and 7 of Method 2: 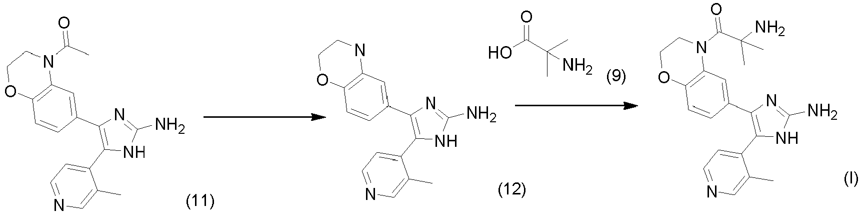 wherein the method comprises deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12), and amino acid coupling of 4-(3,4-Dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor to form compound (I).

The deacylation of Step 6 of Method 2 may be base or acid catalysed, preferably acid catalysed. Suitable acid catalysts for the deacylation of Step 6 of Method 2 include, but are not limited to: HCl such as 6.0 N HCl, whilst suitable base catalysts include, but are not limited to: NaOH such as 2.0 M NaOH. Preferably, the deacylation is acid catalysed using HCl, preferably 6.0 N HCl, and preferably in the presence of methanol (MeOH).

Suitable coupling agents or coupling agent precursors for Step 7 of Method 2 include, but are not limited to thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃. Preferably, the coupling agent or coupling agent precursor is thionyl chloride (SOCl₂).

Sutiable solvents for Step 7 of Method 2 include, but are not limited to: N,N'dimethylpropyleneurea (DMPU), dimethyl sulfoxide (DMSO), dimethylformamide (DMF), hexamethylphosphoramide (HMPA) and acetonitrile (ACN), or combinations thereof. The solvent may be selected from N,N'dimethylpropyleneurea (DMPU) and hexamethylphosphoramide (HMPA). Preferably, the solvent is selected from acetonitrile, N,N'dimethylpropyleneurea (DMPU) and hexamethylphosphoramide (HMPA), or combinations thereof. More preferably, the solvent is selected from acetonitrile and hexamethylphosphoramide (HMPA), or combinations thereof.

The amino acid coupling of Step 7 of Method 2 may use N,N'dimethylpropyleneurea (DMPU) or hexamethylphosphoramide (HMPA) in combination with thionyl chloride.

Preferably, the reagents for the amino acid coupling of Step 7 of Method 2 are selected to be thionyl chloride (SOCl₂) as coupling agent or coupling agent precursor, in either of DMPU and acetonitrile (CH₃CN), or HMPU and acetonitrile.

### Method 3

Compound (I) may be synthesised according to the following method:

### Step 1

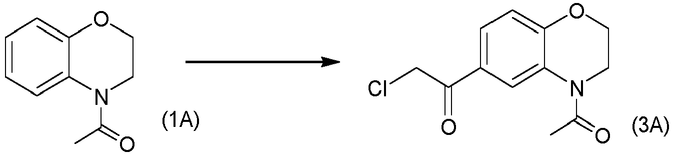

Acylation of 1-(2,3-dihydro-4H-benzo[b][1,4]oxazin-4-yl)ethan-1-one (1A) to form 1-(4-acetyl-3,4-dihydro-2H-benzo[b][1,4]oxazin-6-yl)-2-chloroethan-1-one (3A).

The acylation reaction of Step 1 may be a friedel crafts acylation. Suitable acylating agents for Step 1 include, but are not limited to: acyl halides such as acyl chlorides like acetyl chloride or chlorinated acyl chlorides like chloroacetyl chloride; and anhydrides of carboxylic acids. Preferably, the acylating agent is an acyl halide such as an acyl chloride, and more preferably chloroacetyl chloride.

Additional reagents may be utilised in the acylation of Step 1. The additional reagents may be selected from aluminium chloride, zinc chloride, boron trichloride, titanium chloride. Preferably, aluminium chloride is selected as an additional reagent.

The acylation of Step 1 takes place in a suitable solvent. The solvent for the acylation of Step 1 may be dichloromethane (DCM).

Preferably, the reagent used in the acylation of Step 1 is chloroacetyl chloride as an acylating agent in dichloromethane (DCM), with aluminium chloride (AlCl₃).

### Step 2

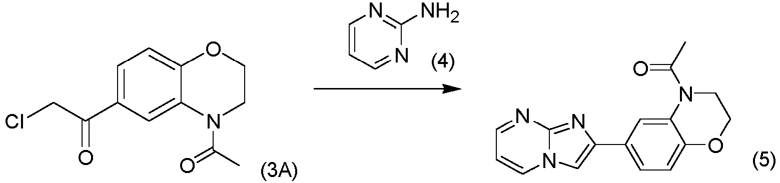

Cyclisation of 1-(4-acetyl-3,4-dihydro-2H-benzo[b][1,4]oxazin-6-yl)-2-chloroethan-1-one (3A) with pyrimidin-2-amine (4) to form 1-(6-(imidazo[1,2-a]pyrimidin-2-yl)-2,3-dihydro-4H-benzo[b][1,4]oxazin-4-yl)ethan-1-one (5).

Suitable solvents for use in the cyclisation of Step 2 include ethanol, methanol, 2-isopropyl alcohol (IPA), acetone and dichloromethane. Preferably, the solvent is selected to be ethanol.

### Step 3

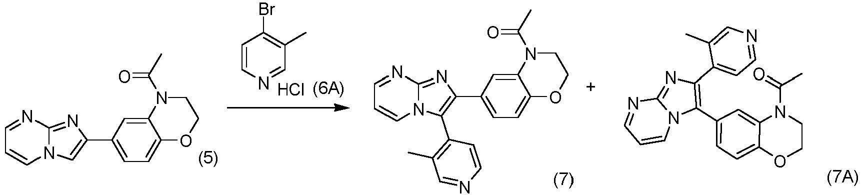

Coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine HCl (6A) in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A).

Suitable metal catalysts for the coupling reaction of Step 3 include, but are not limited to: transition metal-based catalysts such as platinum-based catalysts, palladium-based catalysts, and iridium-based catalysts. Typically, the transition metal-based catalysts are utilised in conjunction with a base. Suitable bases include, but are not limited to: potassium salts such as potassium acetate or potassium carbonate or caesium salts such as caesium carbonate. Preferably, the metal catalyst is a palladium-based catalyst, more preferably palladium (II) acetate. Palladium is particularly effective as catalyst in this coupling reaction. Preferably, the metal catalyst is a palladium-based catalyst utilised in conjunction with a base, more preferably a palladium-based catalyst utilised in conjunction with potassium carbonate, and more preferably palladium (II) acetate utilised in conjunction with potassium carbonate.

The coupling reaction of Step 3 may take place in the presence of a homogeneous catalytic system comprising pivalic acid, and tricyclohexylphosphine tetrafluoroborate ((C₆H₁₁)₃P.HBF₄) (ligand).

Suitable solvents for the coupling reaction of Step 3 include dimethylacetamide (DMA) sulfolane, N,N-dimethyl formamide (DMF), dimethyl carbonate, gamma-valerolactone and acetonitrile (ACN). Preferably, the solvent is selected from sulfolane and N-N dimethyl formamide (DMF). More preferably, the solvent is dimethyl formamide (DMF).

Such a coupling reaction may require control of the residual metal from the metal catalyst in the product-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A). This level of residual metal in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is preferably reduced as far as possible, preferably to less than 150 ppm, or less than 120 ppm, such as less than 100 ppm, or less than 80 ppm, or 50 ppm. Accordingly, only a trace level of residual metal may be present in compound (I), or a pharmaceutically acceptable salt thereof, when formed from -(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A).

In order to control the residual levels of metal present in the products of the coupling reaction 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) after use of a metal catalyst, a metal-scavenging treatment may be employed following production of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to reduce metal content. Suitable metal-scavenging treatments include, but are not limited to: charcoal absorption; absorption on different grades of silica columns or silica plug filtration; acidic treatments; and treatment with metal-scavenging agents such as trithiocyanuric acid, silamate thiol resin, neutral alumina, florocil, quadrasil, and 10% aqueous cysteine, or combinations thereof; and combinations thereof. Preferably, the metal-scavenging treatment comprises treatment with a metal-scavenging agent. Preferably, the metal-scavenging agent is trithiocyanuric acid.

When a metal-scavenging agent is utilised, it will be appreciated that the metal-scavenging agent may be used in excess with respect to the amount of metal of the metal catalyst used in the process. The level of residual metal is then preferably reduced as far as possible as discussed above.

Preferably, the metal catalyst is a palladium-based catalyst, more preferably palladium (II) acetate. Accordingly, the metal-scavenging treatment is preferably capable of scavenging palladium. Preferably, the metal-scavenging treatment comprises treatment with palladium-scavenging agent. Preferably, the palladium-scavenging agent is trithiocyanuric acid.

The treatment with trithiocyanuric acid as metal-scavenging agent may be carried out once or multiple times, preferably twice. Preferably, the metal-scavenging treatment comprises treatment with trithiocyanuric acid in addition to absorption on silica columns or silica plug filtration and charcoal absorption. More preferably, the metal-scavenging treatment comprises treatment with trithiocyanuric acid in addition to silica plug filtration, and charcoal absorption. Typically, the silica plug filtration is carried out first and may use 10% MeOH/DCM. Typically, the treatment with trithiocyanuric acid is carried out alongside the charcoal absorption. The treatment with trithiocyanuric acid may be carried out in solvent selected from dichloromethane, methanol, ethanol, IPA and water, or combinations thereof. For this process, the metal being scavenged is preferably palladium, as a palladium catalyst is preferably utilised.

Solvents such as dichloromethane, methanol, ethanol, IPA and water, or combinations thereof, may be used during the metal-scavenging treatment.

Following metal-scavenging, the level of metal, preferably palladium, in the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) may be less than 150 ppm, or less than 120 ppm, such as less than 100 ppm, or less than 80 ppm, or 50 ppm. When the product 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) is subsequently utilised in further reactions such as those set out in relation to the first or fourth aspects of the present invention, the level of metal, preferably palladium, in compound (I), or a pharmaceutically acceptable salt thereof, may be less than 10 ppm, such as less than 5 ppm. Such an amount is considered an acceptable trace amount.

The level of residual metal in compound (I), or other intermediates of the production process such as such as 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), may be measured by inductively coupled plasma mass spectrometry (ICP-MS).

Preferably, the coupling reaction takes place under an argon atmosphere.

Preferably, the reagents used for the coupling reaction of Step 3 are selected to be palladium (II) acetate (Pd(OAc)₂) and potassium carbonate (K₂CO₃) in the presence of pivalic acid, tricyclohexylphosphine tetrafluoroborate ((C₆H₁₁)₃P.HBF₄), and N,N-dimethyl formamide (DMF). Preferably, the metal-scavenging agent is trithiocyanuric acid.

### Step 4

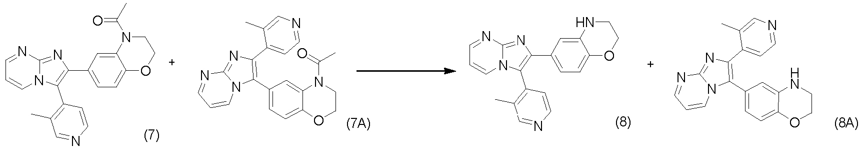

Deacylation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to form 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A).

The deacylation of Step 4 may be acid or base catalysed, preferably base catalysed. Suitable acid catalysts for the deacylation include, but are not limited to: HCl such as 6.0 N HCl, whilst suitable base catalysts include, but are not limited to: NaOH. The NaOH may be used in either organic or aqueous forms, such as 2.0 M NaOH or 30% NaOH solution. Preferably, the deacylation is base catalysed using NaOH, more preferably 30% NaOH solution.

Preferably, the deacylation of Step 4 is carried out in a solvent such as methanol.

Preferably, the deacylation of Step 4 is carried out using a base catalyst, preferably, NaOH, and more preferably 30% aq. NaOH, in methanol.

Preferably, the deacylation of Step 4 is carried out at a temperature of from 50 to 80 °C, such as from 60 to 80 °C, or from 60 to 75 °C. The deacylation is preferably carried out for a duration of 4 to 8 hours, such as 5 to 6 hours.

### Step 5

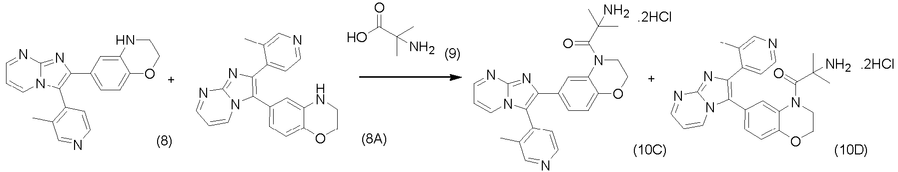

Amino acid coupling of 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A) with 2-amino-2-methylpropanoic acid (9) in the presence of a coupling agent or coupling agent precursor to form a dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or a dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one.

Suitable coupling agents or coupling agent precursors for Step 5 include, but are not limited to, thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), (1-cyano-2-ethoxy-2-oxoethylidenaminooxy)dimethylamino-morpholino-carbenium hexafluorophosphate (COMU), hydroxybenotriazole, fluoro-dipyrrolidinocarbenium hexafluorophosphate (BTFFH), Ti(iOPr)₄ and B(OCH₂CF₃)₃. Preferably, the coupling agent or coupling agent precursor is selected from hydroxybenotriazole, 1-ethyl-2-)3'-dimethylaminopropyl)carbodiimide.HCl, 1,1'-carbonyldiimidazole (CDI), (1-cyano-2-ethoxy-2-oxoethylidenaminooxy)dimethylamino-morpholino-carbenium hexafluorophosphate (COMU), propanephosphonic acid anhydride (T3P), and thionyl chloride (SOCl₂). More preferably, the coupling agent or coupling agent precursor is thionyl chloride.

Sutiable solvents for step 5 include, but are not limited to: N,N'dimethylpropyleneurea (DMPU), dimethyl sulfoxide (DMSO), dimethylformamide (DMF), hexamethylphosphoramide (HMPA), acetonitrile (ACN), N-methyl-2-pyrolidone (NMP), 1,3-dimethyl-2-imidazolidinone (DMI), gamma-valerolactone (GVL), N-methylmorpholine (NMM), sulfolane, dichloromethane (DCM), or combinations thereof. Preferably, the solvent is selected from N-methyl-2-pyrolidone (NMP), 1,3-dimethyl-2-imidazolidinone (DMI), gamma-valerolactone (GVL), N-methylmorpholine (NMM), sulfolane, dichloromethane (DCM), and acetonitrile, or combinations thereof. More preferably, the solvent is selected from acetonitrile or sulfolane, most preferably acetonitrile.

Preferably, the reaction of Step 5 is carried out in the presence of a base, such as pyridine or other tertiary amines, preferably pyridine.

Preferably, in Step 5, the coupling agent or coupling agent precursor is thionyl chloride and the solvent is acetonitrile or sulfolane, preferably acetonitrile, and the reaction is carried out in pyridine.

### Step 6

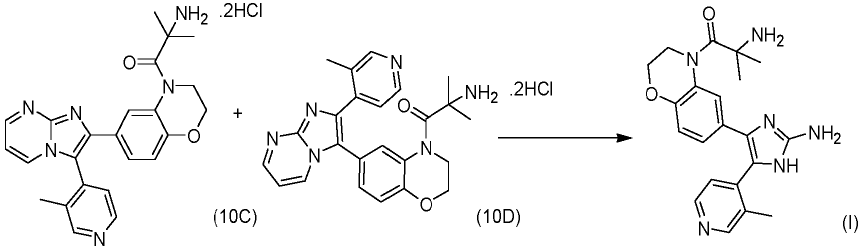

Deprotection of the dihydrochloride salt (10C) of 1-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one to form compound (I).

The deprotection of Step 6 may be base or acid catalysed, preferably base catalysed.

Suitable deprotection reagents for the deprotection include, but are not limited to: NH₂OH.HCl (hydroxylamine hydrochloride), and NH₂NH₂ (hydrazine) or the hydrate thereof. Preferably, the deprotection reagent is NH₂OH.HCl (hydroxylamine hydrochloride). The deprotection reagent may be accompanied by a base. Suitable bases include 1,8-diazabicyclo(5.4.0)undec-7-ene (DBU), N,N-diisopropylethylamine (DIPEA), piperidine, morpholine, pyrollidine, and triethylamine (TEA). Preferably, the base is selected from 1,8-diazabicyclo(5.4.0)undec-7-ene (DBU), N,N-diisopropylethylamine (DIPEA) and triethylamine (TEA). More preferably, the base is selected to be triethylamine (TEA).

The solvent for the deprotection of Step 6 may be selected from any suitable solvent, for example, isopropyl alcohol (IPA), ethanol (EtOH), dichloromethane (DCM), methanol (MeOH), and acetonitrile (CH₃CN). Preferably, the solvent is selected from methanol, dichloromethane and isopropyl alcohol (IPA). More preferably the solvent is isopropyl alcohol (IPA).

The deprotection of Step 6 may be carried out at a temperature of from 30 to 80 °C, such as from 40 to 70 °C, preferably 40 to 60 °C.

Preferably, the reagent for the deprotection of Step 6 is NH₂OH.HCl (hydroxylamine hydrochloride), with triethylamine (TEA) as base, in isopropyl alcohol (IPA).

It will be appreciated that when NH₂OH.HCl (hydroxylamine hydrochloride) is utilised, the deprotection may result in a dihydrochloride salt of compound (I).

Compound (I) may be produced through treatment of the dihydrochloride salt of compound (I) under basic conditions. Compound (I) may be formed through the treatment of the dihydrochloride salt of compound (I) with: an ion-exchange resin such as Amberlite IRN78 and MP-carbonate; addition of base such as potassium carbonate; addition of ammonia in a solvent such as methanol or isopropyl alcohol (IPA); or addition of ammonium hydroxide (NH₄OH). Preferably, compound (I) is formed and isolated through treatment of the dihydrochloride salt of compound (I) with ammonium hydroxide (NH₄OH). Optionally, prior to addition of ammonium hydroxide, the dihydrochloride salt of compound (I) may be neutralised in water. Optionally, dichloromethane may be introduced to aid formation of compound (I).

Preferably, the compound (I) according to the present invention is synthesised according to one of the following Methods 4, 5 and 6. As for Methods 1, 2 and 3, the Methods 4, 5 and 6 represent preferred methods for the synthesis of compound (I), said methods comprising the preferred connected steps detailed below. Preferably, compound (I) is produced according to Method 6. Method 6 constitutes the best mode currently contemplated for practising the invention. It will however be appreciated that, in the context of the present invention, these preferred steps are separable, and one or more of the steps may be substituted with any other suitable step identified by a skilled person. For Methods 4 and 5, where a Step X has an A and B option, for example Step 6A or Step 6B, either of Step XA or XB may be utilised as Step X, i.e. either of Step 6A or 6B may be utilised as Step 6.

As shown in Method 3 above and Method 6 below, compound (I), or a pharmaceutically acceptable salt thereof, may be formed from 1-(2,3-dihydro-4H-benzo[b][1,4]oxazin-4-yl)ethan-1-one (1A), a commercially available compound.

Accordingly, also incorporated herein is a process for the production of compound (I), or a pharmaceutically acceptable salt thereof, starting from 1-(2,3-dihydro-4H-benzo[b][1,4]oxazin-4-yl)ethan-1-one (1A):

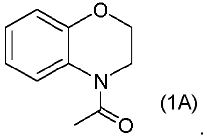

### Method 4

Compound (I) may be synthesised according to the following method:

### Step 1

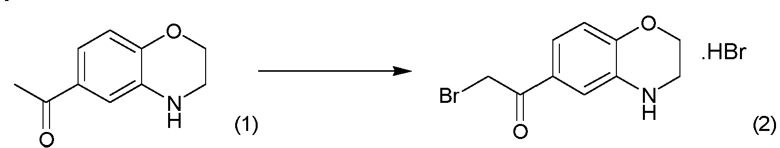

A 20 L four-necked round bottom flask equipped with mechanical stirrer was charged with 1-(3,4-dihydro-2H-1,4-benzoxazin-6-yl)ethan-1-one (1) (300 g, 1.69 mol) at room temperature. A solution of HBr in glacial acetic acid (33% w/w, 2.10 L, 7 V) was added dropwise at 10°C ideally maintaining the internal temperature below ~12°C. To the resulting reaction mixture was added a solution of bromine (86.7 mL, 1.69 mol) in glacial acetic acid (450 mL, 1.5 V with respect to starting material) at 10°C (internal temperature) slowly (~ over 90 min) such that internal temperature was maintained below 10°C. The reaction was monitored by LCMS. Following complete addition of a solution of bromine in glacial acetic, the crude LCMS confirmed ~10% unconsumed starting material (1-(3,4-dihydro-2H-1,4-benzoxazin-6-yl)ethan-1-one (1)) and ~30% conversion to desired mono-brominated product (2) along with ~24% di-brominated by-product. The cooling bath was removed and the reaction mixture was gradually warmed to 25°C (internal temperature) over approximately 1h. The resulting reaction mixture was further stirred at 40°C (internal temperature) for 1.5 h [visible precipitation was observed, the crude LCMS confirmed unconsumed starting material (1) (~4%) along with desired mono-brominated product (2) (55%) and di-brominated by-product (~18%)]. The reaction mixture was allowed to cool to 10 °C, diluted with MTBE (4.9 L, 16 V) and stirred for 1h. The resulting precipitate was filtered, washed with MTBE (600 mL) and dried under reduced pressure to afford 340 g (mixture of mono and dibrominated product) as an off white solid. The LCMS of obtained solid confirmed a mixture of 2-bromo-1-(3,4-dihydro-2H-1,4-benzoxazin-6-yl)ethan-1-one.HBr (2) (~87%, mono brominated), 2,2-dibromo-1-(3,4-dihydro-2H-benzo[b][1,4]oxazin-6-yl)ethan-1-one (~5%, dibrominated by-product) and unconsumed 1-(3,4-dihydro-2H-1,4-benzoxazin-6-yl)ethan-1-one (1) (~1%). This mixture was used in the next step without further purification. The isolated yield calculated on the basis of mono brominated product (2) observed by LCMS was found to be 52%. MS (ESI+) for CHNOS m/z 255.96 [M+H]⁺, ¹H NMR (400 MHz, DMSO-d6): *δ* 7.19-7.28 (m, 2H), 6.77 (d, *J =* 8.4 Hz, 1H), 4.74 (s, 2H), 4.31 (t, *J* = 4.4 Hz, 2H) and 3.32 (t, *J* = 4.4 Hz, 2H).

### Step 2

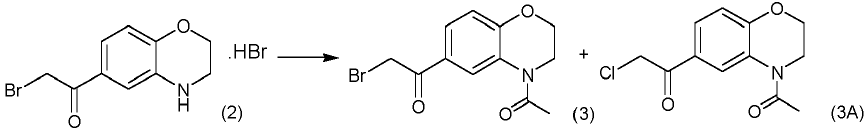

A 10 L four-necked round bottom flask equipped with mechanical stirrer was charged with THF (3.50 L, 7V) and 2-bromo-1-(3,4-dihydro-2H-1,4-benzoxazin-6-yl)ethan-1-one.HBr (2) (500 g, 1.48 mol) at 20°C. To the resulting reaction mixture was added acetyl chloride (476 mL, 6.68 mol) dropwise maintaining the temperature at 20°C. A 2°C increase in the reaction temperature was observed, during the addition of acetyl chloride to the reaction mixture. The reaction mixture was further stirred at 30°C for 16h. The reaction was monitored by LCMS. The crude LCMS confirmed formation of mixture of 1-(4-acetyl-3,4-dihydro-2H-benzo[b][1,4]oxazin-6-yl)-2-bromoethan-1-one & 1-(4-acetyl-3,4-dihydro-2H-benzo[b][1,4]oxazin-6-yl)-2-chloroethan-1-one in 1:1 ratio . The reaction mixture was cooled to ~10° C and basified to pH ~8.0 with 10% aqueous solution of K₂CO₃ (5.0 L) and extracted with ethyl acetate (3 X 5L). The combined organic layer was washed with brine (5.0 L), and concentrated under reduce pressure to afford crude product as a brown liquid. The brown liquid was cooled to 10°C, diluted with hexane (4.0 L) and stirred at 10°C for 1h. The resulting precipitate was filtered, washed with hexane (1.0 L) and dried under vacuum to afford a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (4) as a light brown solid (387 g, 92% pure by LCMS), which was used in next step without further purification. MS (ESI+) for CHNOS *m*/*z* 297.99 [M+H]⁺ & 254.05 [M+H]⁺.

### Step 3

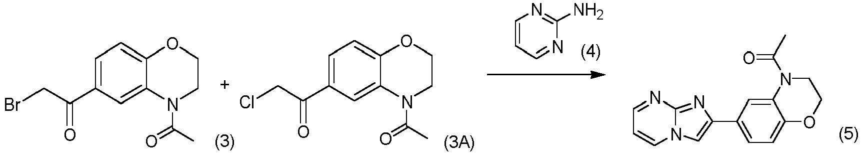

A 5.0 L four-necked round bottom flask equipped with mechanical stirrer was charged with EtOH (2.0 L, 3 V), a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A) (685 g, 2.30 mol, 92% pure by LCMS) followed by addition of pyrimidin-2-amine (4) (546 g, 5.74 mol) at 20°C. The reaction mixture was further stirred and refluxed for 3h. The reaction was monitored with LCMS. The reaction mixture was allowed to cool to room temperature (~ 20°C) and the resulting precipitated solid was filtered, washed with EtOH (2 X 250 mL) and dried under vacuum to afford 1 -(6-(imidazo[1,2-a]pyrimidin-2-yl)-2,3-dihydro-4H-benzo[b][1,4]oxazin-4-yl)ethan-1-one (5) as an off white solid (418 g). MS (ESI+) for CHNOS *m*/*z* 295.06 [M+H] ⁺. ¹H NMR (400 MHz, DMSO-d6 + d-TFA): *δ* 9.27 (d, *J =* 6.40 Hz, 1H), 8.97 -9.02 (m, 1H), 8.65 (s, 1H), 8.44 (bs, 1H), 7.63-7.69 (m, 2H), 7.10 (d, *J =* 8.40 Hz, 1H), 4.33-4.36 (m, 2H), 3.89-3.93 (m, 2H), 2.32 (s, 3H). The regioisomeric structure was confirmed by nOe.

### Step 4

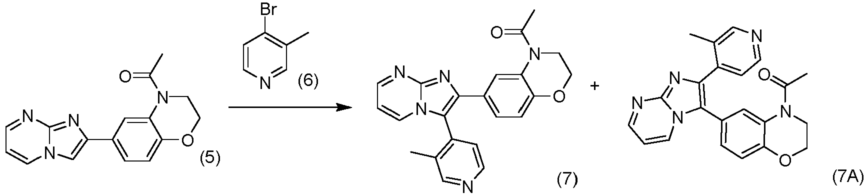

A 10 L four-necked round bottom flask equipped with mechanical stirrer was charged with N,N-dimethylacetamide (2.0 L), 1-(6-(imidazo[1,2-a]pyrimidin-2-yl)-2,3-dihydro-4H-benzo[b][1,4]oxazin-4-yl)ethan-1-one (5) (250 g, 849 mmol), 2,2-dimethylpropanoic acid (34.7 g, 340 mmol), potassium carbonate (587 g, 4.25 mol) and 4-bromo-3-methylpyridine (6) (264 g, 1.44 mol) at 20° C under N₂ atmosphere. The resulting reaction mixture was purged with N₂ (g) for 30 min followed by addition of Pd(OAc)₂ (19.1 g, 84.9 mmol) and PCy₃·HBF₄ (31.2 g, 84.9 mmol) under N₂ atmosphere. The reaction mixture was again purged with N₂ (g) for additional 15 min. The resulting reaction mixture was further stirred at 125°C for 7h. The reaction was monitored with LCMS. The crude LCMS confirmed the formation of regioisomeric mixture of desired product in 98:2 regioisomeric ratio. The reaction mixture was filtered through celite bed (1.3 cm height & 25 cm diameter). The celite bed was washed with 10% MeOH in DCM (15 L). The filtrate was evaporated under reduced pressure to afford the crude residue as a brown liquid. The brown liquid was stirred in heptane (3 x 6.0 L) for 10 h to remove excess of DMA. The heptane/DMA mixture was decanted (traces of desired product was observed in the decanted fraction) to afford waxy solid. The waxy solid was further stirred in MTBE (3.0 L) for 15 min. The resulting precipitate was filtered and washed with MTBE (2.0 L) to afford the desired product as a brown solid. The brown solid was passed through a small silica plug eluting with 10% MeOH in DCM (~35 L). The obtained solvent fraction was concentrated to 1/10 of its original volume. The concentrated fraction was diluted with MTBE (200 mL) and stirred for 30 min. The resulting precipitate was filtered, washed with MTBE (1.0 L) and dried under vacuum to afford regioisomeric mixture of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (220 g) as a light brown solid. Yield: 66% (97% by LCMS, 98:2 regioisomeric ratio). MS (ESI+) for CHNOS m/z 386.18 [M+1]⁺. 1H NMR (400 MHz, DMSO-d6 + d-TFA): *δ* 9.14 (s, 1H), 9.01-9.04 (m, 2H), 8.86 (d, *J* = 6.7 Hz, 1H), 8.23 (d, *J =* 5.8 Hz, 1H), 8.01 (bs, 1H), 7.55-7.58 (m, 1H), 7.41 (d, *J =* 8.3 Hz, 1H), 7.05 (d, *J* = 8.6 Hz, 1H), 4.09-4.33 (m, 2H), 3.71-3.92 (m, 2H), 2.17 (s, 3H), 2.11 (bs, 3H).

### Step 5A

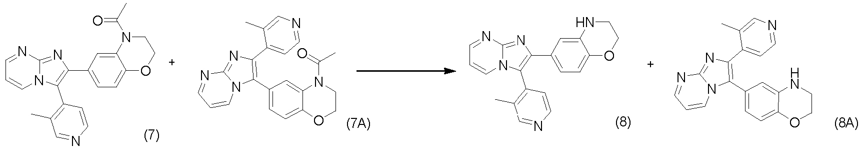

To a solution of a regioisomeric mixture (97:3) of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (24.0 g, 62.3 mmol) in methanol (150 mL) was added 6.0 N aqueous HCl (62 mL) at rt. The reaction mixture was stirred at 90°C for 16 h. The reaction mixture was allowed to cool to room temperature and concentrated to 1/4th of its original volume. The concentrated reaction mixture was basified to pH 8-9 with saturated aq. NaHCO₃ solution and extracted with 10% MeOH in DCM (3 x 250 mL). The organic layer was washed with brine (300 mL), dried over (Na₂SO₄), filtered and concentrated to 1/10th of its original volume under reduced pressure. The concentrated mixture was diluted with heptane (100 mL) and stirred for 30 min. The resulting precipitate was filtered, washed with heptane (100 mL) and dried under reduced pressure to obtain mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (20.0 g) as a yellow solid. Yield: 71% (mixture of two regioisomers in 2:1 regioisomeric ratio, 89% by LCMS). The LCMS showed two peaks with desired mass 56% and 33% respectively. (ESI+) for CHNOS *m*/*z* 344.20 [M+H]⁺.

### Or Step 5B

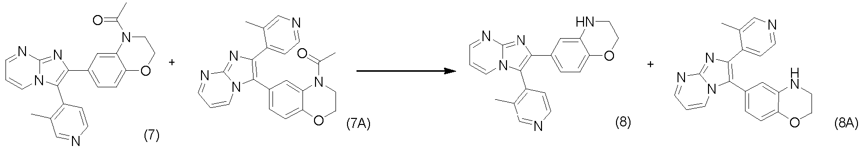

A suspension of a regioisomeric mixture (98:2) of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (10.0 g, 25.9 mmol) in 2.0 M aq. solution of NaOH (52 mL, 104 mmol) was heated at 100°C for 24 h. The reaction was monitored with the LCMS. After completion of the reaction, the reaction mixture was allowed to cool to room temperature and filtered. The filtered cake was washed with the water (~500 mL), dried to obtain mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) as a light brown solid. Yield: 8.1 g, 90% (mixture of two regioisomers in 98:2 regioisomeric ratio, 98.7% by LCMS). MS (ESI+) for CHNOS *m*/*z* 344.20 [M+H]⁺, ¹H NMR (400 MHz, DMSO-d₆): *δ* 8.69 (s, 1H), 8.54-8.62 (m, 2H), 8.25 (d, *J =* 6.8 Hz, 1H), 7.46 (d, *J =* 4.8 Hz, 1H), 6.96-7.03 (m, 2H), 6.46-6.55 (m, 2H), 5.87 (s, 1H), 4.11 (t, *J* = 4.0 Hz, 2H), 3.25 (bs, 2H), 1.94 (s, 3H).

### Step 6A (HMPA mediated amino acid coupling)

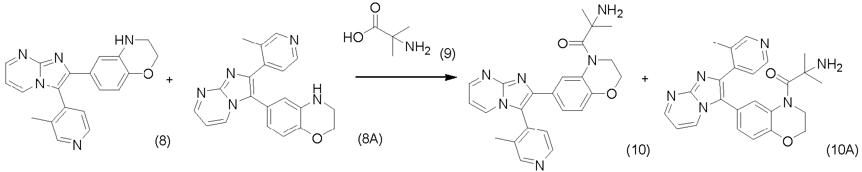

To a suspension of 2-amino-2-methylpropanoic acid (9) (12 g, 116 mmol) in HMPA (84 mL, 7 V) was added a solution of thionyl chloride (9.29 g, 128 mmol) in ACN (8.4 mL) slowly at 3°C (outer temperature was 0°C). The suspension becomes clear after 10 min of the complete addition of solution of thionyl chloride in ACN. The resulting reaction mixture was stirred at 0°C for 20 min followed by portion wise addition of a regioisomeric mixture of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (8 g, 23.3 mmol) at 0°C. The reaction mixture was allowed to warm to room temperature and stirred for 16 h. The reaction was monitored by LCMS. After the completion of the reaction, the reaction mixture was diluted with EtOAc (300 mL). The resulting precipitate was filtered and washed with EtOAc (250 mL). The solid was dissolved in water (50 mL) and neutralized with saturated sodium bicarbonate solution up to the pH of 8 and extracted with 10% MeOH in DCM (4 X 100 mL), concentrated under reduced pressure to 1/10th of original reaction mixture volume. The crude mixture was diluted with MTBE (50 mL) and stirred for 15 min. The resulting precipitate was filtered, washed with MTBE (25 mL) and dried under vacuum to afford regioisomeric mixture of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (9.1 g) as a brown solid. Yield: 66% (97% by LCMS, 98:2 regioisomeric ratio). MS (ESI+) for CHNOS m/z 429.16 [M+1]⁺ ; ¹H NMR (400 MHz, DMSO-d₆): *δ* 8.69 (s, 1H), 8.54-8.62 (m, 2H), 8.29 (d, *J* = 5.2 Hz, 1H), 8.12 (d, *J =* 1.6 Hz, 1H), 7.46 (d, *J* = 4.8 Hz, 1H), 6.98-7.05 (m, 2H), 6.77 (d, *J* = 8.4 Hz, 1H), 4.45- 4.67 (m, 2H), 4.30 (bs, 2H), 2.12 (bs, 2H), 1.97 (s, 3H), 1.34 (s, 6H).

### Or Step 6B (DMPU mediated amino acid coupling)

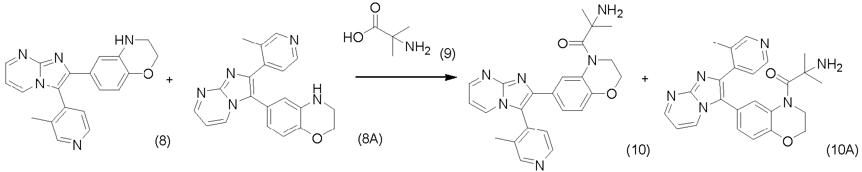

To a solution of 2-amino-2-methylpropanoic acid (9) (2.0 g, 19.4 mmol) in DMPU (14 mL) was added a solution of thionyl chloride (1.55 mL, 21.3 mmol) in ACN (1.4 mL) slowly at 4°C (outer temperature was 0°C). The resulting reaction mixture was stirred at 0°C for 20 min followed by portion wise addition of a regioisomeric mixture (98:1) of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (1.33 g, 3.88 mmol) at 0°C. The reaction mixture was allowed to warm to room temperature and stirred for 16 h. After the completion of the reaction (LCMS monitoring), the reaction mixture was diluted with EtOAc (50 mL). The resulting precipitate was filtered and washed with EtOAc (50 mL). The solid was dissolved in water (100 mL), basified to pH ~8.0 with saturated sodium bicarbonate solution and extracted with 10% MeOH in DCM (3 X 100 mL), dried (Na₂SO₄) and concentrated under reduced pressure to obtain the crude residue. The crude residue was further stirred in the MTBE (20 mL), filtered, washed with MTBE (20 mL) and dried under vacuum to afford regioisomeric mixture of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (1.3 g, 98.7% by LCMS, 98:2 regioisomeric ratio) as a brown solid, which was further used in next step without further purification. MS (ESI+) for CHNOS m/z 429.16 [M+1]⁺; ¹H NMR (400 MHz, DMSO-d₆): *δ* 8.69 (s, 1H), 8.54-8.62 (m, 2H), 8.29 (d, *J =* 5.2 Hz, 1H), 8.12 (d, *J* = 1.6 Hz, 1H), 7.46 (d, *J* = 4.8 Hz, 1H), 6.98-7.05 (m, 2H), 6.77 (d, *J* = 8.4 Hz, 1H), 4.45- 4.67 (m, 2H), 4.30 (bs, 2H), 2.12 (bs, 2H), 1.97 (s, 3H), 1.34 (s, 6H).

### Step 7

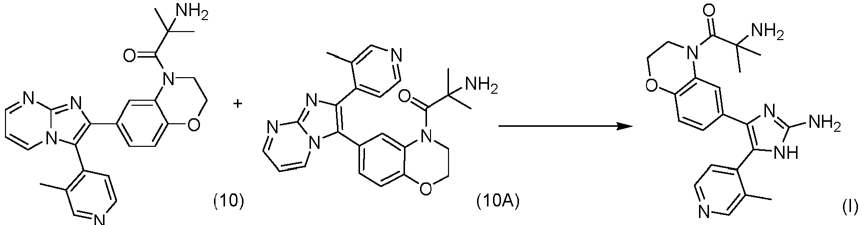

To a suspension of a regioisomeric mixture (98:2) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) (8.6 g, 93% by LCMS, 20.1 mmol) in isopropyl alcohol (60 mL) was added hydroxylamine hydrochloride (3.49 g, 50.2 mmol) at room temperature. The resulting suspension was stirred at 70°C for 22h. The reaction was monitored by LCMS. The crude LCMS showed formation of desired product along with the traces of de-amidated by-product (1-2% by crude LCMS) and unreacted starting material (2-3% by LCMS). The resulting suspension was allowed to cool to room temperature, diluted with acetone (100 mL) and stirred for 1h. The reaction mixture was concentrated to 20% of original volume and precipitation was filtered, washed with acetone (200 mL) and dried under vacuum to afford crude compound (I) as an HCl salt. The obtained solid was dissolved in water (10 mL), basified up to pH-8 by saturated solution of NaHCO₃ and extracted with 10% MeOH in DCM (5 x 100 mL). The combined organic layer was dried over Na₂SO₄, filtered and concentrated under vacuum to obtain the crude residue. The crude residue was stirred in the EtOH (10 mL). The crude product solubilizes in EtOH and after stirring for 1-2 h, again precipitation was observed. The resulting precipitate was filtered and washed with EtOH (10 mL). The precipitate thus obtained was again stirred in EtOH (10 mL) , filtered, followed by the washing with 2% MeOH in DCM (15 mL) and dried under vacuum to afford 2-amino-1-(6-(2-amino-5-(3-methylpyridin-4-yl)-1H-imidazol-4-yl)-2,3-dihydro-4H-benzo[b][1,4]oxazin-4-yl)-2-methylpropan-1-one (compound (I)) as a light yellow fluffy solid. Yield: 3.5 g, 44% (98.3% by LCMS). MS (ESI+) for CHNOS m/z 392.8 [M+H]⁺. ¹H NMR (400 MHz, DMSO*-d₆* + d-TFA): *δ* 8.78-8.84 (m, 2H), 7.93 (d, *J* = 6.1 Hz, 1 H), 7.44 (d, *J* = 2.1 Hz, 1 H), 7.09 (dd, *J* = 2.1, 8.6 Hz, 1 H), 6.97 (d, *J* = 8.6 Hz, 1 H), 4.34 (bs, 2H), 3.97 (bs, 2H), 2.16 (s, 3H), 1.60 (s, 6H).

For Method 4, preferably Step 5B is selected.

### Method 5

Compound (I) may be synthesised according to the following method:

### Step 1

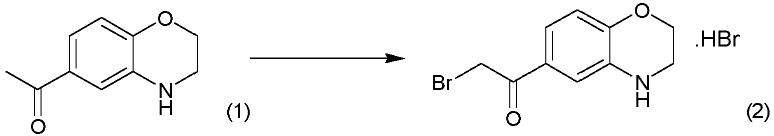

A 20 L four-necked round bottom flask equipped with mechanical stirrer was charged with 1-(3,4-dihydro-2H-1,4-benzoxazin-6-yl)ethan-1-one (1) (300 g, 1.69 mol) at room temperature. A solution of HBr in glacial acetic acid (33% w/w, 2.10 L, 7 V) was added dropwise at 10°C ideally maintaining the internal temperature below ~12°C. To the resulting reaction mixture was added a solution of bromine (86.7 mL, 1.69 mol) in glacial acetic acid (450 mL, 1.5 V with respect to starting material) at 10°C (internal temperature) slowly (~ over 90 min) such that internal temperature was maintained below 10°C. The reaction was monitored by LCMS. Following complete addition of a solution of bromine in glacial acetic, the crude LCMS confirmed ~10% unconsumed starting material (1-(3,4-dihydro-2H-1,4-benzoxazin-6-yl)ethan-1-one (1)) and ~30% conversion to desired mono-brominated product (2) along with ~24% di-brominated by-product. The cooling bath was removed and the reaction mixture was gradually warmed to 25°C (internal temperature) over approximately 1h. The resulting reaction mixture was further stirred at 40°C (internal temperature) for 1.5 h [visible precipitation was observed, the crude LCMS confirmed unconsumed starting material (1) (~4%) along with desired mono-brominated product (2) (55%) and di-brominated by-product (~18%)]. The reaction mixture was allowed to cool to 10 °C, diluted with MTBE (4.9 L, 16 V) and stirred for 1h. The resulting precipitate was filtered, washed with MTBE (600 mL) and dried under reduced pressure to afford 340 g (mixture of mono and dibrominated product) as an off white solid. The LCMS of obtained solid confirmed a mixture of 2-bromo-1-(3,4-dihydro-2H-1,4-benzoxazin-6-yl)ethan-1-one.HBr (2) (~87%, mono brominated), 2,2-dibromo-1-(3,4-dihydro-2H-benzo[b][1,4]oxazin-6-yl)ethan-1-one (~5%, dibrominated by-product) and unconsumed 1-(3,4-dihydro-2H-1,4-benzoxazin-6-yl)ethan-1-one (1) (~1%). This mixture was used in the next step without further purification. The isolated yield calculated on the basis of mono brominated product (2) observed by LCMS was found to be 52%. MS (ESI+) for CHNOS m/z 255.96 [M+H]⁺, ¹H NMR (400 MHz, DMSO-d6): *δ* 7.19-7.28 (m, 2H), 6.77 (d, *J =* 8.4 Hz, 1H), 4.74 (s, 2H), 4.31 (t, *J* = 4.4 Hz, 2H) and 3.32 (t, *J* = 4.4 Hz, 2H).

### Step 2

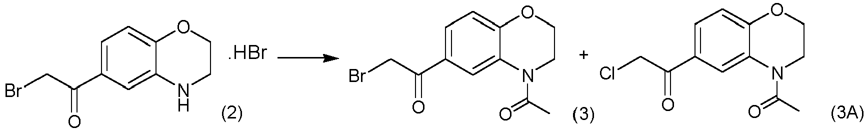

A 10 L four-necked round bottom flask equipped with mechanical stirrer was charged with THF (3.50 L, 7V) and 2-bromo-1-(3,4-dihydro-2H-1,4-benzoxazin-6-yl)ethan-1-one.HBr (2) (~5%) (500 g, 1.48 mol) at 20°C. To the resulting reaction mixture was added acetyl chloride (476 mL, 6.68 mol) dropwise maintaining the temperature at 20°C. A 2°C increase in the reaction temperature was observed, during the addition of acetyl chloride to the reaction mixture. The reaction mixture was further stirred at 30°C for 16h. The reaction was monitored by LCMS. The crude LCMS confirmed formation of mixture of 1-(4-acetyl-3,4-dihydro-2H-benzo[b][1,4]oxazin-6-yl)-2-bromoethan-1-one & 1-(4-acetyl-3,4-dihydro-2H-benzo[b][1,4]oxazin-6-yl)-2-chloroethan-1-one in 1:1 ratio . The reaction mixture was cooled to ~10° C and basified to pH ~8.0 with 10% aqueous solution of K₂CO₃ (5.0 L) and extracted with ethyl acetate (3 X 5L). The combined organic layer was washed with brine (5.0 L), and concentrated under reduce pressure to afford crude product as a brown liquid. The brown liquid was cooled to 10°C, diluted with hexane (4.0 L) and stirred at 10°C for 1h. The resulting precipitate was filtered, washed with hexane (1.0 L) and dried under vacuum to afford a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (4) as a light brown solid (387 g, 92% pure by LCMS), which was used in next step without further purification. MS (ESI+) for CHNOS *m*/*z* 297.99 [M+H]⁺ & 254.05 [M+H]⁺.

### Step 3

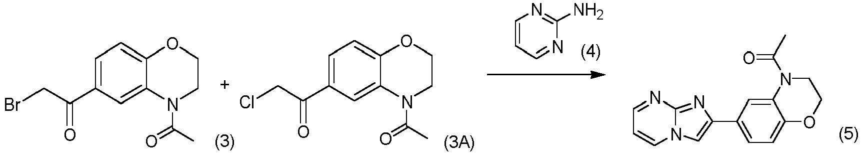

A 5.0 L four-necked round bottom flask equipped with mechanical stirrer was charged with EtOH (2.0 L, 3 V), a mixture of 1-(4-acetyl-3,4-dihydro-2H-benzo[b][1,4]oxazin-6-yl)-2-bromoethan-1-one (3) & 1-(4-acetyl-3,4-dihydro-2H-benzo[b][1,4]oxazin-6-yl)-2-chloroethan-1-one (3A) (685 g, 2.30 mol, 92% pure by LCMS) followed by addition of pyrimidin-2-amine (4) (546 g, 5.74 mol) at 20°C. The reaction mixture was further stirred and refluxed for 3h. The reaction was monitored with LCMS. The reaction mixture was allowed to cool to room temperature (~ 20°C) and the resulting precipitated solid was filtered, washed with EtOH (2 X 250 mL) and dried under vacuum to afford 1 -(6-(imidazo[1,2-a]pyrimidin-2-yl)-2,3-dihydro-4H-benzo[b][1,4]oxazin-4-yl)ethan-1-one (5) as an off white solid (418 g). MS (ESI+) for CHNOS *m*/*z* 295.06 [M+H]⁺. ¹H NMR (400 MHz, DMSO-d6 + d-TFA): *δ* 9.27 (d, *J* = 6.40 Hz, 1H), 8.97 -9.02 (m, 1H), 8.65 (s, 1H), 8.44 (bs, 1H), 7.63-7.69 (m, 2H), 7.10 (d, *J =* 8.40 Hz, 1H), 4.33-4.36 (m, 2H), 3.89-3.93 (m, 2H), 2.32 (s, 3H). The regioisomeric structure was confirmed by nOe.

### Step 4

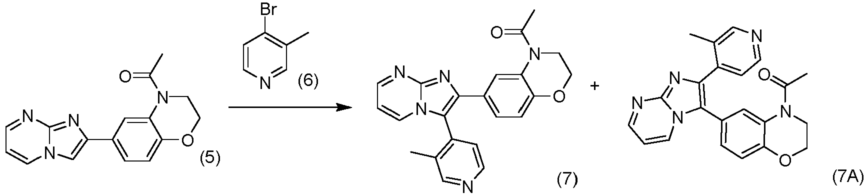

A 10 L four-necked round bottom flask equipped with mechanical stirrer was charged with N,N-dimethylacetamide (2.0 L), 1-(6-(imidazo[1,2-a]pyrimidin-2-yl)-2,3-dihydro-4H-benzo[b][1,4]oxazin-4-yl)ethan-1-one (5) (250 g, 849 mmol), 2,2-dimethylpropanoic acid (34.7 g, 340 mmol), potassium carbonate (587 g, 4.25 mol) and 4-bromo-3-methylpyridine (6) (264 g, 1.44 mol) at 20° C under N₂ atmosphere. The resulting reaction mixture was purged with N₂ (g) for 30 min followed by addition of Pd(OAc)₂ (19.1 g, 84.9 mmol) and PCy₃·HBF₄ (31.2 g, 84.9 mmol) under N₂ atmosphere. The reaction mixture was again purged with N₂ (g) for additional 15 min. The resulting reaction mixture was further stirred at 125°C for 7h. The reaction was monitored with LCMS. The crude LCMS confirmed the formation of regioisomeric mixture of desired product in 98:2 regioisomeric ratio. The reaction mixture was filtered through celite bed (1.3 cm height & 25 cm diameter). The celite bed was washed with 10% MeOH in DCM (15 L). The filtrate was evaporated under reduced pressure to afford the crude residue as a brown liquid. The brown liquid was stirred in heptane (3 x 6.0 L) for 10 h to remove excess of DMA. The heptane/DMA mixture was decanted (traces of desired product was observed in the decanted fraction) to afford waxy solid. The waxy solid was further stirred in MTBE (3.0 L) for 15 min. The resulting precipitate was filtered and washed with MTBE (2.0 L) to afford the desired product as a brown solid. The brown solid was passed through a small silica plug eluting with 10% MeOH in DCM (~35 L). The obtained solvent fraction was concentrated to 1/10 of its original volume. The concentrated fraction was diluted with MTBE (200 mL) and stirred for 30 min. The resulting precipitate was filtered, washed with MTBE (1.0 L) and dried under vacuum to afford regioisomeric mixture of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (220 g) as a light brown solid. Yield: 66% (97% by LCMS, 98:2 regioisomeric ratio). MS (ESI+) for CHNOS m/z 386.18 [M+1]⁺. 1H NMR (400 MHz, DMSO-d6 + d-TFA): *δ* 9.14 (s, 1H), 9.01-9.04 (m, 2H), 8.86 (d, *J* = 6.7 Hz, 1H), 8.23 (d, *J* = 5.8 Hz, 1H), 8.01 (bs, 1H), 7.55-7.58 (m, 1H), 7.41 (d, *J* = 8.3 Hz, 1H), 7.05 (d, *J* = 8.6 Hz, 1H), 4.09-4.33 (m, 2H), 3.71-3.92 (m, 2H), 2.17 (s, 3H), 2.11 (bs, 3H).

### Step 5

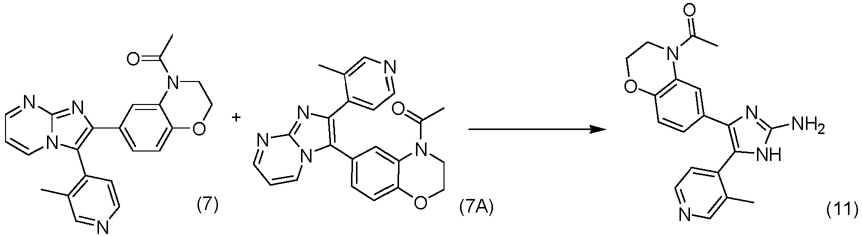

To a suspension of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (10 g, 98% by LCMS, 25.9 mmol) in isopropyl alcohol (70 mL) was added hydroxylamine hydrochloride (4.51 g, 64.9 mmol) at room temperature. The resulting suspension was stirred at 80°C for 6h. The suspension gradually solubilises in 2h at 80°C and re- precipitation was observed in 2-3h. The reaction was monitored by LCMS. The resulting suspension was allowed to cool to room temperature, diluted with acetone (100 mL) and stirred for 1h. The reaction mixture was concentrated to 20% of original volume, precipitate was filtered, washed with acetone (300 mL) and dried under vacuum to afford crude as an HCl salt. The obtained solid was dissolved in the water (10 mL), basified up to pH ~8.0 by saturated aqueous solution of NaHCO₃ at 10°C. The resulting suspension was stirred for 2 h at rt. The resulting precipitate was filtered, washed with water (200 mL) and dried under vacuum to afford the crude residue . The crude residue was further stirred in MTBE (100 mL), filtered and dried under vacuum to obtained the 1-(6-(2-Amino-5-(3-methylpyridin-4-yl)-1H-imidazol-4-yl)-2,3-dihydro-4H-benzo[b][1,4]oxazin-4-yl)ethan-1-one (11) as a light brown solid. Yield: 7.5 g, 83% (93% by LCMS). CHNOS m/z 350.30 [M+H]⁺, ¹H NMR (400 MHz, DMSO-*d₆*): *δ* 10.86 (bs, 1H), 8.39 (s, 1H), 8.31 (d, *J =* 4.8 Hz, 1 H), 7.16 (d, *J* = 4.8 Hz, 1 H), 6.71-7.09 (m, 2H), 5.37 (bs, 2H), 4.21(bs, 2H), 3.79 (bs, 2H), 1.99 (s, 3H), 1.75 (bs, 3H).

### Step 6

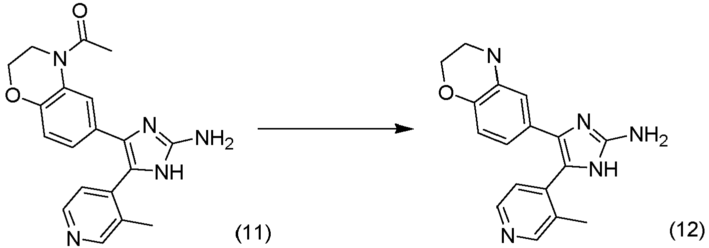

To a solution of 1-(6-(2-Amino-5-(3-methylpyridin-4-yl)-1H-imidazol-4-yl)-2,3-dihydro-4H-benzo[b][1,4]oxazin-4-yl)ethan-1-one (11) (7 g, 20.9 mmol) in methanol (35 mL) was added 6.0 N HCl (28 mL) at room temperature. The reaction mixture was stirred at 90°C for 16 h. The reaction was monitored by LCMS. After completion of the reaction, the reaction mixture was allowed to cool to room temperature and concentrated to afford the crude product. The crude product was dissolved in water (20 mL), basified to pH 8.0-9.0 with 6.0 M aqueous NaOH solution and extracted with 10% MeOH in DCM (3 X 100 mL). The organic layer was washed with brine (200 mL), filtered and concentrated to 1/10th of original reaction mixture volume under reduced pressure. The concentrated mixture was diluted with heptane (100 mL) and stirred for 15 min. The resulting precipitate was filtered, washed with heptane (100 mL) and dried under reduced pressure to afford 4-(3,4-dihydro-2H-benzo[b][1,4]oxazin-6-yl)-5-(3-methylpyridin-4-yl)-1H-imidazol-2-amine (12) as a light brown solid. Yield: 5.1 g, 82% (98% by LCMS). MS (ESI+) for CHNOS *m*/*z* 308.14 [M+H]⁺. ¹H NMR (400 MHz, DMSO-*d₆* + d-TFA): *δ* 8.88 (s, 1H), 8.82 (d, *J =* 6.1 Hz, 1 H), 7.94 (d, *J* = 6.1 Hz, 1 H), 6.68 (d, *J* = 8.2 Hz, 1 H), 6.49 (d, *J* = 2.2 Hz, 1 H ), 6.42 (dd, *J* = 2.2, 8.2 Hz, 1 H), 4.14 (*t, J* = 4.2 Hz, 2 H), 3.27 (*t, J* = 4.2 Hz, 2 H), 2.16 (s, 3H).

### Step 7A (HMPA mediated amino acid coupling)

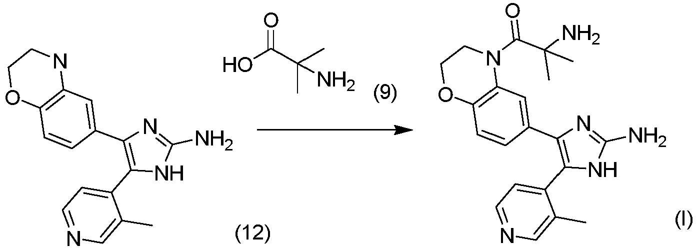

To a suspension of 2-amino-2-methylpropanoic acid (9) (10) (6.0 g, 58.2 mmol) in HMPA (42 mL) was added a solution of thionyl chloride ( 4.64 mL, 64.0 mmol) in ACN (3.00 mL) slowly at 3°C. The suspension becomes a clear solution after 10 min of the addition of solution of thionyl chloride in ACN. The resulting reaction mixture was stirred at 0°C for 30 min followed by portion wise addition of 4-(3,4-dihydro-2H-benzo[b][1,4]oxazin-6-yl)-5-(3-methylpyridin-4-yl)-1H-imidazol-2-amine (12) (3.58 g, 11.6 mmol) at 3°C. The reaction mixture was allowed to warm to room temperature and stirred for 16 h. After the completion of the reaction, the reaction mixture was diluted with EtOAc (100 mL). The resulting precipitate was filtered and washed with EtOAc (200 mL). The solid was dissolved in water (5 mL), basified to pH ~ 8.0 with aqueous saturated sodium bicarbonate solution and extracted with 10% MeOH in DCM (5 x 100 mL). The collected organic layer was dried (Na₂SO₄) and concentrated to 1/5th of its original volume, diluted with DCM (100 mL) and stirred for 15 min. The resulting precipitate was filtered, triturated with 2% MeOH in DCM (25 mL) followed by washing with MTBE (50 mL) and dried under vacuum to afford the 2-amino-1-(6-(2-amino-5-(3-methylpyridin-4-yl)-1H-imidazol-4-yl)-2,3-dihydro-4H-benzo[b][1,4]oxazin-4-yl)-2-methylpropan-1-one (compound (I)) ( (2.4 g, ~96.6% by LCMS and ¹H NMR) as a light yellow solid. Yield: (53%); MS (ESI+)for CHNOS m/z 392.8 [M+H]⁺. ¹H NMR (400 MHz, DMSO-*d₆* + d-TFA): *δ* 8.78-8.84 (m, 2H), 7.93 (d, *J* = 6.1 Hz, 1 H), 7.44 (d, *J =* 2.1 Hz, 1 H), 7.09 (dd, *J* = 2.1, 8.6 Hz, 1 H), 6.97 (d, *J* = 8.6 Hz, 1 H), 4.34 (bs, 2H), 3.97 (bs, 2H), 2.16 (s, 3H) and 1.60 (s, 6H).

### Or Step 7B (DMPU mediated amino acid coupling)

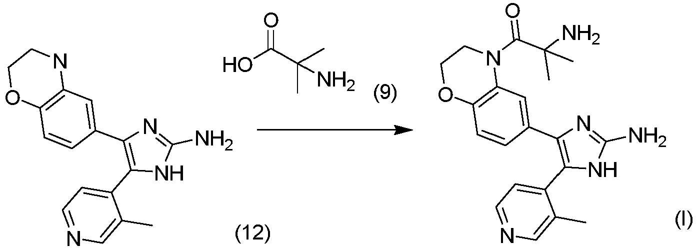

To a solution of 2-amino-2-methylpropanoic acid (9) (3.3 g, 32.0 mmol) in DMPU (23 mL) was added a solution of thionyl chloride (2.55 mL, 35.2 mmol) in ACN (2.5 mL) slowly at 4°C. The resulting reaction mixture was stirred at 0°C for 20 min followed by portion wise addition of a 4-(3,4-dihydro-2H-1,4-benzoxazin-6-yl)-5-(3-methylpyridin-4-yl)-1H-imidazol-2-amine (12) (1.97 g, 6.40 mmol) at 0°C. The reaction mixture was allowed to warm to room temperature and stirred further for 16 h. After the completion of the reaction, the reaction mixture was diluted with ACN (50 mL). The resulting precipitate solid was filtered and washed with ACN (100 mL). The solid was dissolved in water (5.0 mL), basified up to pH ~8.0 with saturated aqueous sodium bicarbonate solution at 10°C and extracted with 10% MeOH in DCM (5 X 50 mL) and concentrated under reduced pressure. The crude residue was stirred in EtOAc (20 mL), filtered and dried under reduced pressure to obtain light brown solid. The solid obtained was further stirred in the EtOH (7 mL), filtered and dried under vacuum to afford 2-amino-1-{6-[2-amino-5-(3-methylpyridin-4-yl)-1H-imidazol-4-yl]-3,4-dihydro-2H-1,4-benzoxazin-4-yl}-2-methylpropan-1-one (compound (I)) as an off white solid (770 mg, 99.7% by LCMS). Yield: (53%); MS (ESI+) for CHNOS m/z 392.8 [M+H]⁺.

For all Methods 1, 2, 4 and 5 above, 4-bromo-3-methylpyridine (6) was formulated according to Step 8 as follows:

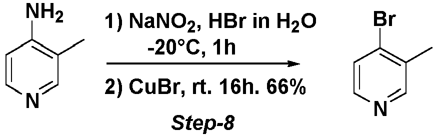

A 20 L, four-necked round bottom flask equipped with mechanical stirrer was charged with 3-methylpyridin-4-amine (25.0 g, 231 mmol) and hydrogen bromide (47% aqueous solution, 360 mL) at -20°C (internal temperature). To the resulting reaction mixture a solution of sodium nitrite (76.6 g, 1.11 mol) in water (520 mL) was added slowly at -20°C over approximately 90 mins. The reaction mixture was stirred at -20°C for 1h and CuBr (7.96 g, 55.5 mmol) was added to it at -20°C. The reaction mixture was warmed to room temperature and further stirred for 16h. The TLC showed reaction to be complete. The reaction mixture was quenched with 50% aq solution of sodium hydroxide (100mL), filtered through celite bed. The celite bed was washed with DCM (100mL) and extracted. The aqueous layer was extracted with DCM (2 X 100 mL). The combined organic layer was filtered and concentrated under reduced pressure (keeping water bath temperature > 40°C to afford a brown liquid. Yield 66% (26 g). MS (ESI+) for CHNOS m/z 172.0 [M+H]⁺. ¹H NMR (400 MHz, DMSO-*d*₆): δ 8.50 (s, 1H), 8.25 (d, *J* = 5.20 Hz, 1 H), 7.66 (d, *J* = 5.2 Hz, 1 H), 2.33 (s, 3H).

For method 3, the hydrochloride salt of 4-bromo-3-methylpyridine (6A) was commercially available, but may also be formed through modification of the process of Step 8 for the production of 4-bromo-3-methylpyridine (6) detailed above.

For Methods 4 and 5, it has been surprisingly and advantageously found that Step 4 facilitates advantageous regioisomer formation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) over 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) at 98% to 2% respectively.

Accordingly, also encompassed in the present invention is a method of synthesising 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) according to Step 4 of Methods 4 and 5 described herein.

In addition, for Methods 4 and 5 it has been surprisingly and advantageously found that the use of NH₂OH.HCl (hydroxylamine hydrochloride) in Step 7 of Method 4 and Step 5 of Method 5 advantageously improves yield.

Accordingly, also encompassed in the present invention is a method of synthesising compound (I) from 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) using NH₂OH.HCl (hydroxylamine hydrochloride).

Preferably, a method of synthesising compound (I) from 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) & 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) according to Step 7 of Method 4 described herein.

Accordingly, also encompassed in the present invention is a method of synthesising 1-(6-(2-Amino-5-(3-methylpyridin-4-yl)-1H-imidazol-4-yl)-2,3-dihydro-4H-benzo[b][1,4]oxazin-4-yl)ethan-1-one (11) from 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) using NH₂OH.HCl (hydroxylamine hydrochloride).

Preferably, a method of synthesising 1-(6-(2-Amino-5-(3-methylpyridin-4-yl)-1H-imidazol-4-yl)-2,3-dihydro-4H-benzo[b][1,4]oxazin-4-yl)ethan-1-one (11) from 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) according to Step 5 of Method 5.

Also encompassed in the present invention is a method of synthesising compound (I) from 4-(3,4-Dihydro-2H-benzo[b][1,4]oxazin-6-yl)-5-(3-methylpyridin-4-yl)-1H-imidazol-2-amine (12) using 2-amino-2-methylpropanoic acid (9), preferably according to Step 7 of Method 5.

Also encompassed in the present invention is a method of synthesising compound (I) from 1-(6-(2-Amino-5-(3-methylpyridin-4-yl)-1H-imidazol-4-yl)-2,3-dihydro-4H-benzo[b][1,4]oxazin-4-yl)ethan-1-one (11) via 4-(3,4-Dihydro-2H-benzo[b][1,4]oxazin-6-yl)-5-(3-methylpyridin-4-yl)-1H-imidazol-2-amine (12) and using 2-amino-2-methylpropanoic acid (9), preferably according to Steps 6 and 7 of Method 5.

### Method 6

Compound (I) may be synthesised according to the following method:

### Step 1

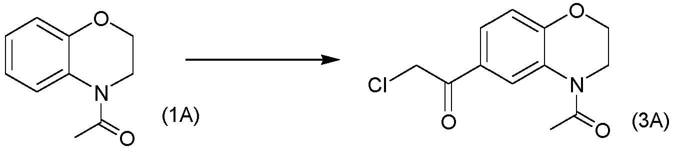

A 20 L round bottomed flask equipped with a mechanical stirrer was charged with dichloromethane (2.6 L, 13V) at 25 °C ± 5 °C under nitrogen atmosphere. Aluminium chloride (AlCl₃) (0.752 kg, 4 mol eq.) was added to the round bottomed flask at 25 ± 5 °C under a nitrogen atmosphere under stirring. This first reaction mixture was stirred for a period of 20 ± 5 minutes under nitrogen atmosphere. The reaction mixture was cooled to 5 ± 5 °C. Separately, a 5L round bottom flask equipped with a mechanical stirrer was charged with 1-(2,3-dihydro-4H-benzo[b][1,4]oxazin-4-yl)ethan-1-one (1A) (0.25 kg, 1 mol eq.) and dichloromethane (0.5 L, 2V). The second reaction mixture was slowly added to the first reaction mixture in the 20L round bottomed flask through an addition funnel qt a temperature of 5 ± 5 °C. The combined reaction mixture was stirred for 10 ± 5 minutes at 5 ± 5 °C. Chloroacetyl chloride (0.145L, 1.3 mol eq.) was slowly added to the combined reaction mixture through an addition funnel at a temperature of 5 ± 5 °C under nitrogen atmosphere. The reaction mixture was heated to 40 ±5 °C. The reaction mixture was stirred at 40 ± 5 °C for 3 ± 1 hours. The reaction mixture was then cooled to 25 ± 5 °C, and added to another 20L round bottomed flask charged with ice cooled water (2.5L, 10V) at 2 to 5 °C under stirring. The mixture was stirred for 30 minutes to 1 hour at 25 ± 5 °C, and then left to settle for 30 minutes so that phase separation occurred. The aqueous (10V) and organic (3.5 L, 14 V) layers were collected in separate containers and the aqueous layer charged back into the 20L round bottomed flask, which was further charged with dichloromethane (1.5 L, 6V) and the mixture stirred for 30 minutes. The mixture was then transferred to a separating funnel, and left to settle for 30 minutes. The aqueous and organic layers (1.5 L) were separated again, and the combined organic layers charged into the 20L round bottomed flask with water (1.25 L, 5V), stirred for 30 minutes, left to settle for 10 to 15 minutes, and then separated. The organic layer was washed with water (4 x 5V) and aqueous (5 L) and organic layers (5 L) separated. The organic layer was charged into a Buchi round bottomed flask and distilled under reduced pressure at 40 °C to afford a concentration organic layer. Charcoal (10% w/w wrt starting material) was introduced into the organic layer and stirred for 120 ± 10 minutes at 25 ± 5 °C. The reaction mass was then filtered through celite bed in Buchner filter, washed with 50% MeOH in DCM and then filtrate collected. A rotavapor was charged with the organic layer and the filtrate distilled off at 40 °C under vacuum until 2V remained. The resulting mixture was charged with methyl tert-butyl ether (MTBE) (6V), stirred for 1 hour at 25 ± 5 °C. The resultant solid formed was filtered over a Buchner funnel under vacuum. The solid material was then washed with MTBE (2V), dried for 30 minutes under vacuum, and then further dried in a vacuum oven for 7 to 8 hours at 55 °C to afford 70% yield of 1-(4-acetyl-3,4-dihydro-2H-benzo[b][1,4]oxazin-6-yl)-2-chloroethan-1-one (3A), having a purity 97.97% (measured by LCMS). The compound was used in the next step without further purification.

### Step 2

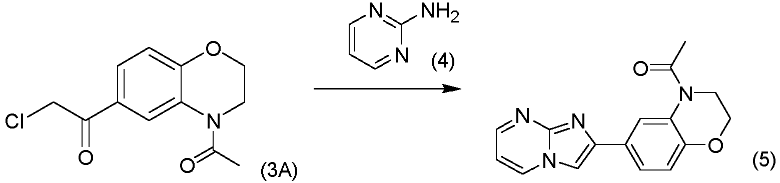

A 2L round bottomed flask equipped with a mechanical stirrer was charged with ethanol (1.0 L, 5 V) at 25 °C ± 5 °C under nitrogen atmosphere. 1-(4-acetyl-3,4-dihydro-2H-benzo[b][1,4]oxazin-6-yl)-2-chloroethan-1-one (3A) (0.21 kg, 1.0 mol eq.) was added to the round bottomed flask at 25 ± 5 °C under a nitrogen atmosphere under stirring. This first reaction mixture was stirred for a period of 10 minutes under nitrogen atmosphere, and then charged 2-amino pyrimidine (4) (0.197 kg, 2.50 mol eq.) to first reaction mixture at 25 °C ± 5 °C under nitrogen atmosphere. The reaction mixture was heated to 75 -78 °C. The reaction mixture was stirred at 78 °C for 16-24 hours. The reaction mixture was then cooled to 25 ± 5 °C. The mixture was stirred for 1 hour at 25 ± 5 °C, and then filtered over Buchner filter, washed wet cake with ethanol (0.2 L) at 25 ± 5 °C and dried in vacuum for 30 minutes. Further dried in a vacuum oven for 5 to 6 hours at 65 ± 5 °C to afford 57.6% yield of 1-(6-(imidazo[1,2-a]pyrimidin-2-yl)-2,3-dihydro-4H-benzo[b][1,4]oxazin-4-yl)ethan-1-one (5), having a purity 99.52% (measured by LCMS). The compound was used in the next step without further purification.

### Step 3

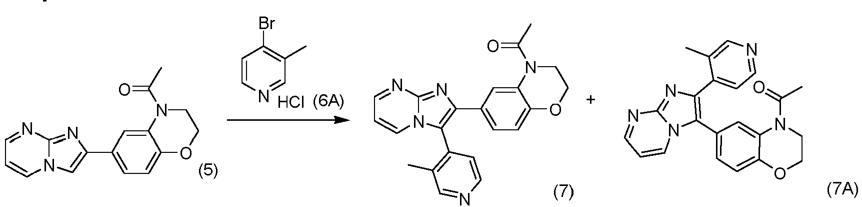

A 3 L round bottomed flask equipped with a mechanical stirrer was charged with dimethyl formamide (850 mL, 10V) at 25 °C ± 5 °C under nitrogen atmosphere. 1-(6-(imidazo[1,2-a]pyrimidin-2-yl)-2,3-dihydro-4H-benzo[b][1,4]oxazin-4-yl)ethan-1-one (5) (85 g, 1.0 mol eq.) was added to the round bottomed flask at 25 ± 5 °C under a nitrogen atmosphere under stirring. This first reaction mixture was stirred for a period of 10 minutes under nitrogen atmosphere. Added 4-bromo-3-methyl-pyridine HCl (6A) (72.2 g, 1.20 mol eq.), potassium carbonate (K₂CO₃) (199.6 g, 5.0 mol eq.), pivalic acid (13.03 mL, 0.40 mol eq.) and tricyclohexyl phosphine tetrafluoroborate (PCy₃.HBF₄) (1.63 g, 0.10 mol eq.) at 25 °C ± 5 °C under nitrogen atmosphere. Reaction mixture degassed with argon for 25-30 minutes at 25±5°C. To the reaction mixture added palladium acetate (Pd(OAc)₂) (6.48 g, 0.10 mol eq.). The combined reaction mixture was heated to 125 ±5 °C. The reaction mixture stirred at 125 ±5 °C for 2-4 hours. The reaction mixture was then cooled to 30 ± 5 °C. The reaction mixture filtered through celite bed over Buchner filter, washed with 10% MeOH in DCM and then filtrate collected. A rotavapor was charged with the organic layer and the filtrate distilled off at 65 ±5 °C under vacuum until 2V remained. To the resulting mixture was charged dichlormethane (DCM) (2V) stirred for 15-20 minutes at 25 ± 5 °C and then was charged methyl tert-butyl ether (MTBE) (10V), stirred for 15-20 min at 25 ± 5 °C, and the resultant solid formed filtered off over a Buchner funnel under vacuum. The solid material was then dried under vacuum and then dried in a vacuum oven for 3-4 hours at 50 ± 5 °C to afford 88% of crude material (7 & 7A), having a purity 77.12% (measured by LCMS). To the obtained compound (7 & 7A) was further performed palladium scavenging operation which include silica plug filtration followed by trithiocyanuric acid operation. Silica plug filtration performed using silica 100-200 mesh, 10% MeOH in DCM and then filtrate collected. A rotavapor was charged with the organic layer and the filtrate distilled off at 45 ± 5 °C under vacuum to afford solid. The solid material was charged in a 3 L round bottomed flask and then charged dichloromethane (10V) under stirring. The mixture stirred for 10 minutes, added trithiocyanuric acid (6.8 g, 20 mol% w/w), charcoal (14.8 g, 20 mol% w/w), sodium bicarbonate (NaHCO₃) (28.22 g, 1.75 eq.) and water (282 mL). The combined mixture stirred for 3 hours at 25 ± 5 °C, and filtered through celite bed over Buchner filter, washed with 10% MeOH in DCM and then filtrate collected. Charge filtrate in separator and left to settle for 15-20 minutes so that phase separation occurred. The aqueous (0.282 L) and organics (5.18 L) collected in separate containers and organic layer charged back in to the 10 L round bottomed flask, which was further charged with 1N NaOH (20V) and the mixture was further stirred for 20 ± 5 minutes. The resulting mixture was charged in 10 L separator and left to settle for 10 to 15 minutes so that phase separation occurred. The aqueous layer (1.48 L) and organic (5.18 L) were collected in separate containers. Organic layer (5.18 L) washed with brine solution (10 L) and separated (5.18 L). The organic layer was charged into a round bottomed flask and distilled off at 40 ±5 °C under vacuum until 1.5V remained. The resulting mixture was charged with methyl tert-butyl ether (MTBE) (3V), and then stirred for 30 minutes at 25 ± 5 °C. The resulting precipitate was filtered over a Buchner funnel under vacuum. The solid material was then washed with MTBE (1V), dried over 30 min under vacuum and then further dried in a vacuum oven for 2-4 hours at 50 ± 5 °C to afford 70.06% yield of (7 & 7A), having a purity 95.21% (measured by LCMS). Palladium results obtained 49-119 ppm. To obtain palladium of less than 100 ppm, trithiocyanuric acid operation was repeated one more time.

### Step 4

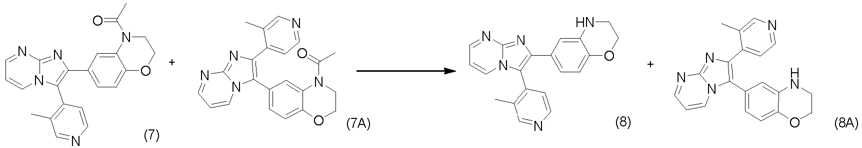

A 10.0 L round bottomed flask equipped with a mechanical stirrer was charged with 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) & 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) (mixture of regioisomers 7 and 7A) (440 g, 1.0 mol eq.) at temperature 25±5°C. Methanol (4.4 L, 10V) was added into the round bottomed flask at temperature 25±5°C. The reaction mass was stirred for 10 min at 25±5°C (RM is in heterogeneous form) followed by addition of 30% aq. NaOH solution (880 mL, 2 V) into the round bottomed flask at 25±5°C under stirring. The reaction mass was heated at 70±5°C, for a period of 5-6 hours. After approximately 4 hours at 65-70°C precipitation of material is observed. Stop heating and allow the reaction mass to attain 25±5°C. More solid formation was observed at 25±5°C in reaction mixture than at heating. The reaction mass was filtered through Buchner funnel under vacuum. Wet cake was washed with methanol (0.88 L, 2V) and dried for 30 minutes under vacuum. Wet cake was unloaded from Buchner filter and added into another 5.0 L round bottomed flask. Milli-Q water (2.2 L, 5 V) was added in 5.0 L round bottomed flask and mixture was stirred for another 30 minutes. Mixture was filtered over Buchner funnel and washed with water (2.2 L, 5V), pH of the filtrate should be ~7 to 8. Material was dried over a Buchner funnel for 30 minutes and further in vacuum oven for a period of 12-14 hours at 65±5°C to afford 66.8 % yield of (8 and 8A), having a purity 98.78% (measured by HPLC). The compound was used in the next step without further purification. Of the regioisomers (8) and (8A), (8) is predominant and in some of the batches 8A is very negligible.

### Step 5

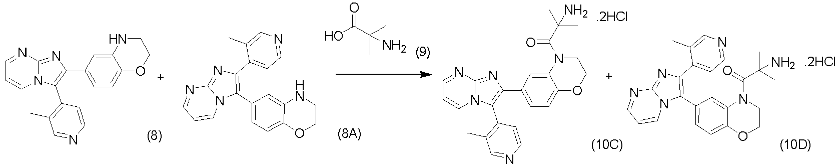

A 10.0 L round bottomed flask equipped with a mechanical stirrer was charged with 2-amino-2-methylpropanoic acid (9) (260 g, 3.34 mol eq.) at 25±5°C. Acetonitrile (3.9 L, 15 V) was added into the round bottomed flask and the mixture was stirred for 10 minutes at 25±5°C under nitrogen atmosphere. Thionyl chloride (403 mL, 7.34 mol eq.) was added into the round bottomed flask over a period of 10 minutes (~2°C exotherm observed). The resulting mixture was heated at 40 ± 2°C for 2 hours (white suspension observed). Mixture was then cooled to 30 °C and then to it was added pyridine (792 mL, 13.0 mol eq.) over a period of 45 minutes (exotherm of 5-7°C observed, mixture became clear). Mixture was further stirred at 35-40°C for a period of 1 hour and then cooled to 30°C followed by addition of 6-(3-(3-methylpyridin-4-yl)imidazo[1,2-a]pyrimidin-2-yl)-3,4-dihydro-2H-benzo[b][1,4]oxazine (8) & 6-(2-(3-methylpyridin-4-yl)imidazo[1,2-a]pyrimidin-3-yl)-3,4-dihydro-2H-benzo[b][1,4]oxazine (8A) (mixture of regioisomers 8 and 8A) (260 g, 1.0 mol eq.), exotherm of 4-5°C observed. Reaction mass was further stirred at 30±5°C for a period of 2-3 hour (yellow precipitation observed after 30-45 minutes of maintenance). Reaction mass was then filtered under nitrogen atmosphere over Buchner funnel to obtain the solid product, which was then washed with acetonitrile (1.04 L, 4V). Material was then dried in vacuum oven at 65±5°C for 6-8 hour to obtain 320 g crude material. Obtained crude material was then dissolved in ethanol (960 mL, 3V with respect to crude) at 25±5°C and stirred for 5-10 minutes. Mixture was then heated at 70±5°C for 30 minutes and then cooled to 40°C. Isopropyl acetate (4.16 L, 13V) was added to the mixture and then allowed to cool to 25±5°C. Mixture was then stirred at 25±5°C for 3-4 hours and then filtered over Buchner funnel under reduced pressure. Obtained solids were then washed with isopropyl acetate (640 mL, 2V). Material was dried over a Buchner funnel and finally in an oven for a period of 5-6 hours at 65±5°C to afford 65.8 % yield of (10C and 10D), having a purity 99.81% (measured by HPLC). Of the regioisomers (10C) and (10D), (10C) is predominant and in some of the batches 10D is not observed.

### Step 6

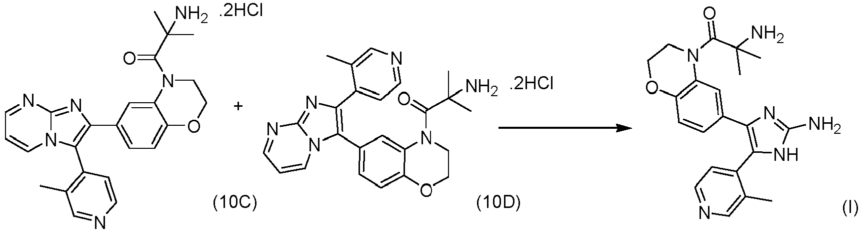

A 10.0 L round bottomed flask equipped with a mechanical stirrer was charged with (10C and 10D) bis HCl salt (250 g, 0.49 mol eq.) at 25±5°C. Isopropanol (1.25 L, 5 V) was added into the round bottomed flask at 25±5°C. Mixture was stirred for 10 minutes under nitrogen atmosphere followed by addition of triethylamine (208 mL, 3.0 mol eq.). The resulting mixture was stirred at 25±5°C for 10 minutes and then at 50°C for 2 hours (heterogeneous reaction mass). Hydroxylamine hydrochloride (104 g, 1.49 mol eq.) was added to the mixture (exotherm of 2-3°C observed). Reaction mass was further stirred at 50°C for 2-3 hours. Reaction becomes clear after 15-20 minutes and solid starts to precipitate after 2 hours. Cooled the reaction mass to 25±5°C. Isopropyl acetate (1.25 L, 5V) was added into the round bottomed flask at 25±5°C and the mixture was stirred for another 1 hour. Isopropyl acetate (2.5 L, 10V) was further added into the round bottomed flask at temperature 25±5°C and stirred for another 1 hour. Solid thus obtained was then filtered over Buchner funnel under reduced pressure to obtain crude material under nitrogen atmosphere. The solid material thus obtained was washed with isopropyl acetate (500 mL, 2V) and dried under vacuum. The solid was further dried in vacuum oven at 60±5°C for 4-6 hours to obtain 350 g crude material. Crude material (350 g) was then refluxed in ethanol (1.75 L, 5V) for 30 minutes and then cooled to 25±5°C and filtered under reduced pressure to obtain desired material. Material was dried over a Buchner funnel and finally in a vacuum oven for a period of 6-8 hours at 60±5°C to afford 84.9 % yield of compound (1) as bis HCl salt having a purity 99.46 % (measured by HPLC). The HCl salt thus obtained was further taken up to get a free base. In a 10.0 L round bottomed flask equipped with a mechanical stirrer and charged with Milli-Q water (1.26 L, 2.4V) at 25±5°C, compound (1) (bis HCl salt) (525 g) was added into the round bottomed flask at 25±5°C. Mixture was stirred for 10 minutes and then cooled to 15±5°C followed by addition of NH₄OH (1.26 L, 2.4V) over a period of 30-45 minutes. Reaction mass was stirred at 15±5°C for 30-45 minutes. Dichloromethane (2.6 L, 5V) was added to the reaction mass (DCM addition helps in solid formation). Mixture was stirred for another 30-45 minutes and then filtered over a Buchner funnel under reduced pressure and washed with water (1.05 L, 2V). Wet solid was charged back into the round bottomed flask and slurried with water (5.25 L, 10V) for 30-45 minutes at 25±5°C. Solids were then again filtered over Buchner funnel and washed with DCM (1.05 L, 2V). Material was dried over a Buchner funnel and finally in a vacuum oven for a period of 12-14 hours at 65±5°C to afford 421 g of compound (1), having a purity 99.01 % (measured by HPLC).

It will be appreciated that a person skilled in the art will be readily able to synthesise pharmaceutically acceptable salts of compound (I), typically using any of the syntheses disclosed herein to form compound (I), followed by known additional step(s) required to form a pharmaceutically acceptable salt thereof.

The advantageous properties of compound (I) will now be described with reference to specific examples.

### Examples

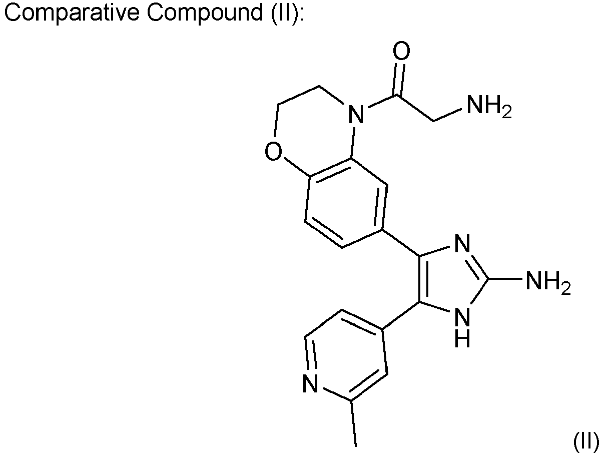

was synthesised by modification of the method outlined for compound (I) above. Such modification includes selection of the appropriate pyridyl starting material and the use glycine as the amino acid. 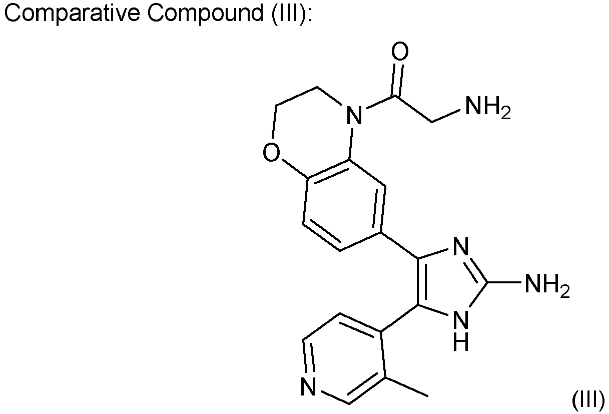 was synthesised by modification includes selection of the appropriate pyridyl starting material and the use glycine as the amino acid.

Compound (I) and Comparative Compounds (II) and (III) were subject to the following analysis relating to minimum inhibitory concentration (MIC) and minimum inhibitory concentration 90 values (MIC₉₀).

### Antibacterial Susceptibility

Minimum Inhibitory Concentrations (MICs) versus *Escherichia coli* (NCTC 13441) and *Klebsiella pneumoniae* (NCTC 13438) (planktonic bacteria) were determined by the broth microdilution procedure in line with the guidelines of the Clinical and Laboratory Standards Institute (Clinical and Laboratory Standards Institute. Methods for Dilution Antimicrobial Susceptibility Tests for Bacteria That Grow Aerobically; Approved Standard-Eleventh Edition, CLSI document M07, 11 January 2018). The broth dilution method involved a two-fold serial dilution of compounds in 96-well microtitre plates, giving a final concentration range of 0.39-200 µM and a maximum final concentration of 2% DMSO. The bacterial strains tested were *Escherichia coli* (NCTC 13441) and *Klebsiella pneumoniae* (NCTC 13438). Strains were grown in cation-adjusted Müller-Hinton broth or on Luria Bertani agar at 37°C in an ambient atmosphere. The MIC (µM) was determined as the lowest concentration of compound that inhibits growth following a 20-24 hour incubation period. The results are set out in Table 1.

**Table 1**

| | *Escherichia coli* (NCTC 13441) (µM) | *Klebsiella pneumoniae* (NCTC 13438) (µM) |
|---|---|---|
| Compound (I) | ≤0.39 | 1.56-3.13 |

Minimum Inhibitory Concentrations (MICs) versus *Proteus mirabilis (DSM 4479)* were determined for compound (I) and comparative compounds (II) and (III) using the methodology outlined above. The results are set out in Table 2. Compound (I) shows a superior potency versus comparative compounds (II) and (III).

**Table 2**

| | *Proteus mirabilis* (µM) |
|---|---|
| Compound (I) | 25 |
| Comparative Compound (II) | >200 |
| Comparative Compound (III) | >200 |

Minimum Inhibitory Concentration (MIC₉₀) versus 100 isolates of *Escherichia coli* and *Klebsiella pneumonia* were determined. The Minimum Inhibitory Concentration at which 90% of the isolates (strains) are inhibited (MIC₉₀) were measured by broth microdilution in line with EUCAST susceptibility testing standards (www.eucast.org). Bacterial inocula were prepared at ca 1 x 10^6 CFU/mL by diluting 100-fold a 0.5 McFarland suspension. Antibacterial panels containing 50 µl of antibacterial solutions at 2 x the final concentrations were diluted 2-fold with 50 µl of inoculum to give a final inoculum of ca 5 x 10^5 CFU/ml and desired test concentrations of antibacterial agents (0.03 - 64 µg/mL). The plates were incubated according to the guidelines of the Clinical and Laboratory Standards Institute (Clinical and Laboratory Standards Institute. Methods for Dilution Antimicrobial Susceptibility Tests for Bacteria That Grow Aerobically; Approved Standard-Eleventh Edition, CLSI document M07, 11 January 2018). The MIC₉₀ (µg/ml) is the MIC value at which greater than or equal to 90% of the isolates (strains) within a test population are inhibited. The results are set out in Table 3.

**Table 3**

| | *Escherichia coli* (100 isolates) | | *Klebsiella pneumonia* (100 isolates) | |
|---|---|---|---|---|
| | MIC₉₀ (µg/mL) | Range (µg/mL) | MIC₉₀ (µg/mL) | Range (µg/mL) |
| Compound (I) | 1.00 | 0.12 - 4.00 | 2.00 | 0.25-8.00 |

Minimum Inhibitory Concentrations (MICs) versus *Proteus spp.* and *Providencia spp.* were determined for compound (I) and comparative compound (III) using the methodology outlined above for the 100 isolates of *Escherichia coli* and *Klebsiella* *pneumonia.* The results are set out in Table 4. Compound (I) shows a superior potency versus comparative compound (III).

**Table 4**

| | *Proteus spp.* (10 isolates) MIC Range (µg/mL) | *Providencia spp.* (10 isolates) MIC Range (µg/mL) |
|---|---|---|
| Compound (I) | 8-32 | 0.5-16 |
| Comparative Compound (III) | 64->64 | 1->64 |

Compound (I) and Comparative Compounds (II) and (III) were subject to the following analysis relating to lipophilicity and volume of distribution (Vss).

### Lipophilicity

Log P values of compound (I) and comparative compound (III) were measured using the SiriusT3 instrument from Sirius Analytical by performing an acid-based titration and measuring the shift in pKa when the sample solution is in contact with an immiscible solvent (octanol). Log P for the comparative compound (II) was calculated using Marvin (physico-chemical calculation software). Results are shown in Table 5. The lipophilicity data for compound (I) and comparative compounds (II) and (III) was collected in separate experiments carried out on different days, and compared in Table 5.

**Table 5**

| | Compound (I) | Comparative Compound (II) | Comparative Compound (III) |
|---|---|---|---|
| Log P | 1.6 | 0.07 | 1.03 |

### Volume of Distribution

The volume of distribution (Vss) of compound (I) and comparative compounds (II) and (III) was determined from analysis of plasma concentration time profiles obtained from rodent species (mice), following 5 mg/kg IV bolus administration respectively. The volume of distribution was obtained by analysing the rodent plasma profiles for compound (I) and comparative compounds (II) and (III) using a 2 compartment IV bolus model in PK Solver (Excel). The outputted Vss values are shown in Table 6. The volume of distribution data for compound (I) and comparative compound (II) was collected in separate experiments carried out on different days, and compared in Table 6.

**Table 6**

| | Compound (I) | Comparative Compound (II) | Comparative Compound (III) |
|---|---|---|---|
| Vss (L/kg) | 19.7 | 7.7 | 7.9 |

### Discussion of Results

Without being bound by theory, the present inventors consider that above results relating to lipophilicity and volume of distribution demonstrate that compound (I), or a pharmaceutically acceptable salt thereof, may be advantageously used in the treatment of infection with, or disease caused or exacerbated by, Gram-negative bacteria of the order Enterobacterales and/or Gram-negative bacteria of the genus *Haemophilus,* in particular in the treatment of multi-site infections with Gram-negative bacteria of the order Enterobacterales and/or Gram-negative bacteria of the genus *Haemophilus.* The present inventors consider that the above results demonstrate that compound (I), or a pharmaceutically acceptable salt thereof, has the ability to be distributed effectively around a subject's body to all infection sites at sufficient concentration so as to enable effective treatment at every site. As well as sufficient amounts of the compound being available to exert a pharmacological effect, there is a balance to be maintained between the distribution of the compound from the blood plasma being sufficiently rapid such that the drug can act quickly against the infection, and avoiding overly rapid release which would prevent the drug reaching all possible infection sites in sufficient concentration.

Such a balance is considered to be demonstrated by the above results, which are discussed below in more detail.

A Log P value of a compound provides an indication of polarity and thus, the ability of the compound to reach the target tissue in a subject, i.e. the ease at which the drug can cross membrane barriers and be distributed around a subject's body to possible intracellular infection sites.

From Table 5, it can be seen that compound (I) of the present invention has a higher Log P value and thus lower polarity relative to both comparative compounds (II) and (III), indicating improved tissue distribution of compound (I) compared to the more polar comparative compounds (II) and (III). The present inventors consider that this improved tissue distribution of compound (I) enables the compound to be distributed around a subject's body to all possible infection sites, with enough drug reaching each of the infection sites to enable treatment. If the drug is distributed less effectively, such as will be seen for comparative compound (II) and (III) based on the Log P values of Table 5, there will be insufficient drug concentration across the infection sites and treatment of the infection hindered. As discussed above, there is a balance to be achieved regarding the efficiency at which the drug is distributed around the subject's body. The Log P value of compound (I) indicates that it is distributed to the multi-sites of infection and provides appropriate drug concentration at all of these infection sites versus comparative compounds (II) and (III).

Volume distribution (Vss) provides an indication of the degree to which the compound is distributed in a subject's body tissue rather than the blood plasma, i.e. the propensity of the compound to leave the blood plasma and be distributed in the subject's body tissue.

From Table 6, it can be seen that compound (I) has surprising and unexpectedly better tissue penetration characteristics compared to comparative compound (II) and comparative compound (III), which are both structurally very similar to compound (I).

The present inventors have determined that compound (I) demonstrates good permeability. The lipophilicity and permeability of compound (I) allows the compound to reach and penetrate the target tissue in a subject. The surprising and unexpectedly better tissue distribution and penetration characteristics of compound (I) versus comparative compounds (II) and (III) result from the advantageous lipophilicity and permeability of compound (I).

### In Vitro Safety Pharmacology Study

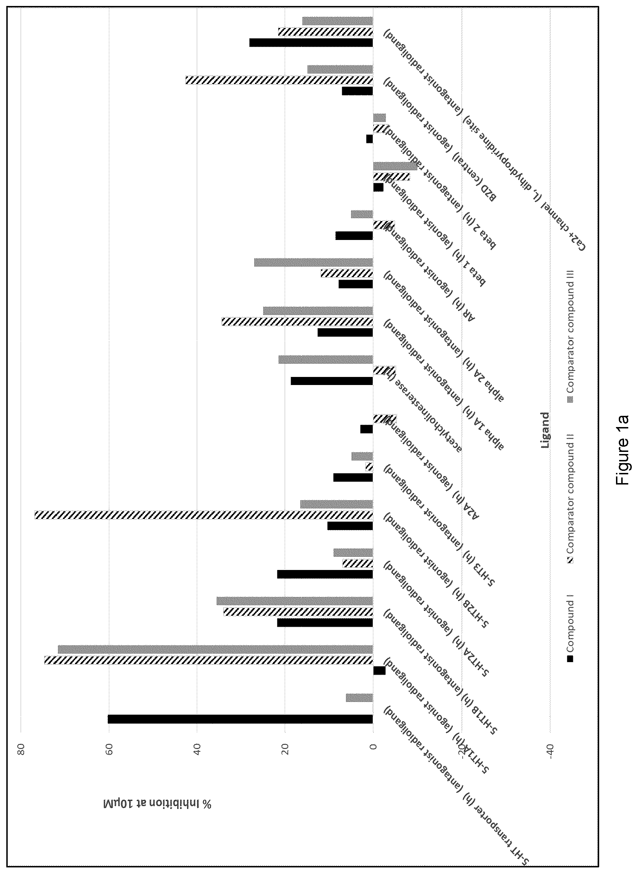

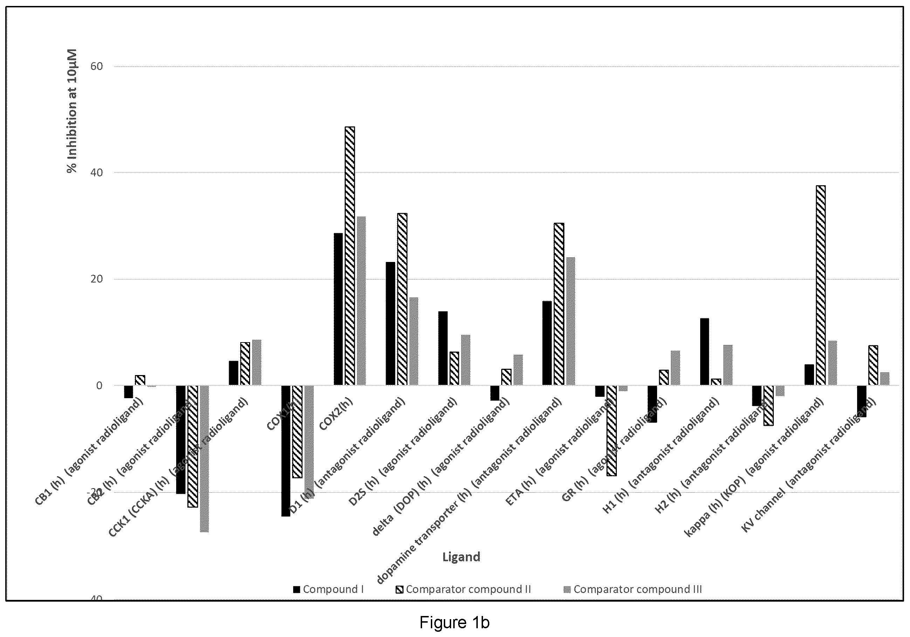

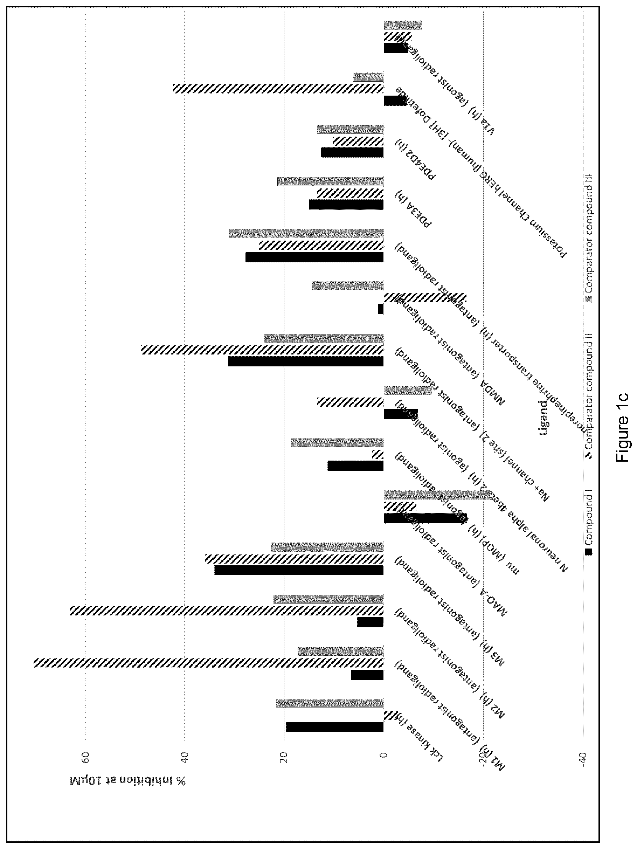

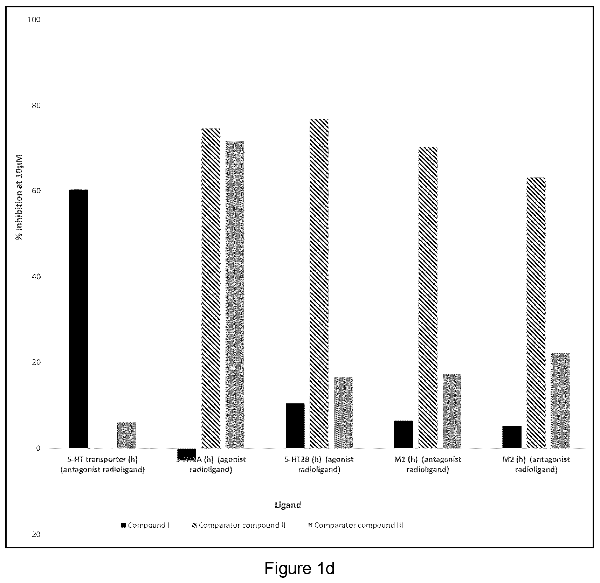

*In vitro* pharmacology binding assays were carried out for compound (I), comparative compound (II) and comparative compound (III) to evaluate the percentage of inhibition against 44 ligands (including receptors, transporters, ion channels, enzymes and kinases) at a concentration of 10µM of compound (I), comparative compound (II) or comparative compound (III). Compound binding was calculated as a percentage of inhibition of the binding of a radioactively labelled ligand specific for each target. Results showing an inhibition higher than 50% are considered to represent discernible effects of compound (I), comparative compound (II) or comparative compound (III). Results for all 44 ligands tested are shown in Figures 1a, 1b, and 1c, whilst Figure 1d shows only the results showing an inhibition higher than 50%. The *in vitro* pharmacology binding assays for compound (I) and comparative compounds (II) and (III) was carried out separately over different days, and compared in Figures 1a, 1b, 1c, and 1d.

From Figures 1a, 1b, 1c, and 1d, it can be seen that compound (I) has a different CEREP profile than both of comparative compounds (II) and (III). Compound (I) demonstrated inhibition higher than 50% for only a single ligand, i.e. fewer (and different) ligands than comparative compounds (II) and/or (III).

### Proof-of-concept Studies

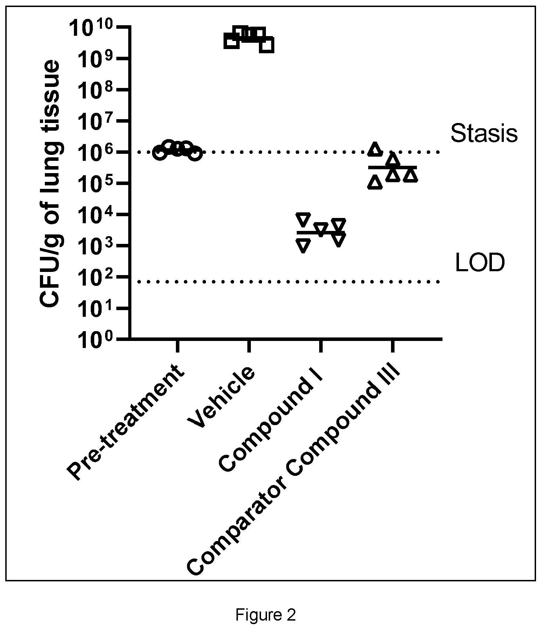

Compound (I) and comparative compound (III) were evaluated in proof-of-concept studies in murine models for the treatment of a respiratory infection with Gram-negative bacteria of the order Enterobacterales, a bacteraemia or bloodstream infection with Gram-negative bacteria of the order Enterobacterales, and a urinary tract infection with Gram-negative bacteria of the order Enterobacterales. Results are shown in Figures 2, 3, and 4a-c. Each dot, triangle or square indicator represents a single mouse. LOD refers to the limit of detection of the bacteria. Stasis shows the level of bacteria (colony-forming unit (CFU)) in the subject pre-treatment. Each of the proof-of-concept studies was carried out separately. By vehicle is meant pharmaceutically acceptable carrier or excipient.

For the proof-of-concept study for the treatment of a respiratory infection caused or exacerbated by Gram-negative bacteria of the order Enterobacterales, CD-1 mice were infected with *Klebsiella pneumoniae* ATCC 43816. Two hours after infection ('pre-treatment' in Figure 2), a vehicle (20% hydroxypropyl β cyclodextrin) containing no compound (I) or comparative compound (III), or a vehicle (20% hydroxypropyl β cyclodextrin) containing compound (I) or comparative compound (III), was administered by IV bolus at 20mg/kg, TID (8 hrs apart), over one day.

From Figure 2, it can be seen that, at the end of the study (26 h from initial infection: 'compound (I)' and 'comparative compound (III)' in Figure 2) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. In fact, the CFU value for comparative compound (III) in Figure 2 remained relatively close to the stasis level, i.e. the instance in which no killing of the bacteria has occurred. From Figure 2, it can be seen that at the end of the study (26h from initial infection), compound (I) reduced bacterial burden in the lung by 6.24 log compared to vehicle, whereas comparative compound (III) only demonstrated a 4.16 log reduction compared to vehicle. Compound (I) is thus 2 log more efficacious than comparative compound (III) in the treatment of a respiratory infection with *Klebsiella pneumoniae,* in particular multidrug-resistant *Klebsiella pneumonia.*

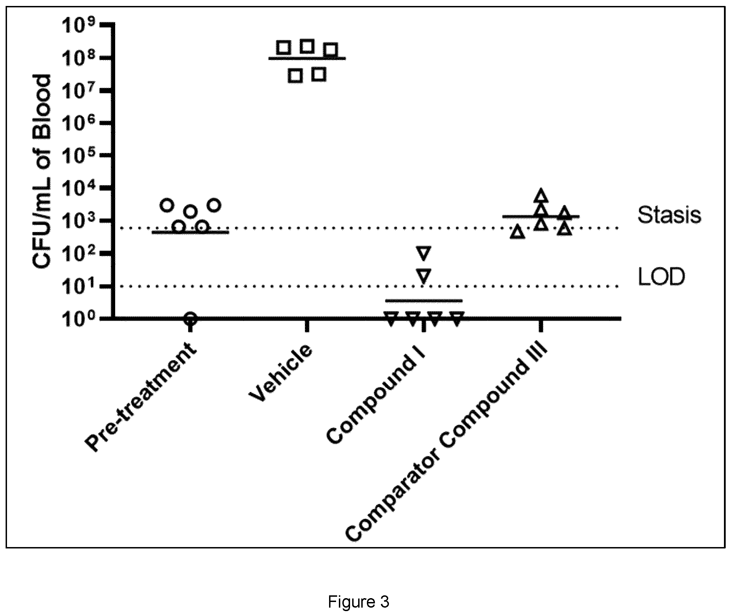

For the proof-of-concept study for the treatment of a bacteraemia or bloodstream infection with Gram-negative bacteria of the order Enterobacterales, CD-1 mice were infected with human urine isolate *E. coli* BAA-2469 (NDM-1 positive). One hour after infection ('pre-treatment' in Figure 3), a vehicle (20% hydroxypropyl β cyclodextrin) containing no compound (I) or comparative compound (III), or a vehicle (20% hydroxypropyl β cyclodextrin) containing compound (I) or comparative compound (III), was administered by as a single dose IV bolus at 20 mg/kg.

From Figure 3, it can be seen that, at the end of the study (9h from initial infection: 'compound (I)' and 'comparative compound (III)' in Figure 3) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. In fact, the CFU value for comparative compound (III) in Figure 3 remained on or above the stasis level, i.e. the instance in which no killing of the bacteria has occurred. From Figure 3, it can be seen that at the end of the study (9h after initial infection), compound (I) reduced the bacterial burden in the blood below the limit of detection (LOD), with a 7.43 log reduction compared to vehicle. In contrast, comparative compound (III) only demonstrated a 4.86 log reduction compared to vehicle. Compound (I) is thus 2.5 log more efficacious than comparative compound (III) in the treatment of a bacteraemia or bloodstream infection with *Escherichia Coli,* in particular multidrug-resistant *Escherichia Coli.*

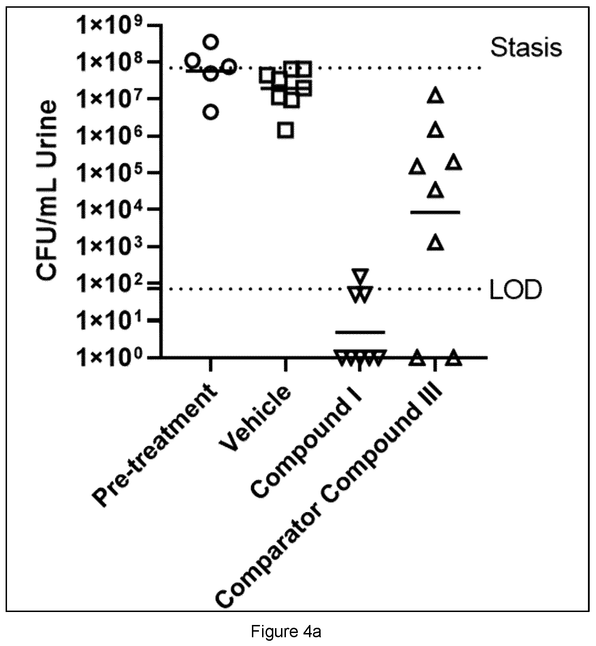

For the proof-of-concept study for the treatment of a urinary tract infection with Enterobacterales, female C3H/HeN mice were infected with E. *coli* UTI89. Twenty-four hours after infection ('pre-treatment' in Figures 4a-c), a vehicle (20% hydroxypropyl β cyclodextrin) containing no compound (I) or comparative compound (III), or a vehicle (20% hydroxypropyl β cyclodextrin) containing compound (I) or comparative compound (III), was administered by IV bolus of 20 mg/kg, TID (8 hrs apart), over 3 days.

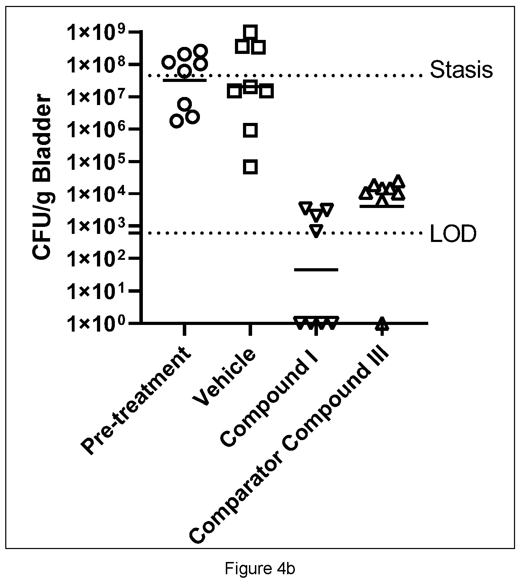

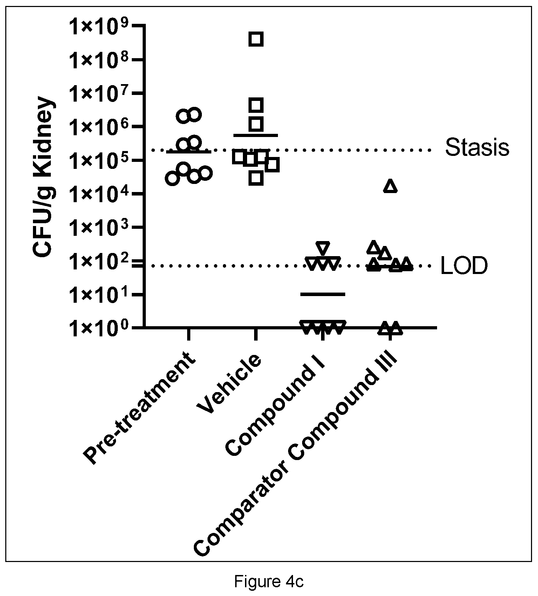

As urinary tract infections may affect the bladder and kidneys, bacterial burden in each of the urine, bladder and kidney was assessed (Figures 4a-c respectively). From Figures 4a and 4b, it can be seen that, at the end of the study (96h after initial infection: 'compound (I)' and 'comparative compound (III)' in Figures 4a and 4b) the colony-forming unit (CFU) was unexpectedly significantly reduced for compound (I) with respect to comparative compound (III), i.e. the bacterial burden has been significantly reduced through treatment with compound (I) versus comparative compound (III), compared to pre-treatment and vehicle. From Figure 4c, it can be seen that, at the end of the study (96h after initial infection: 'compound (I)' and 'comparative compound (III)' in Figures 4c) the colony-forming unit (CFU) was also reduced for compound (I) with respect to comparative compound (III), i.e. the bacterial burden has been reduced through treatment with compound (I) versus comparative compound (III), compared to pre-treatment and vehicle. From Figure 4a, it can be seen that at the end of the study (96h after initial infection), compound (I) reduced the bacterial burden in the urine to below the limit of detection (LOD), with a 6.59 log reduction compared to vehicle. In contrast, comparative compound (III) only demonstrated a reduction of 3.36 log compared to vehicle. Compound (I) is thus 3 log more efficacious than comparative compound (III) in reducing the CFU in the urine, and thus facilitates the treatment of urinary tract infections (UTIs) with *Escherichia coli.* From Figure 4b, it can be seen that at the end of the study (96h after initial infection) compound (I) reduced the bacterial burden in the bladder by 5.67 log compared to vehicle. In comparison, comparative compound (III) only demonstrated a reduction of 3.71 log compared to vehicle. Compound (I) is thus 3 log more efficacious than comparative compound (III) in reducing the CFU in the bladder, and thus facilitates the treatment of urinary tract infections (UTIs) with *Escherichia coli.* From Figure 4c, it can be see that at the end of the study (96h after initial infection) compound (I) reduced the bacterial burden in the kidney by 4.73 log compared to vehicle.

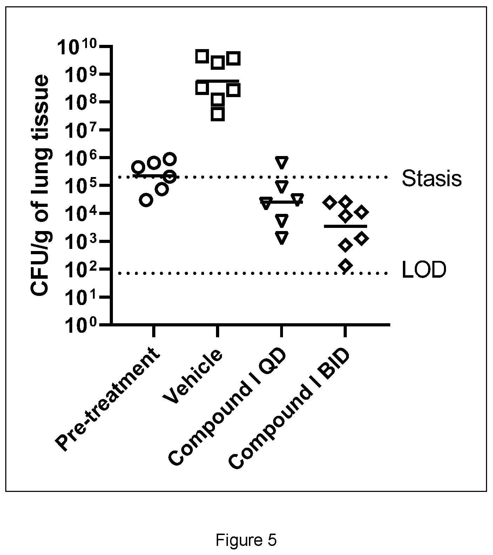

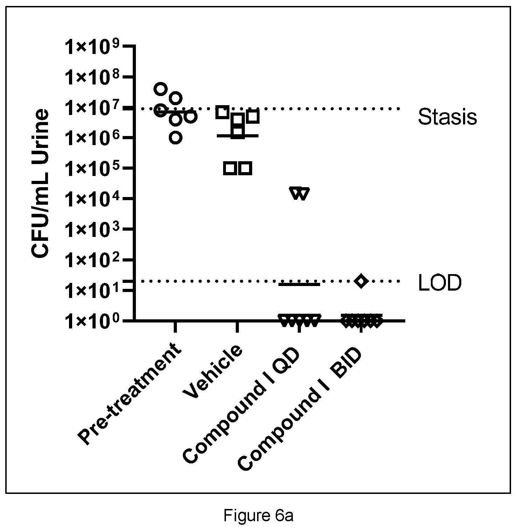

Compound (I) was evaluated in further proof-of-concept studies in murine models for the treatment of a respiratory infection with Gram-negative bacteria of the order Enterobacterales, and a urinary tract infection with Gram-negative bacteria of the order Enterobacterales. Results are shown in Figures 5 and 6a-c. Each dot, triangle or square indicator represents a single mouse. LOD refers to the limit of detection of the bacteria. Stasis shows the level of bacteria (colony-forming unit (CFU)) in the subject pre-treatment. Each of the proof-of-concept studies was carried out separately. By vehicle is meant pharmaceutically acceptable excipient or carrier.

For the further proof-of-concept study for the treatment of a respiratory infection caused or exacerbated by Gram-negative bacteria of the order Enterobacterales, male CD-1 mice were infected with *Klebsiella pneumoniae* ATCC 43816.

Two hours after infection ('pre-treatment' in Figure 5), mice were either treated with a vehicle control (phosphate buffer) containing no compound (I), or a vehicle (phosphate buffer) containing compound (I), administered by intravenous infusion for 1 hour (QD) with a dose of 20 mg/kg of compound (I). For the mice that received the vehicle control containing no compound (I), and some mice who received the vehicle containing compound (I), an additional 1-hour continuous intravenous infusion was administered 3 hours after the time at which the first administration begun (BID, 3 hrs apart).

From Figure 5, it can be seen that, at the end of the study (26 h from initial infection: 'compound (I) QD' and 'compound (I) BIO' in Figure 5) the colony-forming unit (CFU) was reduced for compound (I), i.e. the bacterial burden has been reduced through treatment with compound (I), compared to pre-treatment and vehicle ('vehicle BID' in Figure 5). This was especially seen for compound (I) when administered twice (BID, 3 hrs apart) (P value of <0.0001 compared to both pre-treatment and vehicle). The P value for compound (I) when administered once (QD) was 0.0042 compared to pre-treatment and <0.0001 compared to vehicle). From Figure 5, it can be seen that at the end of the study (26h from initial infection), compound (I) reduced bacterial burden in the lung by 4.35 and 5.20 log (for once (QD) and twice (BID, 3 hrs apart) administration respectively) compared to vehicle, and 0.95 and 1.80 log ( for once (QD) and twice (BID, 3 hrs apart) administration respectively) compared to pre-treatment (stasis levels). Compound (I) is thus effective in the treatment of a respiratory infection with *Klebsiella pneumoniae,* in particular multidrug-resistant *Klebsiella pneumonia.*

For the further proof-of-concept study for the treatment of a urinary tract infection with Enterobacterales, female C3H/HeN mice were infected with *E*. *coli* UTI89. Twenty-four hours after infection ('pre-treatment' in Figures 6a-c), mice were either treated with a vehicle control (20% hydroxypropyl β cyclodextrin) containing no compound (I), or a vehicle (20% hydroxypropyl β cyclodextrin) containing compound (I), administered by intravenous infusion for 1 hour (QD) with a dose of 20 mg/kg of compound (I). For some mice who received vehicle containing compound (I), an additional 1-hour continuous intravenous infusion was administered 5 hours after the time at which the first administration begun (BID, 5 hrs apart). The once or twice daily administration was continued for three days. For QD, administration took place between 24 to 25 hours, 48 to 49 hours, and 72 to 73 hours after infection, and for BID, administration took place between 24 to 25 hours, 29 to 30 hours, 48 to 49 hours, 53 to 54 hours, 72 to 73 hours and 77 to 78 hours after infection.

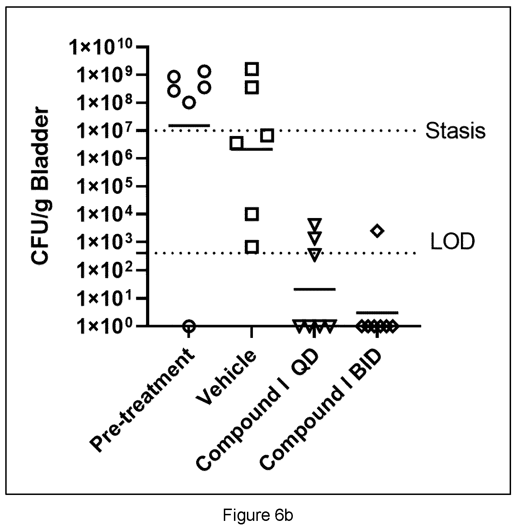

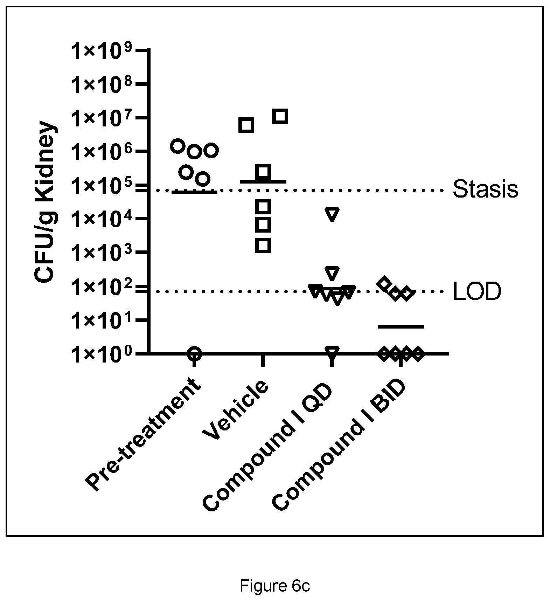

As urinary tract infections may affect the bladder and kidneys, bacterial burden in each of the urine, bladder and kidney was assessed (Figures 6a-c respectively). From Figures 6a, 6b and 6c, it can be seen that, at the end of the study (96h after initial infection: 'compound (I) QD' and 'compound (I) BID' in Figures 6a, 6b and 6c) the colony-forming unit (CFU) was significantly reduced for compound (I), i.e. the bacterial burden has been significantly reduced through treatment with compound (I), compared to pre-treatment and vehicle. This was especially seen for compound (I) when administered twice daily (BID, 5 hrs apart). From Figure 6a, it can be seen that at the end of the study (96h after initial infection), compound (I) reduced the bacterial burden in the urine to below the limit of detection (LOD) for both QD and BID daily administration, with a 4.86 and 5.87 log reduction (for once (QD) and twice (BID, 5 hrs apart) daily administration respectively) compared to vehicle. The P value for compound (I), whether administered once (QD) or twice (BID, 5 hrs apart) was <0.0001 compared to vehicle. Compound (I) is thus effective in reducing CFU in the urine, and thus facilitates the treatment of urinary tract infections (UTIs) with *Escherichia coli.* From Figure 6b, it can be seen that at the end of the study (96h after initial infection) compound (I) reduced the bacterial burden in the bladder to below the limit of detection (LOD) for both QD and BID daily administration, with a 5.01 and 5.84 log reduction (for once (QD) and twice (BID, 5 hrs apart) daily administration respectively) compared to vehicle.

The P value for administration once per day (QD) was 0.001 compared to vehicle, and for administration twice per day (BID, 5 hrs apart) was 0.0002. Compound (I) is thus effective in reducing the CFU in the bladder, and thus facilitates the treatment of urinary tract infections (UTIs) with *Escherichia coli.* From Figure 6c, it can be see that at the end of the study (96h after initial infection) compound (I) reduced the bacterial burden in the kidney to below the limit of detection (LOD) for BID administration, with 3.17 and 4.3 log reduction (for once (QD) and twice (BID, 5 hrs apart) daily administration respectively) compared to vehicle. The P value for administration once per day (QD) was 0.0007 compared to vehicle, and for administration twice per day (BID, 5 hrs apart) was <0.0001. Compound (I) is thus effective in reducing the CFU in the kidney, and thus facilitates the treatment of urinary tract infections (UTIs) with *Escherichia coli.*

## Claims

1. A process for the production of compound (I) 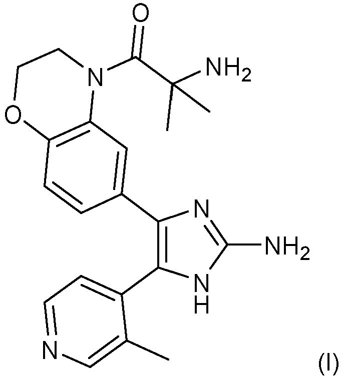 or a pharmaceutically acceptable salt thereof, the process comprising deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof

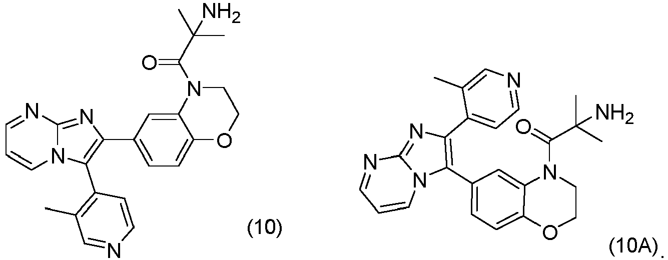

2. The process according to claim 1, wherein the process is for the production of compound (I).

3. The process according to claim 1 or 2, wherein the process comprises the deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A); preferably wherein the process comprises the deprotection of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A).

4. The process according to claim 1 or 2, wherein the process comprises the deprotection of a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably of a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A); and/or wherein the process comprises the deprotection of the dihydrochloride salt (10C) of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one: 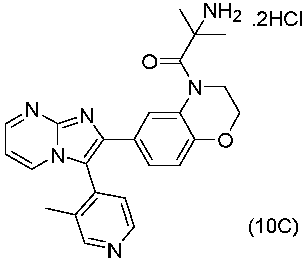 and/or the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one:

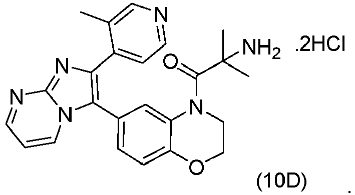

5. The process according to any of claims 1 to 4, wherein the deprotection is acid catalysed using an acid catalyst, or wherein the deprotection is base catalysed using a base catalyst, or wherein a deprotection agent is utilised and selected from NH₂OH.HCl (hydroxylamine hydrochloride) and hydrazine, or the hydrate thereof; preferably wherein the deprotection agent is NH₂OH.HCl (hydroxylamine hydrochloride); and optionally wherein the deprotection agent is accompanied by a base selected from 1,8-diazabicyclo(5.4.0)undec-7-ene (DBU), N,N-diisopropylethylamine (DIPEA), piperidine, morpholine, pyrollidine, and triethylamine (TEA), preferably, 1,8-diazabicyclo(5.4.0)undec-7-ene (DBU), N,N-diisopropylethylamine (DIPEA) and triethylamine (TEA), and more preferably triethylamine (TEA); and/or wherein the deprotection comprises a solvent selected from isopropyl alcohol (IPA), ethanol (EtOH), dichloromethane (DCM), methanol (MeOH), and acetonitrile (CH₃CN), preferably methanol, dichloromethane and isopropyl alcohol (IPA), and more preferably isopropyl alcohol (IPA).

6. The process according to claim 1, 2, 4, or claim 5 where dependent thereon, wherein the deprotection may be carried out at a temperature of from 30 to 80 °C, such as from 40 to 70 °C, preferably 40 to 60 °C.

7. The process according to any of claims 1 to 6, wherein the process further comprises a preceding step comprising an amino acid coupling of 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A), 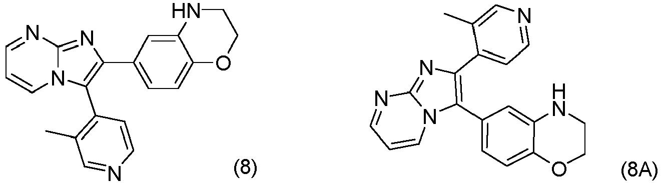 to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof; preferably wherein the preceding step comprises amino acid coupling of 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A) with 2-amino-2-methylpropanoic acid (9) in the presence of a coupling agent or coupling agent precursor to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), or the salts thereof 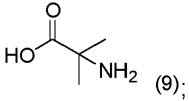 and more preferably wherein the preceding step comprises amino acid coupling of 6-(3-(3-methylpyridin-4-yl)imidazo[1,2-a]pyrimidin-2-yl)-3,4-dihydro-2H-benzo[b][1,4]oxazine (8) and 6-(2-(3-methylpyridin-4-yl)imidazo[1,2-a]pyrimidin-3-yl)-3,4-dihydro-2H-benzo[b][1,4]oxazine (8A), to form 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A).

8. The process according to claim 7, where dependent upon claims 1 to 3, or claim5 or 6 when dependent on claims 1 to 3, wherein 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A) is formed, and wherein the coupling agent or coupling agent precursor is selected from thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃, preferably the coupling agent or coupling agent precursor is thionyl chloride (SOCl₂); and optionally wherein the amino acid coupling uses a solvent selected from N,N'dimethylpropyleneurea (DMPU), dimethyl sulfoxide (DMSO), dimethylformamide (DMF), hexamethylphosphoramide (HMPA) and acetonitrile (ACN), or combinations thereof, preferably the solvent is selected from acetonitrile, N,N'dimethylpropyleneurea (DMPU) and hexamethylphosphoramide (HMPA), or combinations thereof, and more preferably, the solvent is selected from acetonitrile and hexamethylphosphoramide (HMPA), or combinations thereof; and/or wherein the amino acid coupling uses N,N'dimethylpropyleneurea (DMPU) or hexamethylphosphoramide (HMPA) in combination with thionyl chloride.

9. The process according to claim 7, where dependent upon claims 1, 2, 4, or claim 5 or 6 when dependent on claims 1, 2, or 4, wherein a salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10) and/or a salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably a hydrochloride salt of 2-amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one (10), and/or a hydrochloride salt of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one (10A), preferably the dihydrochloride salt (10C) of -Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-one and/or the dihydrochloride salt (10D) of 2-amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-one, is formed, and wherein the coupling agent or coupling agent precursor is selected from thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), (1-cyano-2-ethoxy-2-oxoethylidenaminooxy)dimethylamino-morpholino-carbenium hexafluorophosphate (COMU), hydroxybenotriazole, fluoro-dipyrrolidinocarbenium hexafluorophosphate (BTFFH), Ti(iOPr)₄ and B(OCH₂CF₃)₃, preferably the coupling agent or coupling agent precursor is selected from hydroxybenotriazole, 1-ethyl-2-)3'-dimethylaminopropyl)carbodiimide.HCl, 1,1'-carbonyldiimidazole (CDI), (1-cyano-2-ethoxy-2-oxoethylidenaminooxy)dimethylamino-morpholino-carbenium hexafluorophosphate (COMU), propanephosphonic acid anhydride (T3P), and thionyl chloride (SOCl₂), and more preferably, the coupling agent or coupling agent precursor is thionyl chloride; and optionally wherein the amino acid coupling reaction uses a solvent selected from N,N'dimethylpropyleneurea (DMPU), dimethyl sulfoxide (DMSO), dimethylformamide (DMF), hexamethylphosphoramide (HMPA), acetonitrile (ACN), N-methyl-2-pyrolidone (NMP), 1,3-dimethyl-2-imidazolidinone (DMI), gamma-valerolactone (GVL), N-methylmorpholine (NMM), sulfolane, dichloromethane (DCM), or combinations thereof, preferably the solvent is selected from N-methyl-2-pyrolidone (NMP), 1,3-dimethyl-2-imidazolidinone (DMI), gamma-valerolactone (GVL), N-methylmorpholine (NMM), sulfolane, dichloromethane (DCM), and acetonitrile, or combinations thereof, and more preferably, the solvent is selected from acetonitrile or sulfolane, most preferably acetonitrile; and/or wherein a base is also used as a reagent in the amino acid coupling, preferably a tertiary amine, more preferably pyridine.

10. The process according to any of claims 7 to 9, wherein the process further comprises an additional preceding step comprising the deacylation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to form 6-(3-(3-methylpyridin-4-yl)imidazo[1,2-a]pyrimidin-2-yl)-3,4-dihydro-2H-benzo[b][1,4]oxazine (8) and/or 6-(2-(3-methylpyridin-4-yl)imidazo[1,2-a]pyrimidin-3-yl)-3,4-dihydro-2H-benzo[b][1,4]oxazine (8A); preferably wherein the additional preceding step comprises the deacylation of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) to form 6-(3-(3-methylpyridin-4-yl)imidazo[1,2-a]pyrimidin-2-yl)-3,4-dihydro-2H-benzo[b][1,4]oxazine (8) and 6-(2-(3-methylpyridin-4-yl)imidazo[1,2-a]pyrimidin-3-yl)-3,4-dihydro-2H-benzo[b][1,4]oxazine (8A); optionally wherein the additional step is acid or base catalysed with an acid or base catalyst, preferably base catalysed with a base catalyst; and more preferably wherein the additional step is base catalysed using NaOH, preferably 30% aq. NaOH solution.

11. A process for the production of compound (I) 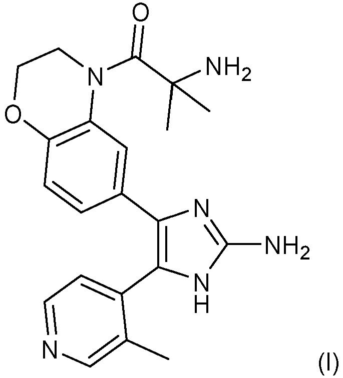 or a pharmaceutically acceptable salt thereof, the process comprising the following step:
coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A)

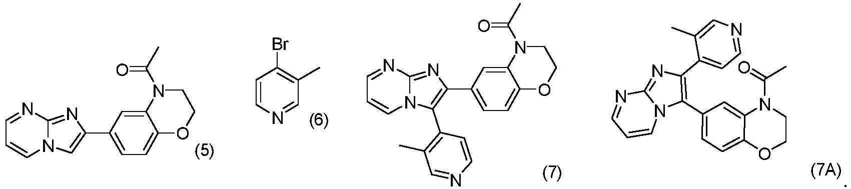

12. A use of a metal-scavenging agent in a process for the production of compound (I) 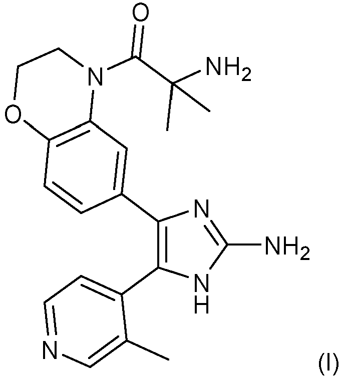 or a pharmaceutically acceptable salt thereof, wherein the process comprises the step of coupling of 1-(6-(imidazo[1,2-a]pyrimidin-2-yl)-2,3-dihydro-4H-benzo[b][1,4]oxazin-4-yl)ethan-1-one (5) with 4-bromo-3-methylpyridine (6), or a salt thereof, in the presence of a metal catalyst to form 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), 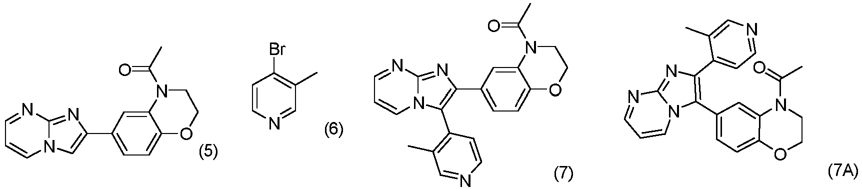 wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) are treated with the metal-scavenging agent; preferably wherein the metal-scavenging treatment comprises treatment with a metal-scavenging agent and the metal-scavenging agent is trithiocyanuric acid.

13. The process or use according to claim 11 or 12, wherein compound (I) is formed; and/or wherein -(6-(3-(3-methylpyridin-4-yl)imidazo[1,2-a]pyrimidin-2-yl)-2,3-dihydro-4H-benzo[b][1,4]oxazin-4-yl)ethan-1-one (7) and 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) are formed; optionally wherein 1-(6-(imidazo[1,2-a]pyrimidin-2-yl)-2,3-dihydro-4H-benzo[b][1,4]oxazin-4-yl)ethan-1-one (5) is coupled with 4-bromo-3-methylpyridine (6); and/or wherein 1-(6-(imidazo[1,2-a]pyrimidin-2-yl)-2,3-dihydro-4H-benzo[b][1,4]oxazin-4-yl)ethan-1-one (5) is coupled with a salt of 4-bromo-3-methylpyridine (6), preferably 4-bromo-3-methylpyridine hydrochloride (6A)

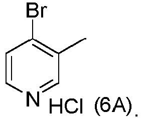

14. The process according to claim 11, or claim 13 when dependent thereon, wherein 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A) undergo a metal-scavenging treatment, preferably wherein the metal-scavenging treatment comprises treatment with a metal-scavenging agent; preferably wherein the metal-scavenging treatment comprises treatment with a metal-scavenging agent and the metal-scavenging agent is trithiocyanuric acid.

15. The process or use according to any of claims 11 to 14, wherein the metal catalyst is a transition metal-based catalyst such as a platinum-based catalyst, palladium-based catalyst, and iridium-based catalyst, preferably the metal catalyst is a palladium-based catalyst, and more preferably the metal catalyst is palladium (II) acetate.

16. A process for the production of compound (I) 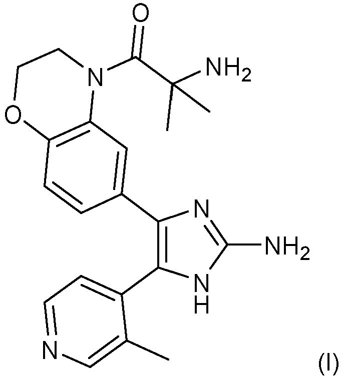 or a pharmaceutically acceptable salt thereof, the process comprising amino acid coupling of 4-(3,4-Dihydro-2H-benzo[b][1,4]oxazin-6-yl)-5-(3-methylpyridin-4-yl)-1H-imidazol-2-amine (12) with 2-amino-2-methylpropanoic acid (9) in the presence of a coupling agent or coupling agent precursor:

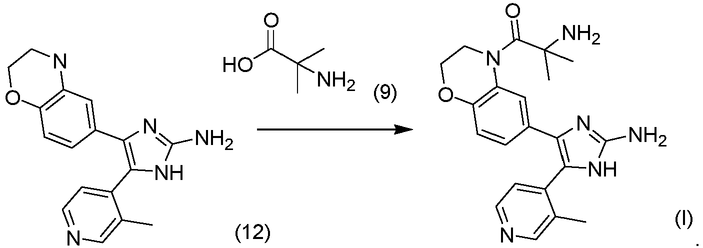

17. The process according to claim 16, wherein compound (I) is formed; and/or wherein the coupling agent or coupling agent precursor is from thionyl chloride (SOCl₂), 1-[Bis(dimethylamino)methylene]-1H-1,2,3-triazolo[4,5-b]pyridinium 3-oxide hexafluorophosphate (HATU), 4-dimethylaminopyridine (DMAP), propanephosphonic acid anhydride (T3P), 1-ethyl-2-)3'-dimethylaminopropyl)carbodiimide.HCl, 1,1'-carbonyldiimidazole (CDI), Ghosez reagent (1-chloro---N,N,2-trimethyl-1-propenylamine), fluoro-dipyrrolidinocarbenium hexafluorophosphate (BTFFH), Ti(iOPr)₄ and B(OCH₂CF₃)₃, preferably, the coupling agent or coupling agent precursor is thionyl chloride (SOCl₂); and optionally wherein the amino acid coupling uses a solvent selected from N,N'dimethylpropyleneurea (DMPU), dimethyl sulfoxide (DMSO), dimethylformamide (DMF), hexamethylphosphoramide (HMPA) and acetonitrile (ACN), or combinations thereof, preferably a solvent selected from acetonitrile, N,N'dimethylpropyleneurea (DMPU) and hexamethylphosphoramide (HMPA), or combinations thereof, and more preferably a solvent selected from acetonitrile and hexamethylphosphoramide (HMPA), or combinations thereof, and/or wherein the amino acid coupling uses N,N'dimethylpropyleneurea (DMPU) or hexamethylphosphoramide (HMPA) in combination with thionyl chloride.

18. The process according to claim 16 or 17, wherein the process further comprises a preceding step for the formation of compound (12) of the deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12) 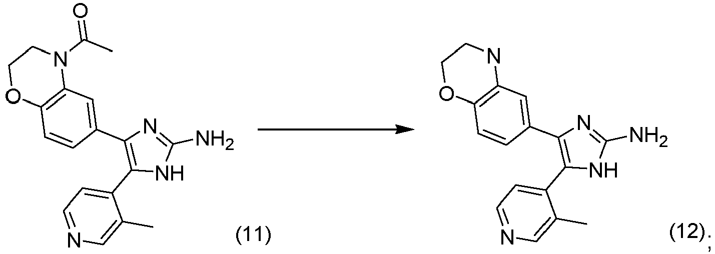 and optionally wherein the deacylation may be base or acid catalysed, preferably acid catalysed; and/or wherein the process further comprises an additional preceding step for the formation of 1-(6-(2-Amino-5-(3-methylpyridin-4-yl)-1H-imidazol-4-yl)-2,3-dihydro-4H-benzo[b][1,4]oxazin-4-yl)ethan-1-one (11) of the deprotection of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), to form 1-(6-(2-Amino-5-(3-methylpyridin-4-yl)-1H-imidazol-4-yl)-2,3-dihydro-4H-benzo[b][1,4]oxazin-4-yl)ethan-1-one (11); preferably wherein the deprotection may be acid catalysed using an acid catalyst or the deprotection may be base catalysed using a base catalyst or a deprotection agent is utilised and selected from NH₂OH.HCl (hydroxylamine hydrochloride) and hydrazine, or the hydrate thereof; preferably wherein a deprotection agent is used and is NH₂OH.HCl (hydroxylamine hydrochloride); and optionally wherein the deprotection comprises a solvent selected from isopropyl alcohol (IPA), ethanol (EtOH), dichloromethane (DCM), methanol (MeOH), and acetonitrile (CH₃CN), preferably selected from methanol, dichloromethane and isopropyl alcohol (IPA), and more preferably, wherein the solvent is isopropyl alcohol (IPA).

19. A process for the production of compound (I), 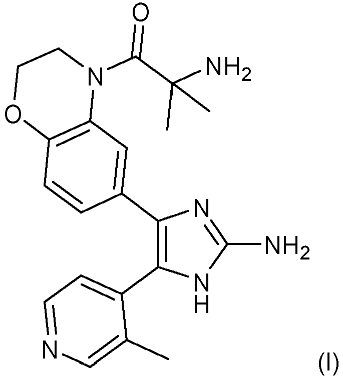 the process comprising the step of deacylation of 1-(6-(2-Amino-5-(3-methylpyridin-4-yl)-1H-imidazol-4-yl)-2,3-dihydro-4H-benzo[b][1,4]oxazin-4-yl)ethan-1-one (11) to form 4-(3,4-Dihydro-2H-benzo[b][1,4]oxazin-6-yl)-5-(3-methylpyridin-4-yl)-1H-imidazol-2-amine (12) 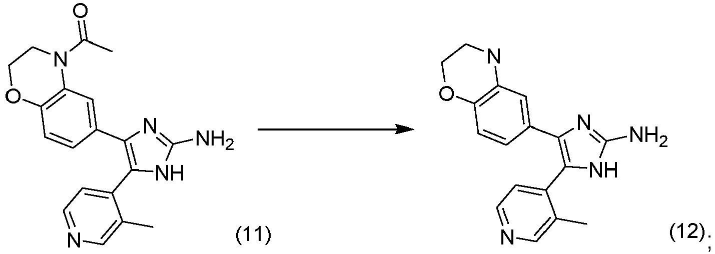 and optionally wherein the deacylation may be base or acid catalysed, preferably acid catalysed; and/or wherein the process further comprises an additional preceding step for the formation of 1-(6-(2-Amino-5-(3-methylpyridin-4-yl)-1H-imidazol-4-yl)-2,3-dihydro-4H-benzo[b][1,4]oxazin-4-yl)ethan-1-one (11) of the deprotection of 1-(6-(3-(3-methylpyridin-4-yl)imidazo[1,2-a]pyrimidin-2-yl)-2,3-dihydro-4H-benzo[b][1,4]oxazin-4-yl)ethan-1-one (7) and/or 1-(6-(2-(3-methylpyridin-4-yl)imidazo[1,2-a]pyrimidin-3-yl)-2,3-dihydro-4H-benzo[b][1,4]oxazin-4-yl)ethan-1-one (7A), to form 1-(6-(2-Amino-5-(3-methylpyridin-4-yl)-1H-imidazol-4-yl)-2,3-dihydro-4H-benzo[b][1,4]oxazin-4-yl)ethan-1-one (11); preferably wherein the deprotection may be acid catalysed using an acid catalyst or the deprotection may be base catalysed using a base catalyst or a deprotection agent is utilised and selected from NH₂OH.HCl (hydroxylamine hydrochloride) and hydrazine, or the hydrate thereof; preferably wherein a deprotection agent is used and is NH₂OH.HCl (hydroxylamine hydrochloride); and optionally wherein the deprotection comprises a solvent selected from isopropyl alcohol (IPA), ethanol (EtOH), dichloromethane (DCM), methanol (MeOH), and acetonitrile (CH₃CN), preferably selected from methanol, dichloromethane and isopropyl alcohol (IPA), and more preferably, wherein the solvent is isopropyl alcohol (IPA).

## Patentansprüche

1. Verfahren zur Herstellung von Verbindung (I) 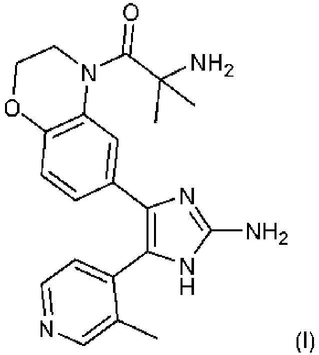 oder eines pharmazeutisch verträglichen Salzes davon, wobei das Verfahren die Entschützung von 2-Amino-2-methyl-1-(6-(3-(3-methylpyridin-4-yl)imidaz [1,2-a]pyrimidin-2-yl)-2,3-dihydro-4H-benzo[b][1,4]oxazin-4-yl)propan-1-on (10) und/oder 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) oder deren Salze umfasst.

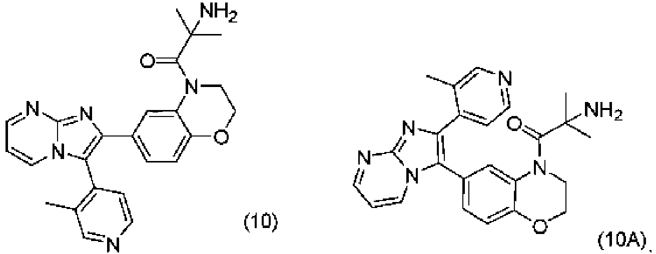

2. Verfahren nach Anspruch 1, wobei das Verfahren zur Herstellung von Verbindung (I) dient.

3. Verfahren nach Anspruch 1 oder 2, wobei das Verfahren die Entschützung von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) umfasst; wobei bevorzugt das Verfahren die Entschützung von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) umfasst.

4. Verfahren nach Anspruch 1 oder 2, wobei das Verfahren die Entschützung eines Salzes von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder eines Salzes von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A), bevorzugt eines Hydrochloridsalzes von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder eines Hydrochloridsalzes von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) umfasst; und/oder wobei das Verfahren die Entschützung des Dihydrochloridsalzes (10C) von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on umfasst: 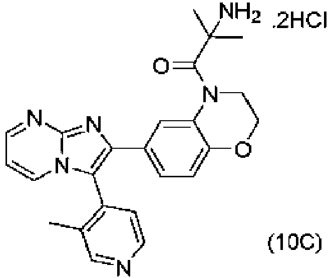 und/oder des Dihydrochloridsalzes (10D) von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on:

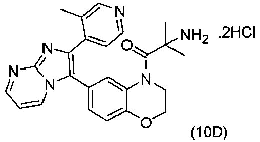

5. Verfahren nach einem der Ansprüche 1 bis 4, wobei die Entschützung säurekatalysiert unter Verwendung eines Säurekatalysators erfolgt, oder wobei die Entschützung basenkatalysiert unter Verwendung eines Basenkatalysators erfolgt, oder wobei ein Entschützungsmittel verwendet wird, ausgewählt aus NH₂OH·HCl (Hydroxylaminhydrochlorid) und Hydrazin oder dessen Hydrat; bevorzugt ist das Entschützungsmittel NH₂OH·HCl (Hydroxylaminhydrochlorid); und optional wird das Entschützungsmittel zusammen mit einer Base verwendet, ausgewählt aus 1,8-Diazabicyclo(5.4.0)undec-7-en (DBU), N,N-Diisopropylethylamin (DIPEA), Piperidin, Morpholin, Pyrrolidin und Triethylamin (TEA), bevorzugt 1,8-Diazabicyclo(5.4.0)undec-7-en (DBU), N,N-Diisopropylethylamin (DIPEA) und Triethylamin (TEA), stärker bevorzugt Triethylamin (TEA); und/oder wobei die Entschützung ein Lösungsmittel umfasst, ausgewählt aus Isopropylalkohol (IPA), Ethanol (EtOH), Dichlormethan (DCM), Methanol (MeOH) und Acetonitril (CH₃CN), bevorzugt Methanol, Dichlormethan und Isopropylalkohol (IPA), stärker bevorzugt Isopropylalkohol (IPA).

6. Verfahren nach Anspruch 1, 2, 4 oder Anspruch 5 sofern dieser davon abhängig ist, wobei die Entschützung bei einer Temperatur von 30 bis 80 °C, beispielsweise von 40 bis 70 °C, bevorzugt von 40 bis 60 °C durchgeführt werden kann.

7. Verfahren nach einem der Ansprüche 1 bis 6, wobei das Verfahren weiter einen vorhergehenden Schritt umfasst, der eine Aminosäurekupplung von 6-(3-(3-methylpyridin-4-yl)imidazo[1,2-a]pyrimidin-2-yl)-3,4-dihydro-2H-benzo[b][1,4]oxazin (8) und/oder 6-(2-(3-methylpyridin-4-yl)imidazo[1,2-a]pyrimidin-3-yl)-3,4-dihydro-2H-benzo[b][1,4]oxazin (8A) umfasst, 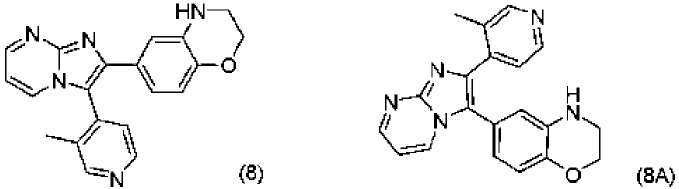 um 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) oder deren Salze herzustellen; bevorzugt umfasst der vorhergehende Schritt eine Aminosäurekupplung von 6-(3-(3-methylpyridin-4-yl)imidazo[1,2-a]pyrimidin-2-yl)-3,4-dihydro-2H-benzo[b][1,4]oxazin (8) und/oder 6-(2-(3-methylpyridin-4-yl)imidazo[1,2-a]pyrimidin-3-yl)-3,4-dihydro-2H-benzo[b][1,4]oxazin (8A) mit 2-Amino-2-methylpropansäure (9) in Gegenwart eines Kupplungsreagenzes oder einer Kupplungsreagenzvorstufe, um 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) oder deren Salze 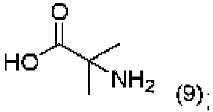 und stärker bevorzugt umfasst der vorhergehende Schritt eine Aminosäurekupplung von 6-(3-(3-methylpyridin-4-yl)imidazo[1,2-a]pyrimidin-2-yl)-3,4-dihydro-2H-benzo[b][1,4]oxazin (8) und 6-(2-(3-methylpyridin-4-yl)imidazo[1,2-a]pyrimidin-3-yl)-3,4-dihydro-2H-benzo[b][1,4]oxazin (8A), um 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) herzustellen.

8. Verfahren nach Anspruch 7, abhängig von den Ansprüchen 1 bis 3 oder Anspruch 5 oder 6, sofern diese von den Ansprüchen 1 bis 3 abhängig sind, wobei 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A) hergestellt werden, und wobei das Kupplungsreagenz oder die Kupplungsreagenzvorstufe ausgewählt ist aus Thionylchlorid (SOCl₂), 1-[Bis(dimethylamino)methylen]-1H-1,2,3-triazolo[4,5-b]pyridinium-3-oxid-hexafluorphosphat (HATU), 4-Dimethylaminopyridin (DMAP), Propanphosphonsäureanhydrid (T3P), 1-Ethyl-2-(3-dimethylaminopropyl)carbodiimid·HCl, 1,1'-Carbonyldiimidazol (CDI), Ghosez-Reagenz (1-Chlor-N,N,2-trimethyl-1-propenylamin), Fluor-dipyrrolidinocarbeniumhexafluorphosphat (BTFFH), Ti(iOPr)₄ und B(OCH₂CF₃)₃, wobei bevorzugt das Kupplungsreagenz oder die Kupplungsreagenzvorstufe Thionylchlorid (SOCl₂) ist; und optional wobei die Aminosäurekupplung ein Lösungsmittel verwendet, ausgewählt aus N,N'-Dimethylpropylenharnstoff (DMPU), Dimethylsulfoxid (DMSO), Dimethylformamid (DMF), Hexamethylphosphorsäuretriamid (HMPA) und Acetonitril (ACN) oder Kombinationen davon, wobei das Lösungsmittel bevorzugt ausgewählt ist aus Acetonitril, N,N'-Dimethylpropylenharnstoff (DMPU) und Hexamethylphosphorsäuretriamid (HMPA) oder Kombinationen davon, stärker bevorzugt ausgewählt aus Acetonitril und Hexamethylphosphorsäuretriamid (HMPA) oder Kombinationen davon; und/oder wobei die Aminosäurekupplung N,N'-Dimethylpropylenharnstoff (DMPU) oder Hexamethylphosphorsäuretriamid (HMPA) in Kombination mit Thionylchlorid verwendet.

9. Verfahren nach Anspruch 7, abhängig von den Ansprüchen 1, 2 oder 4 oder Anspruch 5 oder 6, sofern diese von den Ansprüchen 1, 2 oder 4 abhängig sind, wobei ein Salz von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder ein Salz von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A), bevorzugt ein Hydrochloridsalz von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on (10) und/oder ein Hydrochloridsalz von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on (10A), stärker bevorzugt das Dihydrochloridsalz (10C) von 2-Amino-2-methyl-1-(6-(3-(3-methylpyridin-4-yl)imidazo[1,2-a]pyrimidin-2-yl)-2,3-dihydro-4H-benzo[b][1,4]oxazin-4-yl)propan-1-on und/oder das Dihydrochloridsalz (10D) von 2-Amino-2-methyl-1-(6-(2-(3-methylpyridin-4-yl)imidazo[1,2-a]pyrimidin-3-yl)-2,3-dihydro-4H-benzo[b][1,4]oxazin-4-yl)propan-1-on hergestellt wird, und wobei das Kupplungsreagenz oder die Kupplungsreagenzvorstufe ausgewählt ist aus Thionylchlorid (SOCl₂), 1-[Bis(dimethylamino)methylen]-1H-1,2,3-triazolo[4,5-b]pyridinium-3-oxid-hexafluorphosphat (HATU), 4-Dimethylaminopyridin (DMAP), Propanphosphonsäureanhydrid (T3P), 1-Ethyl-2-(3-dimethylaminopropyl)carbodiimid·HCl, 1,1'-Carbonyldiimidazol (CDI), Ghosez-Reagenz (1-Chlor-N,N,2-trimethyl-1-propenylamin), (1-Cyano-2-ethoxy-₂-oxoethylidenaminooxy)dimethylamino-morpholino-carbeniumhexafluorphosphat (COMU), Hydroxybenzotriazol, Fluor-dipyrrolidinocarbeniumhexafluorphosphat (BTFFH), Ti(iOPr)₄ und B(OCH₂CF₃)₃, wobei bevorzugt das Kupplungsreagenz oder die Kupplungsreagenzvorstufe ausgewählt ist aus Hydroxybenzotriazol, 1-Ethyl-2-(3-dimethylaminopropyl)carbodiimid·HCl, 1,1'-Carbonyldiimidazol (CDI), (1-Cyano-2-ethoxy-2-oxoethylidenaminooxy)dimethylamino-morpholino-carbeniumhexafluorphosphat (COMU), Propanphosphonsäureanhydrid (T3P) und Thionylchlorid (SOCl₂), stärker bevorzugt Thionylchlorid; und optional wobei die Aminosäurekupplungsreaktion ein Lösungsmittel verwendet, ausgewählt aus N,N'-Dimethylpropylenharnstoff (DMPU), Dimethylsulfoxid (DMSO), Dimethylformamid (DMF), Hexamethylphosphorsäuretriamid (HMPA), Acetonitril (ACN), N-Methyl-2-pyrrolidon (NMP), 1,3-Dimethyl-2-imidazolidinon (DMI), Gamma-Valerolacton (GVL), N-Methylmorpholin (NMM), Sulfolan, Dichlormethan (DCM) oder Kombinationen davon, wobei das Lösungsmittel bevorzugt ausgewählt ist aus N-Methyl-2-pyrrolidon (NMP), 1,3-Dimethyl-2-imidazolidinon (DMI), Gamma-Valerolacton (GVL), N-Methylmorpholin (NMM), Sulfolan, Dichlormethan (DCM) und Acetonitril oder Kombinationen davon, stärker bevorzugt ausgewählt aus Acetonitril oder Sulfolan, am stärksten bevorzugt Acetonitril; und/oder wobei zusätzlich eine Base als Reagenz in der Aminosäurekupplung verwendet wird, bevorzugt ein tertiäres Amin, stärker bevorzugt Pyridin.

10. Verfahren nach einem der Ansprüche 7 bis 9, wobei das Verfahren weiter einen zusätzlichen vorhergehenden Schritt umfasst, der die Deacylierung von 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) umfasst, um 6-(3-(3-methylpyridin-4-yl)imidazo[1,2-a]pyrimidin-2-yl)-3,4-dihydro-2H-benzo[b][1,4]oxazin (8) und/oder 6-(2-(3-methylpyridin-4-yl)imidazo[1,2-a]pyrimidin-3-yl)-3,4-dihydro-2H-benzo[b][1,4]oxazin (8A) herzustellen; bevorzugt umfasst der zusätzliche vorhergehende Schritt die Deacylierung von 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A), um 6-(3-(3-methylpyridin-4-yl)imidazo[1,2-a]pyrimidin-2-yl)-3,4-dihydro-2H-benzo[b][1,4]oxazin (8) und 6-(2-(3-methylpyridin-4-yl)imidazo[1,2-a]pyrimidin-3-yl)-3,4-dihydro-2H-benzo[b][1,4]oxazin (8A) herzustellen; optional wobei der zusätzliche Schritt säure- oder basenkatalysiert unter Verwendung eines Säure- oder Basenkatalysators erfolgt, bevorzugt basenkatalysiert unter Verwendung eines Basenkatalysators; und stärker bevorzugt erfolgt der zusätzliche Schritt basenkatalysiert unter Verwendung von NaOH, bevorzugt einer 30%igen wässrigen NaOH-Lösung.

11. Verfahren zur Herstellung von Verbindung (I) 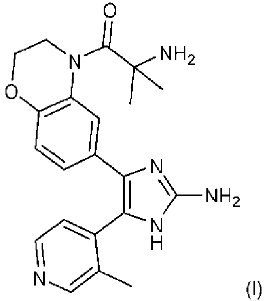 oder eines pharmazeutisch verträglichen Salzes davon, wobei das Verfahren den folgenden Schritt umfasst:
Kupplung von 1-(6-(imidazo[1,2-a]pyrimidin-2-yl)-2,3-dihydro-4H-benzo[b][1,4]oxazin-4-yl)ethan-1-on (5) mit 4-Brom-3-methylpyridin (6) oder einem Salz davon in Gegenwart eines Metallkatalysators, um 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) herzustellen.

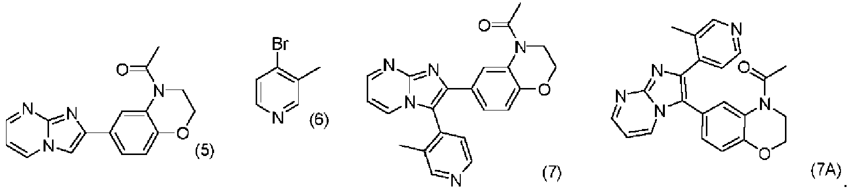

12. Verwendung eines Metallfängerreagenzes in einem Verfahren zur Herstellung von Verbindung (I) 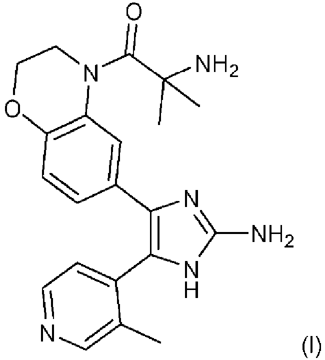 oder eines pharmazeutisch verträglichen Salzes davon, wobei das Verfahren den Schritt der Kupplung von 1-(6-(imidazo[1,2-a]pyrimidin-2-yl)-2,3-dihydro-4H-benzo[b][1,4]oxazin-4-yl)ethan-1-on (5) mit 4-Brom-3-methylpyridin (6) oder einem Salz davon in Gegenwart eines Metallkatalysators umfasst, um 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) herzustellen, 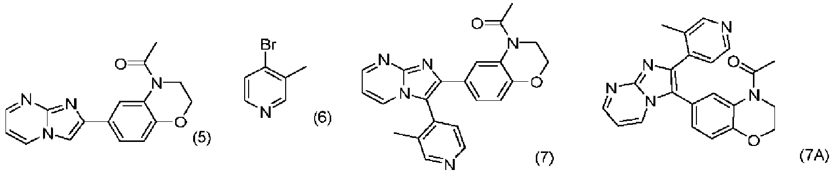 wobei 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) mit dem Metallfängerreagenz behandelt werden; bevorzugt umfasst die Metallfängerbehandlung die Behandlung mit einem Metallfängerreagenz, wobei das Metallfängerreagenz Trithiocyanursäure ist.

13. Verfahren oder Verwendung nach Anspruch 11 oder 12, wobei die Verbindung (I) hergestellt wird; und/oder wobei 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) hergestellt werden; optional wobei 1-(6-(imidazo[1,2-a]pyrimidin-2-yl)-2,3-dihydro-4H-benzo[b][1,4]oxazin-4-yl)ethan-1-on (5) mit 4-Brom-3-methylpyridin (6) gekoppelt wird; und/oder wobei 1-(6-(imidazo[1,2-a]pyrimidin-2-yl)-2,3-dihydro-4H-benzo[b][1,4]oxazin-4-yl)ethan-1-on (5) mit einem Salz von 4-Brom-3-methylpyridin (6), bevorzugt 4-Brom-3-methylpyridinhydrochlorid (6A), gekoppelt wird.

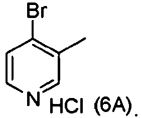

14. Verfahren nach Anspruch 11, oder Anspruch 13, sofern dieser davon abhängig ist, wobei 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A) einer Metallfängerbehandlung unterzogen werden, wobei bevorzugt die Metallfängerbehandlung eine Behandlung mit einem Metallfängerreagenz umfasst; bevorzugt umfasst die Metallfängerbehandlung eine Behandlung mit einem Metallfängerreagenz, wobei das Metallfängerreagenz Trithiocyanursäure ist.

15. Verfahren oder Verwendung nach einem der Ansprüche 11 bis 14, wobei der Metallkatalysator ein auf einem Übergangsmetall basierender Katalysator ist, wie beispielsweise ein Platin-basierter Katalysator, ein Palladium-basierter Katalysator oder ein Iridium-basierter Katalysator, bevorzugt ist der Metallkatalysator ein Palladium-basierter Katalysator und stärker bevorzugt Palladium(II)-acetat.

16. Verfahren zur Herstellung von Verbindung (I) 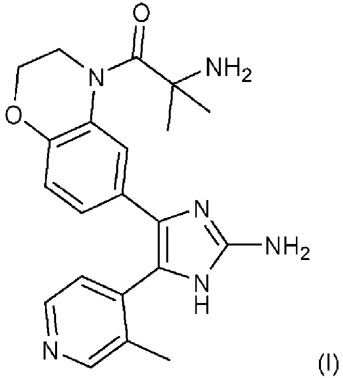 oder eines pharmazeutisch verträglichen Salzes davon, wobei das Verfahren eine Aminosäurekupplung von 4-(3,4-Dihydro-2H-benzo[b][1,4]oxazin-6-yl)-5-(3-methylpyridin-4-yl)-1H-imidazol-2-amin (12) mit 2-Amino-2-methylpropansäure (9) in Gegenwart eines Kupplungsreagenzes oder einer Kupplungsreagenzvorstufe umfasst:

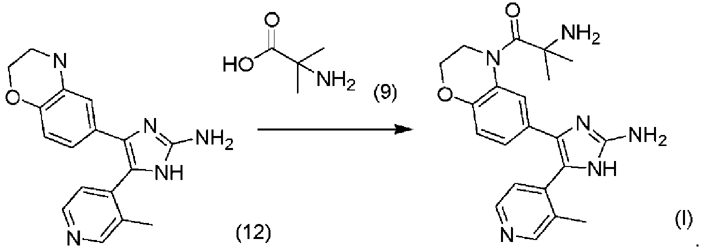

17. Verfahren nach Anspruch 16, wobei die Verbindung (I) hergestellt wird; und/oder wobei das Kupplungsreagenz oder die Kupplungsreagenzvorstufe ausgewählt ist aus Thionylchlorid (SOCl₂), 1-[Bis(dimethylamino)methylen]-1H-1,2,3-triazolo[4,5-b]pyridinium-3-oxid-hexafluorphosphat (HATU), 4-Dimethylaminopyridin (DMAP), Propanphosphonsäureanhydrid (T3P), 1-Ethyl-2-(3-dimethylaminopropyl)carbodiimid·HCl, 1,1'-Carbonyldiimidazol (CDI), Ghosez-Reagenz (1-Chlor-N,N,2-trimethyl-1-propenylamin), Fluor-dipyrrolidinocarbeniumhexafluorphosphat (BTFFH), Ti(iOPr)₄ und B(OCH₂CF₃)₃, wobei bevorzugt das Kupplungsreagenz oder die Kupplungsreagenzvorstufe Thionylchlorid (SOCl₂) ist; und optional wobei die Aminosäurekupplung ein Lösungsmittel verwendet, ausgewählt aus N,N'-Dimethylpropylenharnstoff (DMPU), Dimethylsulfoxid (DMSO), Dimethylformamid (DMF), Hexamethylphosphorsäuretriamid (HMPA) und Acetonitril (ACN) oder Kombinationen davon, bevorzugt ein Lösungsmittel ausgewählt aus Acetonitril, N,N'-Dimethylpropylenharnstoff (DMPU) und Hexamethylphosphorsäuretriamid (HMPA) oder Kombinationen davon, stärker bevorzugt ein Lösungsmittel ausgewählt aus Acetonitril und Hexamethylphosphorsäuretriamid (HMPA) oder Kombinationen davon; und/oder wobei die Aminosäurekupplung N,N'-Dimethylpropylenharnstoff (DMPU) oder Hexamethylphosphorsäuretriamid (HMPA) in Kombination mit Thionylchlorid verwendet.

18. Verfahren nach Anspruch 16 oder 17, wobei das Verfahren weiter einen vorhergehenden Schritt zur Herstellung von Verbindung (12) umfasst, nämlich die Deacylierung von 1-(6-(2-Amino-5-(3-methylpyridin-4-yl)-1H-imidazol-4-yl)-2,3-dihydro-4H-benzo[b][1,4]oxazin-4-yl)ethan-1-on (11), um 4-(3,4-Dihydro-2H-benzo[b][1,4]oxazin-6-yl)-5-(3-methylpyridin-4-yl)-1H-imidazol-2-amin (12) herzustellen, 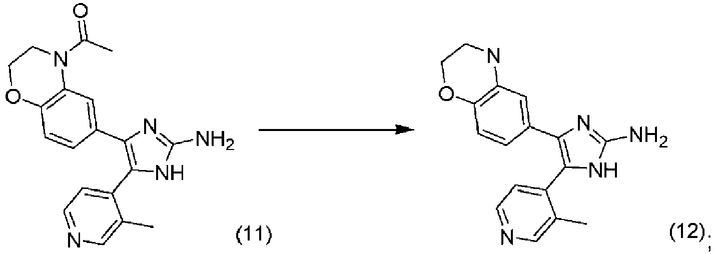 und optional wobei die Deacylierung säure- oder basenkatalysiert erfolgen kann, bevorzugt säurekatalysiert; und/oder wobei das Verfahren weiter einen zusätzlichen vorhergehenden Schritt zur Herstellung von 1-(6-(2-Amino-5-(3-methylpyridin-4-yl)-1H-imidazol-4-yl)-2,3-dihydro-4H-benzo[b][1,4]oxazin-4-yl)ethan-1-on (11) umfasst, nämlich die Entschützung von 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A), um 1-(6-(2-Amino-5-(3-methylpyridin-4-yl)-1H-imidazol-4-yl)-2,3-dihydro-4H-benzo[b][1,4]oxazin-4-yl)ethan-1-on (11) herzustellen; bevorzugt kann die Entschützung säurekatalysiert unter Verwendung eines Säurekatalysators erfolgen oder die Entschützung kann basenkatalysiert unter Verwendung eines Basenkatalysators erfolgen oder es wird ein Entschützungsmittel verwendet, ausgewählt aus NH₂OH·HCl (Hydroxylaminhydrochlorid) und Hydrazin oder dessen Hydrat; bevorzugt wird als Entschützungsmittel NH₂OH·HCl (Hydroxylaminhydrochlorid) verwendet; und optional umfasst die Entschützung ein Lösungsmittel, ausgewählt aus Isopropylalkohol (IPA), Ethanol (EtOH), Dichlormethan (DCM), Methanol (MeOH) und Acetonitril (CH₃CN), bevorzugt ausgewählt aus Methanol, Dichlormethan und Isopropylalkohol (IPA), stärker bevorzugt ist das Lösungsmittel Isopropylalkohol (IPA).

19. Verfahren zur Herstellung von Verbindung (I), 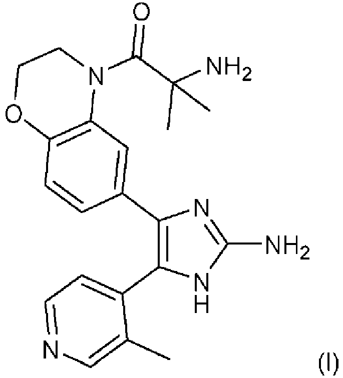 wobei das Verfahren den Schritt der Deacylierung von 1-(6-(2-Amino-5-(3-methylpyridin-4-yl)-1H-imidazol-4-yl)-2,3-dihydro-4H-benzo[b][1,4]oxazin-4-yl)ethan-1-on (11) umfasst, um 4-(3,4-Dihydro-2H-benzo[b][1,4]oxazin-6-yl)-5-(3-methylpyridin-4-yl)-1H-imidazol-2-amin (12) herzustellen, 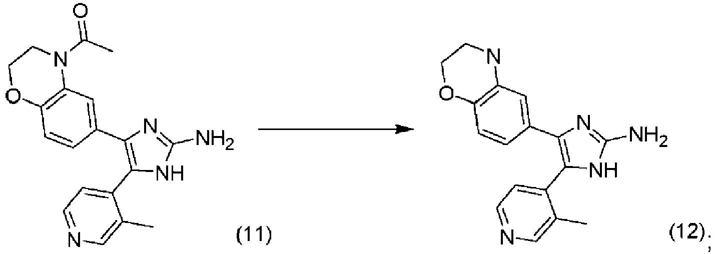 und optional wobei die Deacylierung basen- oder säurekatalysiert erfolgen kann, bevorzugt säurekatalysiert; und/oder wobei das Verfahren weiter einen zusätzlichen vorhergehenden Schritt zur Herstellung von 1-(6-(2-Amino-5-(3-methylpyridin-4-yl)-1H-imidazol-4-yl)-2,3-dihydro-4H-benzo[b][1,4]oxazin-4-yl)ethan-1-on (11) umfasst, nämlich die Entschützung von 1-(6-(3-(3-methylpyridin-4-yl)imidazo[1,2-a]pyrimidin-2-yl)-2,3-dihydro-4H-benzo[b][1,4]oxazin-4-yl)ethan-1-on (7) und/oder 1-(6-(2-(3-methylpyridin-4-yl)imidazo[1,2-a]pyrimidin-3-yl)-2,3-dihydro-4H-benzo[b][1,4]oxazin-4-yl)ethan-1-on (7A), um 1-(6-(2-Amino-5-(3-methylpyridin-4-yl)-1H-imidazol-4-yl)-2,3-dihydro-4H-benzo[b][1,4]oxazin-4-yl)ethan-1-on (11) herzustellen; bevorzugt kann die Entschützung säurekatalysiert unter Verwendung eines Säurekatalysators erfolgen oder die Entschützung kann basenkatalysiert unter Verwendung eines Basenkatalysators erfolgen oder es wird ein Entschützungsmittel verwendet, ausgewählt aus NH₂OH·HCl (Hydroxylaminhydrochlorid) und Hydrazin oder dessen Hydrat; bevorzugt wird als Entschützungsmittel NH₂OH·HCl (Hydroxylaminhydrochlorid) verwendet; und optional umfasst die Entschützung ein Lösungsmittel, ausgewählt aus Isopropylalkohol (IPA), Ethanol (EtOH), Dichlormethan (DCM), Methanol (MeOH) und Acetonitril (CH₃CN), bevorzugt ausgewählt aus Methanol, Dichlormethan und Isopropylalkohol (IPA), stärker bevorzugt ist das Lösungsmittel Isopropylalkohol (IPA).

## Revendications

1. Processus pour la production du composé (I) 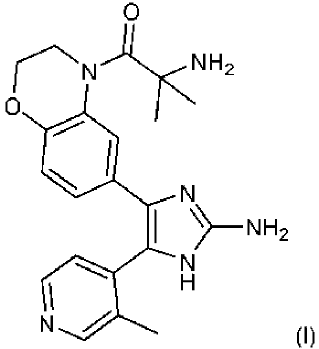 ou d'un sel pharmaceutiquement acceptable de celui-ci, le processus comprenant la déprotection de 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et/ou de 2-amino-2-méthyl-1-(6-(2-(3-méthylpyridin-4-yl)imidazo[1,2-a]pyrimidin-3- yl)-2,3-dihydro-4H-benzo[b][1,4]oxazin-4-yl)propan-1-one (10A), ou des sels de ceux-ci

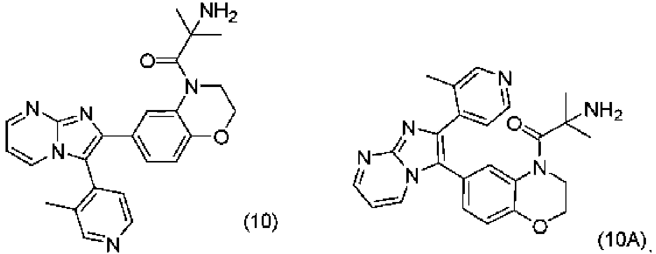

2. Processus selon la revendication 1, dans lequel le processus est destiné à la préparation du composé (I).

3. Processus selon la revendication 1 ou revendication 2, dans lequel le processus comprend la déprotection de 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et/ou de 2-amino-2-méthyl-1-(6-(2-(3-méthylpyridin-4-yl)imidazo[1,2-a]pyrimidin-3- yl)-2,3-dihydro-4H-benzo[b][1,4]oxazin-4-yl)propan-1-one (10A) ; de préférence dans lequel le processus comprend la déprotection de 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et de 2-amino-2-méthyl-1-(6-(2-(3- méthylpyridin-4-yl)imidazo[1,2-a]pyrimidin-3-yl)-2,3-dihydro-4H- benzo[b][1,4]oxazin-4-yl)propan-1-one (10A).

4. Processus selon la revendication 1 ou revendication 2, dans lequel le processus comprend la déprotection d'un sel de 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et/ou d'un sel de 2-amino-2-méthyl-1-(6-(2-(3-méthylpyridin-4- yl)imidazo[1,2-a]pyrimidin-3-yl)-2,3-dihydro-4H-benzo[b][1,4]oxazin-4-yl)propan-1-one (10A), de préférence d'un sel chlorhydrate de 2-amino-2-méthyl-1-(6-(3-(3- méthylpyridin-4-yl)imidazo[1,2-a]pyrimidin-2-yl)-2,3-dihydro-4H- benzo[b][1,4]oxazin-4-yl)propan-1-one (10) et/ou d'un sel chlorhydrate de 2-amino-2-méthyl-1-(6-(2-(3-méthylpyridin-4-yl)imidazo[1,2-a]pyrimidin-3-yl)-2,3-dihydro-4H-benzo[b][1,4]oxazin-4-yl)propan-1-one (10A) ; et/ou dans lequel le processus comprend la déprotection du sel dichlorhydrate (10C) de 2-amino-2-méthyl-1-(6-(3-(3-méthylpyridin-4-yl)imidazo[1,2-a]pyrimidin-2-yl)-2,3-dihydro- 4H-benzo[b][1,4]oxazin-4-yl)propan-1-one : 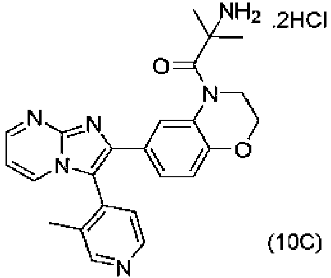 et/ou du sel dichlorhydrate (10D) de 2-amino-2-méthyl-1-(6-(2-(3-méthylpyridin-4-yl)imidazo[1,2-a]pyrimidin-3-yl)-2,3-dihydro-4H-benzo[b][1,4]oxazin-4-yl)propan-1-one :

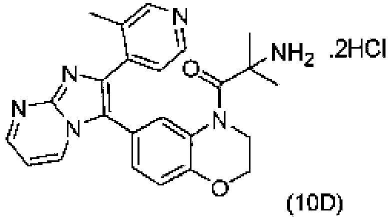

5. Processus selon l'une quelconque des revendications 1 à 4, dans lequel la déprotection est catalysée par un acide en utilisant un catalyseur acide, ou dans lequel la déprotection est catalysée par une base en utilisant un catalyseur basique, ou dans lequel un agent de déprotection est utilisé et sélectionné parmi NH₂OH·HCl (chlorhydrate d'hydroxylamine) et l'hydrazine, ou l'hydrate de ceux-ci ; de préférence dans lequel l'agent de déprotection est NH₂OH·HCl (chlorhydrate d'hydroxylamine) ; et facultativement dans lequel l'agent de déprotection est accompagné d'une base sélectionnée parmi le 1,8-diazabicyclo(5.4.0)undec-7-ène (DBU), la N,N- diisopropyléthylamine (DIPEA), la pipéridine, la morpholine, la pyrrolidine et la triéthylamine (TEA), de préférence le 1,8-diazabicyclo(5.4.0)undec-7-ène (DBU), la N,N- diisopropyléthylamine (DIPEA) et la triéthylamine (TEA), et plus préférablement la triéthylamine (TEA) ; et/ou dans lequel la déprotection comprend un solvant sélectionné parmi l'alcool isopropylique (IPA), l'éthanol (EtOH), le dichlorométhane (DCM), le méthanol (MeOH) et l'acétonitrile (CH₃CN), de préférence le méthanol, le dichlorométhane et l'alcool isopropylique (IPA), et plus préférablement l'alcool isopropylique (IPA).

6. Processus selon la revendication 1, 2, 4, ou la revendication 5
lorsqu'elle en dépend,
dans lequel la déprotection peut être effectuée à une température de 30 à 80 °C, telle que de 40 à 70 °C, de préférence 40 à 60 °C.

7. Processus selon l'une quelconque des revendications 1 à 6, dans lequel le processus comprend en outre une étape précédente comprenant un couplage d'acide aminé de 6-(3-(3-méthylpyridin-4-yl)imidazo[1,2-a]pyrimidin-2-yl)-3,4-dihydro-2H-benzo[b][1,4]oxazine (8) et/ou de 6-(2-(3-méthylpyridin-4-yl)imidazo[1,2-a]pyrimidin-3-yl)-3,4-dihydro-2H-benzo[b][1,4]oxazine (8A), 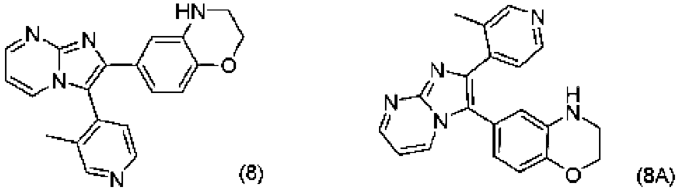 pour former 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et/ou 2-amino-2-méthyl-1-(6-(2-(3-méthylpyridin-4-yl)imidazo[1,2-a]pyrimidin-3-yl)-2,3-dihydro-4H-benzo[b][1,4]oxazin-4-yl)propan-1-one (10A), ou les sels de ceux-ci ; de préférence dans lequel l'étape précédente comprend un couplage d'acide aminé de 6-(3-(3-méthylpyridin-4-yl)imidazo[1,2-a]pyrimidin-2-yl)-3,4-dihydro-2H-benzo[b][1,4]oxazine (8) et/ou de 6-(2-(3-méthylpyridin-4-yl)imidazo[1,2-a]pyrimidin-3-yl)-3,4-dihydro-2H-benzo[b][1,4]oxazine (8A) avec l'acide 2-amino-2-méthylpropanoïque (9) en présence d'un agent de couplage ou d'un précurseur d'agent de couplage pour former 2-amino-2-méthyl-1-(6-(3-(3-méthylpyridin-4-yl)imidazo[1,2-a]pyrimidin-2-yl)-2,3-dihydro-4H-benzo[b][1,4]oxazin-4-yl)propan-1-one (10) et/ou 2-amino-2-méthyl-1-(6-(2-(3-méthylpyridin-4-yl)imidazo[1,2-a]pyrimidin-3-yl)-2,3-dihydro-4H-benzo[b][1,4]oxazin-4-yl)propan-1-one (10A), ou les sels de ceux-ci 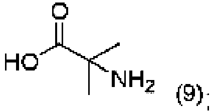 et plus préférablement dans lequel l'étape précédente comprend un couplage d'acide aminé de 6-(3-(3-méthylpyridin-4-yl)imidazo[1,2-a]pyrimidin-2-yl)-3,4-dihydro-2H-benzo[b][1,4]oxazine (8) et de 6-(2-(3-méthylpyridin-4-yl)imidazo[1,2-a]pyrimidin-3-yl)-3,4-dihydro-2H-benzo[b][1,4]oxazine (8A), pour former 2-amino-2-méthyl-1-(6- (3-(3-méthylpyridin-4-yl)imidazo[1,2-a]pyrimidin-2-yl)-2,3-dihydro-4H- benzo[b][1,4]oxazin-4-yl)propan-1-one (10) et 2-amino-2-méthyl-1-(6-(2-(3- méthylpyridin-4-yl)imidazo[1,2-a]pyrimidin-3-yl)-2,3-dihydro-4H- benzo[b][1,4]oxazin-4-yl)propan-1-one (10A).

8. Processus selon la revendication 7, lorsqu'elle dépend des revendications 1 à 3, ou la revendication 5 ou revendication 6 lorsqu'elle dépend des revendications 1 à 3, dans lequel 2-amino-2-méthyl-1-(6-(3-(3- méthylpyridin-4-yl)imidazo[1,2-a]pyrimidin-2-yl)-2,3-dihydro-4H- benzo[b][1,4]oxazin-4-yl)propan-1-one (10) et/ou 2-amino-2-méthyl-1-(6-(2-(3- méthylpyridin-4-yl)imidazo[1,2-a]pyrimidin-3-yl)-2,3-dihydro-4H-benzo[b][1,4]oxazin-4-yl)propan-1-one (10A) sont formés, et dans lequel l'agent de couplage ou le précurseur d'agent de couplage est sélectionné parmi le chlorure de thionyle (SOCl₂), le 1-[Bis(diméthylamino)méthylène]-1 H-1,2,3-triazolo[4,5-b]pyridinium 3-oxyde hexafluorophosphate (HATU), la 4-diméthylaminopyridine (DMAP), l'anhydride d'acide propanephosphonique (T3P), le 1-éthyl-2-)3'-diméthylaminopropyl)carbodiimide.HCl, le 1,1'-carbonyldiimidazole (CDI), le réactif de Ghosez (1-chloro-N,N,2-triméthyl-1-propénylamine), l'hexafluorophosphate de fluoro-dipyrrolidinocarbénium (BTFFH), le Ti(iOPr)₄ et le B(OCH₂CF₃)₃, de préférence l'agent de couplage ou le précurseur d'agent de couplage est le chlorure de thionyle (SOCl₂) ; et facultativement dans lequel le couplage d'acide aminé utilise un solvant sélectionné parmi la N.N'diméthylpropylèneurée (DMPU), le diméthylsulfoxyde (DMSO), le diméthylformamide (DMF), l'hexaméthylphosphoramide (HMPA) et l'acétonitrile (ACN), ou des combinaisons de ceux-ci, de préférence le solvant est sélectionné parmi l'acétonitrile, la N.N'diméthylpropylèneurée (DMPU) et l'hexaméthylphosphoramide (HMPA), ou des combinaisons de ceux-ci, et plus préférablement, le solvant est sélectionné parmi l'acétonitrile et l'hexaméthylphosphoramide (HMPA), ou des combinaisons de ceux-ci ; et/ou dans lequel le couplage d'acide aminé utilise la N.N'diméthylpropylèneurée (DMPU) ou l'hexaméthylphosphoramide (HMPA) en combinaison avec le chlorure de thionyle.

9. Processus selon la revendication 7, lorsqu'elle dépend des revendications 1, 2, 4, ou la revendication 5 ou revendication 6 lorsqu'elle dépend des revendications 1, 2 ou 4, dans lequel un sel de 2-amino-2-méthyl-1-(6-(3-(3-méthylpyridin-4-yl)imidazo[1,2-a]pyrimidin-2-yl)-2,3-dihydro-4H- benzo[b][1,4]oxazin-4-yl)propan-1-one (10) et/ou un sel de 2-amino-2-méthyl-1-(6-(2-(3-méthylpyridin-4-yl)imidazo[1,2-a]pyrimidin-3-yl)-2,3-dihydro-4H- benzo[b][1,4]oxazin-4-yl)propan-1-one (10A), de préférence un sel chlorhydrate de 2- amino-2-méthyl-1-(6-(3-(3-méthylpyridin-4-yl)imidazo[1,2-a]pyrimidin-2-yl)-2,3- dihydro-4H-benzo[b][1,4]oxazin-4-yl)propan-1-one (10), et/ou un sel chlorhydrate de 2-amino-2-méthyl-1-(6-(2-(3-méthylpyridin-4-yl)imidazo[1,2-a]pyrimidin-3- yl)-2,3-dihydro-4H-benzo[b][1,4]oxazin-4-yl)propan-1-one (10A), de préférence le sel dichlorhydrate (10C) de 2-amino-2-méthyl-1-(6-(3-(3-méthylpyridin-4- yl)imidazo[1,2-a]pyrimidin-2-yl)-2,3-dihydro-4H-benzo[b][1,4]oxazin-4-yl)propan-1-one et/ou le sel dichlorhydrate (10D) de 2-amino-2-méthyl-1-(6-(2-(3- méthylpyridin-4-yl)imidazo[1,2-a]pyrimidin-3-yl)-2,3-dihydro-4H-benzo[b][1,4]oxazin-4-yl)propan-1-one, sont formés, et dans lequel l'agent de couplage ou le précurseur d'agent de couplage est sélectionné parmi le chlorure de thionyle (SOCl₂), le 1-[Bis(diméthylamino)méthylène]-1 H-1,2,3-triazolo[4,5-b]pyridinium 3-oxyde hexafluorophosphate (HATU), la 4-diméthylaminopyridine (DMAP), l'anhydride d'acide propanephosphonique (T3P), le 1-éthyl-2-)3'-diméthylaminopropyl)carbodiimide.HCl, le 1,1'-carbonyldiimidazole (CDI), le réactif de Ghosez (1-chloro-N,N,2-triméthyl-1-propénylamine), le (1-cyano-2-éthoxy-2 - oxoéthylidénaminooxy)diméthylamino-morpholino-carbénium hexafluorophosphate (COMU), l'hydroxybenzotriazole, l'hexafluorophosphate de fluoro-dipyrrolidinocarbénium (BTFFH), le Ti(iOPr)₄ et le B(OCH₂CF₃)₃, de préférence l'agent de couplage ou le précurseur d'agent de couplage est sélectionné parmi l'hydroxybenzotriazole, le 1-éthyl-2-)3'-diméthylaminopropyl)carbodiimide.HCl, le 1,1'-carbonyldiimidazole (CDI), le (1-cyano-2-éthoxy-2-oxoéthylidénaminooxy)diméthylamino-morpholino-carbénium hexafluorophosphate (COMU), l'anhydride d'acide propanephosphonique (T3P) et le chlorure de thionyle (SOCl2), et plus préférablement, l'agent de couplage ou le précurseur d'agent de couplage est le chlorure de thionyle ; et facultativement dans lequel la réaction de couplage d'acide aminé utilise un solvant sélectionné parmi la N.N'diméthylpropylèneurée (DMPU), le diméthylsulfoxyde (DMSO), le diméthylformamide (DMF), l'hexaméthylphosphoramide (HMPA), l'acétonitrile (ACN), la N-méthyl-2-pyrrolidone (NMP), la 1,3-diméthyl-2-imidazolidinone (DMI), la gamma-valérolactone (GVL), la N- méthylmorpholine (NMM), le sulfolane, le dichlorométhane (DCM) ou des combinaisons de ceux-ci, de préférence le solvant est sélectionné parmi la N-méthyl-2-pyrrolidone (NMP), la 1,3- diméthyl-2-imidazolidinone (DMI), la gamma-valérolactone (GVL), la N- méthylmorpholine (NMM), le sulfolane, le dichlorométhane (DCM) et l'acétonitrile, ou des combinaisons de ceux-ci, et plus préférablement, le solvant est sélectionné parmi l'acétonitrile ou le sulfolane, le plus préférentiellement l'acétonitrile ; et/ou dans lequel une base est également utilisée en tant que réactif dans le couplage d'acide aminé, de préférence une amine tertiaire, plus préférablement la pyridine.

10. Processus selon l'une quelconque des revendications 7 à 9, dans lequel le processus comprend en outre une étape précédente supplémentaire comprenant la désacylation de 1-(6-(3-(3-méthylpyridin-4-yl)imidazo[1,2-a]pyrimidin-2-yl)-2,3-dihydro-4H-benzo[b][1,4]oxazin-4-yl)ethan-1-one (7) et/ou de 1-(6-(2-(3-méthylpyridin-4-yl)imidazo[1,2-a]pyrimidin-3-yl)-2,3-dihydro-4H-benzo[b][1,4]oxazin-4-yl)ethan-1-one (7A) pour former 6-(3-(3-méthylpyridin-4-yl)imidazo[1,2-a]pyrimidin-2-yl)-3,4- dihydro-2H-benzo[b][1,4]oxazine (8) et/ou 6-(2-(3-méthylpyridin-4- yl)imidazo[1,2-a]pyrimidin-3-yl)-3,4-dihydro-2H-benzo[b][1,4]oxazine (8A) ; de préférence dans lequel l'étape précédente supplémentaire comprend la désacylation de 1-(6-(3-(3-méthylpyridin-4-yl)imidazo[1,2-a]pyrimidin-2-yl)-2,3-dihydro-4H- benzo[b][1,4]oxazin-4-yl)ethan-1-one (7) et de 1-(6-(2-(3-méthylpyridin-4- yl)imidazo[1,2-a]pyrimidin-3-yl)-2,3-dihydro-4H-benzo[b][1,4]oxazin-4-yl)ethan-1-one (7A) pour former 6-(3-(3-méthylpyridin-4-yl)imidazo[1,2-a]pyrimidin-2-yl)-3,4- dihydro-2H-benzo[b][1,4]oxazine (8) et 6-(2-(3-méthylpyridin-4-yl)imidazo[1,2-a]pyrimidin-3-yl)-3,4-dihydro-2H-benzo[b][1,4]oxazine (8A) ; facultativement dans lequel l'étape supplémentaire est catalysée par un acide ou une base avec un catalyseur acide ou basique, de préférence catalysée par une base avec un catalyseur basique ; et plus préférablement dans lequel l'étape supplémentaire est catalysée par une base en utilisant NaOH, de préférence une solution de NaOH à 30 % aqueuse.

11. Processus pour la production du composé (I) 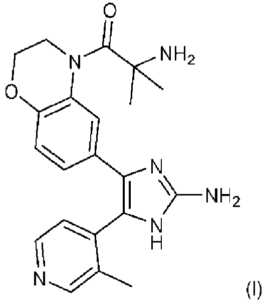 ou d'un sel pharmaceutiquement acceptable de celui-ci, le processus comprenant l'étape suivante :
couplage de 1-(6-(imidazo[1,2-a]pyrimidin-2-yl)-2,3-dihydro-4H- benzo[b][1,4]oxazin-4-yl)ethan-1-one (5) avec 4-bromo-3-méthylpyridine (6), ou un sel de celle-ci, en présence d'un catalyseur métallique pour former 1-(6-(3-(3-méthylpyridin-4-yl)imidazo[1,2-a]pyrimidin-2-yl)-2,3-dihydro-4H-benzo[b][1,4]oxazin-4-yl)ethan-1-one (7) et/ou 1-(6-(2-(3-méthylpyridin-4-yl)imidazo[1,2-a]pyrimidin-3- yl)-2,3-dihydro-4H-benzo[b][1,4]oxazin-4-yl)ethan-1-one (7A)

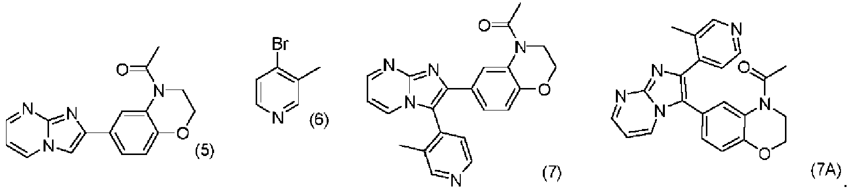

12. Utilisation d'un agent de piégeage des métaux dans un processus de production du composé (I) 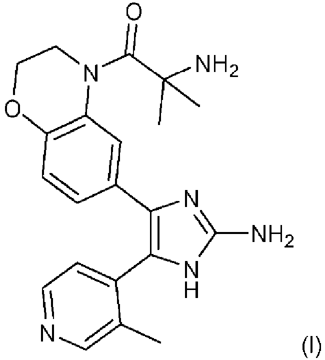 ou d'un sel pharmaceutiquement acceptable de celui-ci, dans lequel le processus comprend l'étape de couplage de 1-(6-(imidazo[1,2-a]pyrimidin-2-yl)-2,3-dihydro-4H-benzo[b][1,4]oxazin-4-yl)ethan-1-one (5) avec 4-bromo-3-méthylpyridine (6), ou un sel de celle-ci, en présence d'un catalyseur métallique pour former 1-(6-(3-(3-méthylpyridin- 4-yl)imidazo[1,2-a]pyrimidin-2-yl)-2,3-dihydro-4H-benzo[b][1,4]oxazin-4- yl)ethan-1-one (7) et/ou 1-(6-(2-(3-méthylpyridin-4-yl)imidazo[1,2-a]pyrimidin-3-yl)-2,3-dihydro-4H-benzo[b][1,4]oxazin-4-yl)ethan-1-one (7A), 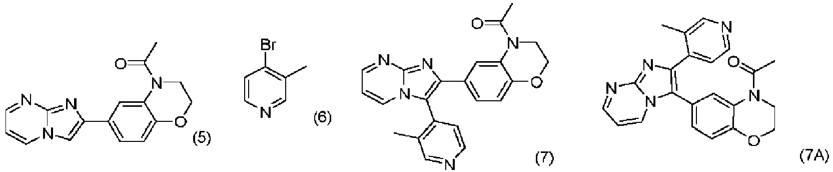 dans laquelle 1-(6-(3-(3-méthylpyridin-4-yl)imidazo[1,2-a]pyrimidin-2-yl)-2,3-dihydro-4H-benzo[b][1,4]oxazin-4-yl)ethan-1-one (7) et/ou 1-(6-(2-(3-méthylpyridin-4-yl)imidazo[1,2-a]pyrimidin-3-yl)-2,3-dihydro-4H-benzo[b][1,4]oxazin-4-yl)ethan-1-one (7A) sont traités avec l'agent de piégeage des métaux ; de préférence dans lequel le traitement de piégeage des métaux comprend un traitement avec un agent de piégeage des métaux et l'agent de piégeage des métaux est l'acide trithiocyanurique.

13. Processus ou utilisation selon la revendication 11 ou revendication 12, dans lequel le composé (I) est formé ; et/ou dans lequel 1-(6-(3-(3-méthylpyridin-4-yl)imidazo[1,2-a]pyrimidin-2-y I)-2,3-dihydro-4H-benzo[b][1,4]oxazin-4-yl)ethan-1-one (7) et 1-(6-(2-(3-méthylpyridin-4-yl)imidazo[1,2-a]pyrimidin-3-yl)-2,3-dihydro-4H- benzo[b][1,4]oxazin-4-yl)ethan-1-one (7A) sont formés ; facultativement dans lequel 1-(6- (imidazo[1,2-a]pyrimidin-2-yl)-2,3-dihydro-4H-benzo[b][1,4]oxazin-4-yl)ethan-1-one (5) est couplé avec 4-bromo-3-méthylpyridine (6) ; et/ou dans lequel 1-(6- (imidazo[1,2-a]pyrimidin-2-yl)-2,3-dihydro-4H-benzo[b][1,4]oxazin-4-yl)ethan-1-one (5) est couplé avec un sel de 4-bromo-3-méthylpyridine (6), de préférence le chlorhydrate de 4-bromo- 3-méthylpyridine (6A)

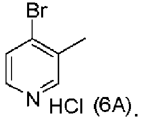

14. Processus selon la revendication 11, ou la revendication 13 lorsqu'elle en dépend, dans lequel 1-(6-(3-(3-méthylpyridin-4-yl)imidazo[1,2-a]pyrimidin-2-yl)-2,3-dihydro-4H-benzo[b][1,4]oxazin-4-yl)ethan-1-one (7) et/ou 1-(6-(2-(3-méthylpyridin-4-yl)imidazo[1,2-a]pyrimidin-3-yl)-2,3-dihydro-4H-benzo[b][1,4]oxazin-4-yl)ethan-1-one (7A) subissent un traitement de piégeage des métaux, de préférence dans lequel le traitement de piégeage des métaux comprend un traitement avec un agent de piégeage des métaux ; de préférence dans lequel le traitement de piégeage des métaux comprend un traitement avec un agent de piégeage des métaux et l'agent de piégeage des métaux est l'acide trithiocyanurique.

15. Processus ou utilisation selon l'une quelconque des revendications 11 à 14, dans lequel le catalyseur métallique est un catalyseur à base de métal de transition tel qu'un catalyseur à base de platine, un catalyseur à base de palladium et un catalyseur à base d'iridium, de préférence le catalyseur métallique est un catalyseur à base de palladium, et plus préférablement le catalyseur métallique est l'acétate de palladium (II).

16. Processus pour la production du composé (I) 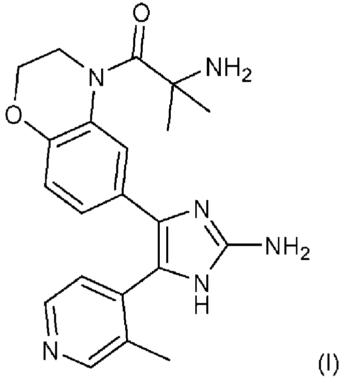 ou d'un sel pharmaceutiquement acceptable de celui-ci, le processus comprenant un couplage d'acide aminé de 4-(3,4-Dihydro-2H-benzo[b][1,4]oxazin-6-yl)-5-(3-méthylpyridin-4-yl)-1H-imidazol-2-amine (12) avec l'acide 2-amino-2-méthylpropanoïque (9) en présence d'un agent de couplage ou d'un précurseur d'agent de couplage :

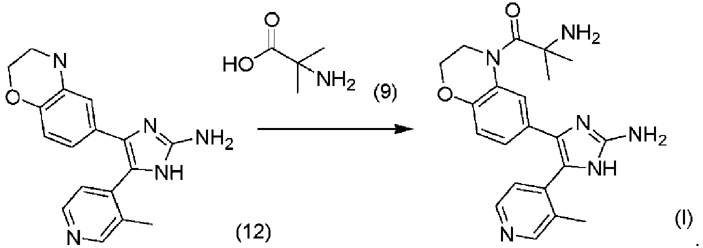

17. Processus selon la revendication 16, dans lequel le composé (I) est formé ; et/ou dans lequel l'agent de couplage ou le précurseur d'agent de couplage est sélectionné parmi le chlorure de thionyle (SOCl₂), le 1-[Bis(diméthylamino)méthylène]-1H-1,2,3-triazolo[4,5-b]pyridinium 3- oxyde hexafluorophosphate (HATU), la 4-diméthylaminopyridine (DMAP), l'anhydride d'acide propanephosphonique (T3P), la 1-éthyl-2-)3'-diméthylaminopropyl)carbodiimide.HCl, la 1,1'-carbonyldiimidazole (CDI), le réactif de Ghosez (1-chloro-N,N,2-triméthyl-1 -propénylamine), l'hexafluorophosphate de fluoro-dipyrrolidinocarbénium (BTFFH), le Ti(iOPr)₄ et le B(OCH₂CF₃)₃, de préférence, l'agent de couplage ou le précurseur d'agent de couplage est le chlorure de thionyle (SOCl₂) ; et facultativement dans lequel le couplage d'acide aminé utilise un solvant sélectionné parmi la N.N'diméthylpropylèneurée (DMPU), le diméthylsulfoxyde (DMSO), le diméthylformamide (DMF), l'hexaméthylphosphoramide (HMPA) et l'acétonitrile (ACN), ou des combinaisons de ceux-ci, de préférence un solvant sélectionné parmi l'acétonitrile, la N.N'diméthylpropylèneurée (DMPU) et l'hexaméthylphosphoramide (HMPA), ou des combinaisons de ceux-ci, et plus préférablement un solvant sélectionné parmi l'acétonitrile et l'hexaméthylphosphoramide (HMPA), ou des combinaisons de ceux-ci ; et/ou dans lequel le couplage d'acide aminé utilise la N.N'diméthylpropylèneurée (DMPU) ou l'hexaméthylphosphoramide (HMPA) en combinaison avec le chlorure de thionyle.

18. Processus selon la revendication 16 ou revendication 17, dans lequel le processus comprend en outre une étape précédente pour la formation du composé (12) de la désacylation de 1-(6-(2-Amino-5-(3-méthylpyridin-4-yl)-1H-imidazol-4-yl)-2,3-dihydro-4H-benzo[b][1,4]oxazin-4-yl)ethan-1-one (11) pour former 4-(3,4-Dihydro-2H-benzo[b][1,4]oxazin-6-yl)-5-(3-méthylpyridin-4-yl)-1H-imidazol-2-amine (12) 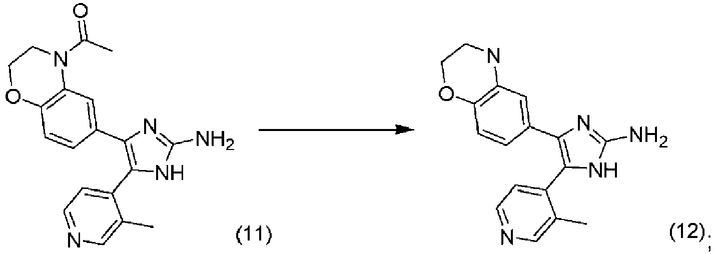 et facultativement dans lequel la désacylation peut être catalysée par une base ou un acide, de préférence catalysée par un acide ; et/ou dans lequel le processus comprend en outre une étape précédente supplémentaire pour la formation de 1-(6-(2-Amino-5-(3-méthylpyridin-4-yl)-1H- imidazol-4-yl)-2,3-dihydro-4H-benzo[b][1,4]oxazin-4-yl)ethan-1-one (11) de la déprotection de 1-(6-(3-(3-méthylpyridin-4-yl)imidazo[1,2-a]pyrimidin-2-yl)-2,3- dihydro-4H-benzo[b][1,4]oxazin-4-yl)ethan-1-one (7) et/ou de 1-(6-(2-(3-méthylpyridin-4-yl)imidazo[1,2-a]pyrimidin-3-yl)-2,3-dihydro-4H- benzo[b][1,4]oxazin-4-yl)ethan-1-one (7A), pour former 1-(6-(2-Amino-5-(3-méthylpyridin-4-yl)-1H-imidazol-4-yl)-2,3-dihydro-4H-benzo[b][1,4]oxazin-4-yl)ethan-1-one (11) ; de préférence dans lequel la déprotection peut être catalysée par un acide en utilisant un catalyseur acide ou la déprotection peut être catalysée par une base en utilisant un catalyseur basique ou un agent de déprotection est utilisé et sélectionné parmi NH₂OH.HCl (chlorhydrate d'hydroxylamine) et l'hydrazine, ou l'hydrate de ceux-ci ; de préférence dans lequel un agent de déprotection est utilisé et est NH₂OH.HCl (chlorhydrate d'hydroxylamine) ; et facultativement dans lequel la déprotection comprend un solvant sélectionné parmi l'alcool isopropylique (IPA), l'éthanol (EtOH), le dichlorométhane (DCM), le méthanol (MeOH) et l'acétonitrile (CH₃CN), de préférence sélectionné parmi le méthanol, le dichlorométhane et l'alcool isopropylique (IPA), et plus préférablement, dans lequel le solvant est l'alcool isopropylique (IPA).

19. Processus pour la production du composé (I), 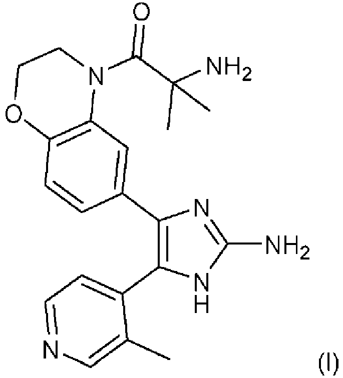 le processus comprenant l'étape de désacylation de 1-(6-(2-Amino-5-(3- méthylpyridin-4-yl)-1H-imidazol-4-yl)-2,3-dihydro-4H-benzo[b][1,4]oxazin-4- yl)ethan-1-one (11) pour former 4-(3,4-Dihydro-2H-benzo[b][1,4]oxazin-6-yl)-5-(3- méthylpyridin-4-yl)-1 H-imidazol-2-amine (12) 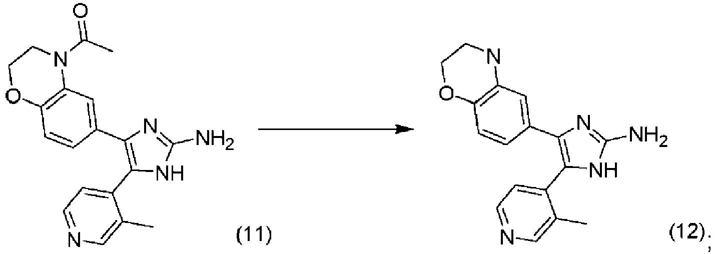 et facultativement dans lequel la désacylation peut être catalysée par une base ou un acide, de préférence catalysée par un acide ; et/ou dans lequel le processus comprend en outre une étape précédente supplémentaire pour la formation de 1-(6-(2-Amino-5-(3-méthylpyridin-4-yl)-1H- imidazol-4-yl)-2,3-dihydro-4H-benzo[b][1,4]oxazin-4-yl)ethan-1-one (11) de la déprotection de 1-(6-(3-(3-méthylpyridin-4-yl)imidazo[1,2-a]pyrimidin-2-yl)-2,3- dihydro-4H-benzo[b][1,4]oxazin-4-yl)ethan-1-one (7) et/ou de 1-(6-(2-(3-méthylpyridin-4-yl)imidazo[1,2-a]pyrimidin-3-yl)-2,3-dihydro-4H- benzo[b][1,4]oxazin-4-yl)ethan-1-one (7A), pour former 1-(6-(2-Amino-5-(3- méthylpyridin-4-yl)-1H-imidazol-4-yl)-2,3-dihydro-4H-benzo[b][1,4]oxazin-4- yl)ethan-1-one (11) ; de préférence dans lequel la déprotection peut être catalysée par un acide en utilisant un catalyseur acide ou la déprotection peut être catalysée par une base en utilisant un catalyseur basique ou un agent de déprotection est utilisé et sélectionné parmi NH₂OH.HCl (chlorhydrate d'hydroxylamine) et l'hydrazine, ou l'hydrate de ceux-ci ; de préférence dans lequel un agent de déprotection est utilisé et est NH₂OH.HCl (chlorhydrate d'hydroxylamine) ; et facultativement dans lequel la déprotection comprend un solvant sélectionné parmi l'alcool isopropylique (IPA), l'éthanol (EtOH), le dichlorométhane (DCM), le méthanol (MeOH) et l'acétonitrile (CH₃CN), de préférence sélectionné parmi le méthanol, le dichlorométhane et l'alcool isopropylique (IPA), et plus préférablement, dans lequel le solvant est l'alcool isopropylique (IPA).
